# EUROPEAN PATENT APPLICATION

(11) **EP 2 465 859 A1**
(43) Date of publication of application: **20.06.2012**
(21) Application number: 11002682.0
(22) Date of filing: 31.03.2011
(51) Int. Cl.: C07D 473/06, C07D 473/08, C07D 473/12, A61K 31/522, A61P 25/28

(54) **8-Triazolylxanthine derivatives, processes for their production and their use as adenosine receptor antagonists**

(30) Priority: 08.12.2010 US 421055 P
(71) Applicant: Life & Brain GmbH, 53127 Bonn (DE)
(72) Inventor: Müller, Christa, 53113 Bonn (DE); Zimmer, Andreas, 53125 Bonn (DE); Labs, Stefanie, 53117 Bonn (DE); Buchholz, Stephanie, 51067 Köln (DE); Hinz, Sonja, 53111 Bonn (DE)
(74) Representative: Castell, Klaus

(57) **Abstract**

The invention relates to derivatives of the general formulae I and II 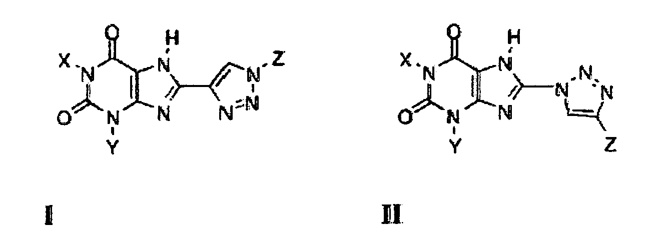 to processes for the production thereof, to pharmaceutical preparations containing said compounds and/or physiologically compatible salts, or solvates which can be produced therefrom as well as to the pharmaceutical use of said compounds, the salts, or solvates thereof as adenosine receptor antagonists, in particular for the treatment of neurodegenerative disorders, e.g. stroke, amylotrophic lateral sclerosis, dementia, Alzheimer's disease, Parkinson's disease, ischemia/reperfusion injury, inflammation, and/or neurological disorder. The blockade of adenosine receptors could also be useful for other indications regarding the metabolism, e.g. diabetic retinopathy, diabetes mellitus, hyperbaric oxygen-induced retinopathy and/or obesity. Applications could also be the treatment of allergic diseases and autoimmune diseases, including mast cell degranulation, asthma, bronchoconstriction, pulmonary fibrosis, inflammatory or obstructive airways disease and/or chronic obstructive pulmonary disease (COPD). In addition, they could be used to treat cancer, e.g. proliferating tumor, cell proliferation disorders, angiogenesis, lung cancer, breast cancer, pancreatic cancer, thyroid cancer, skin cancer, vascular endothelial cancer, cancer of the central nervous system, esophageal cancer, cancer of the larynx, gastrointestinal cancer, colon cancer, colorectal cancer, rectal cancer, liver cancer, renal cancer, prostate cancer, bladder cancer, cervical cancer, ovarian cancer, endometrial cancer, melanoma, squamous cell carcinoma, basal cell carcinoma, non-small cell lung cancer selected from squamous cell carcinoma, adenocarcinoma, large cell carcinoma, adenosquam- ous carcinoma and/or undifferentiated carcinoma. The diseases associated with adenosine receptors are also diabetes, diarrhea, inflammatory bowel disease and/or gastrointestinal tract disorders. Adenosine receptor antagonists could be effective for treating a hepatic disease or condition for reducing fat deposition in the liver or fibrosis of the liver. The use of compounds of general formulae I and II can be associated with many applications e.g., scleroderm arthritis, atherosclerosis, urticaria, myocardial infarction, myocardial reperfusion after ischemia, vasodilation, hypertension, hypersensitivity, myocardial ischemia, heart attack and/or retinopathy of prematurity.

## Description

### Field of the invention

The invention relates to derivatives of the general formulae **I** and **II** 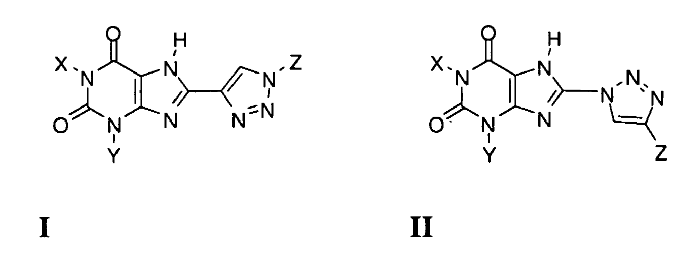 to processes for the production thereof, to pharmaceutical preparations containing said compounds and/or physiologically compatible salts, solvates or prodrugs which can be produced therefrom as well as to the pharmaceutical use of said compounds, the salts, solvates or prodrugs thereof as adenosine receptor antagonists, in particular for the treatment of neurodegenerative disorders, e.g. stroke, amylotrophic lateral sclerosis, dementia, Alzheimer's disease, Parkinson's disease, ischemia/reperfusion injury, inflammation, and/or additional neurological disorder. The blockade of adenosine receptors could also be useful for other indications regarding the metabolism, e.g. diabetic retinopathy, diabetes mellitus, hyperbaric oxygen-induced retinopathy and/or obesity. Applications could also be the treatment of allergic diseases and autoimmune diseases, including mast cell degranulation, asthma, bronchoconstriction, pulmonary fibrosis, inflammatory or obstructive airways disease and/or chronic obstructive pulmonary disease (COPD). In addition, they could be used to treat cancer, e.g. proliferating tumor, cell proliferation disorders, angiogenesis, lung cancer, breast cancer, pancreatic cancer, thyroid cancer, skin cancer, vascular endothelial cancer, cancer of the central nervous system, esophageal cancer, cancer of the larynx, gastrointestinal cancer, colon cancer, colorectal cancer, rectal cancer, liver cancer, renal cancer, prostate cancer, bladder cancer, cervical cancer, ovarian cancer, endometrial cancer, melanoma, squamous cell carcinoma, basal cell carcinoma, non-small cell lung cancer, squamous cell carcinoma, adenocarcinoma, large cell carcinoma, adenosquamous carcinoma and/or undifferentiated carcinoma. Diseases associated with adenosine receptors are also diabetes, diarrhea, inflammatory bowel disease and/or gastrointestinal tract disorders. Adenosine receptor antagonists could be effective for treating a hepatic disease or condition for reducing fat deposition in the liver or fibrosis of the liver. The use of compounds of general formulae **I** and **II** can be associated with many applications e.g., scleroderm arthritis, atherosclerosis, urticaria, myocardial infarction, myocardial reperfusion after ischemia, vasodilation, hypertension, hypersensitivity, myocardial ischemia, heart attack and/or retinopathy of prematurity.

### Background of the invention

Microglia activation is associated with morphological changes, upregulation of cell surface molecules and production of cytokines. Microglia cells express adenosine receptors.

The A_{2B} receptors mediate an increased IL6 synthesis (Fiebich et al., J. Neurochem. 1996, 66, 1426-1431), which is a proinflammatory cytokine and leads to neuroinflammation. Additionally, microglia activation is involved in the pathogenesis of multiple neurodegenerative diseases. For example, in ischemic injury (e.g. after a stroke) the adenosine concentration is strongly elevated in the penumbral region of the brain. This could be neuroprotective, but also pro-inflammatory depending on the receptor subtype and cell type affected. Thus, adenosine receptors play an important role in the pathogenesis of ischemic injury.

Ischemic stroke is defined by the reduction of the cerebral blood flow in the territory of a major cerebral artery because of a transient or permanent occlusion by a local thrombosis or embolus. During this occlusion complex molecular events occur, which lead to neuronal death. An important molecular change during ischemia is the loss of oxygen and glucose which results in changes of the intracellular ion and metabolite homeostasis. Additionally, the cessation of blood flow results in generation of free radicals and an increased intracellular Ca²⁺ concentration which is associated with necrotic and apoptotic cell death. The decreased pH and increased NO-generation contribute to the infarct size. Additionally there is a release of glutamate from the core of the infarcted area and a prolonged increase of Ca²⁺ via activation of ionotropic glutamate receptors in the penumbral region. Molecular changes also occur during reperfusion, where additional tissue damage is mediated by reactive oxygen and nitrogen species and the activation of inflammatory genes. Thus, the activation of PARP-1, the stress-kinase signaling and the recruitment of leukocytes into the damaged area play an important role.

Although much is known about what occurs at the molecular level during neurodegenerative disorders, there are no satisfactory therapeutic approaches until now.

This is the reason why there are considerable efforts to discover new therapeutic agents that limit the neuronal injury e.g. in stroke patients, so-called neuroprotective agents.

### Summary of the invention

The object of the invention is to provide novel and more effective compounds having a small side-effect profile for the treatment of patients suffering from neurodegenerative disorders, in particular of patients suffering from stroke, amylotrophic lateral sclerosis or Alzheimer's disease by adenosine A_{2B} receptor antagonists. Also, other indications, which could benefit from a blockade of A_{2B} receptor blockade are claimed.

This object has been solved by the subject matter of the present claims 1, 4 and 8. Preferred embodiments are shown in the dependent claims. According to the invention, this object is achieved by producing and characterizing active substances which correspond to general formulae **I** and **II**.

### Description of the invention

The inventors have recognized that A_{2B} receptor antagonists as neuroprotective agents are very useful drugs since the adenosine concentration in ischemic injury is strongly elevated in the penumbral region of the brain. This could be neuroprotective but also proinflammatory depending on the receptor subtype and cell type affected. It is known that the A_{2B} receptor has a low affinity to adenosine, with an EC₅₀ in micromolar concentrations in most tissues, in contrast to the EC₅₀ of other adenosine receptors which typically lies between 10 nM and 1 µM. Because of that higher adenosine concentrations are required for activation of the A_{2B} receptor. These higher concentrations of adenosine are achieved during pathophysiological conditions (like hypoxia, inflammation and ischemia). Therefore it has been assumed that increased A_{2B}-signaling in the penumbral region results in excessive expression of cytokines, which leads to neurodegeneration. So the inventors suggested that the inhibition of A_{2B} receptors via A_{2B} receptor antagonists might be neuroprotective in ischemic injury. The inhibition of A_{2B} receptors via A_{2B} receptor antagonists is useful for all neurodegenerative disorders, in particular stroke, all stages of dementia (particularly Alzheimer's disease), amylotrophic lateral sclerosis and the treatment of aboved mentioned diseases.

The inventors identified the chemical class of 8-triazolylxanthine derivatives as adenosine A_{2B} receptor antagonists. Particular useful compounds are those of general formula **I** and **II** 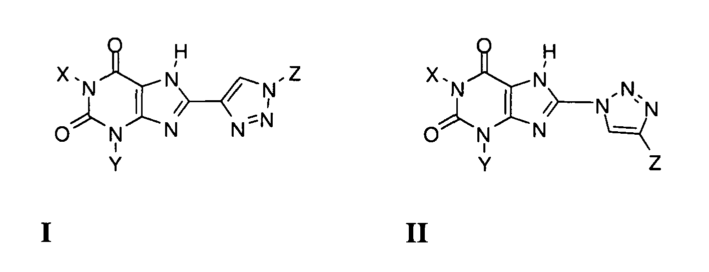 wherein
X, Y are independently
H
C₁-C₁₀ alkyl (linear or branched)
C₂-C₁₀ alkenyl
C₂-C₁₀ alkynyl
C₃-C₆ cycloalkyl (substituted or unsubstituted, saturated or unsaturated)
C₃-C₆ heterocycloalkyl (saturated or unsaturated, wherein 1 to 3 ring atoms are independently selected from N, O and S)
aryl (single or multiple substituted or unsubstituted)
C₃-C₆ heteroaryl (substituted or unsubstituted, wherein 1 to 3 ring atoms are independently selected from N, O and S)
benzyl (single or multiple substituted or unsubstituted)
Z is
H
C₁-C₁₀ alkyl (linear or branched)
C₂-C₁₀ alkenyl
C₂-C₁₀ alkynyl
C₃-C₆ cycloalkyl (substituted or unsubstituted, saturated or unsaturated)
C₃-C₆ heterocycloalkyl (saturated or unsaturated, wherein 1 to 3 ring atoms are independently selected from N, O and S)
aryl (single or multiple substituted or unsubstituted)
C₃-C₆ heteroaryl (substituted or unsubstituted, wherein 1 to 3 ring atoms are independently selected from N, O and S)
benzyl (single or multiple substituted or unsubstituted)
as well as pharmaceutically compatible salts, solvates and prodrugs of these compounds.

Unless otherwise stated, the following terms used in the specification and claims are defined for the purposes of this application and have the following meaning: "Alkyl" means a linear saturated monovalent hydrocarbon radical of one to ten carbon atoms or a branched saturated monovalent hydrocarbon radical of three to ten carbon atoms, e.g., methyl, ethyl, propyl, 2-propyl, butyl (including all isomeric forms), pentyl (including all 15 isomeric forms), and the like.

"Alkenyl" means a linear monovalent hydrocarbon radical of two to ten carbon atoms or a branched monovalent hydrocarbon radical of three to ten carbon atoms containing a double bond, e.g., ethenyl, propenyl, 2-propenyl, butenyl (including all isomeric forms), pentyl (including all isomeric forms), and the like.

"Alkynyl" means a linear monovalent hydrocarbon radical of two to ten carbon atoms or a branched monovalent hydrocarbon radical of three to ten carbon atoms containing a triple bond, e.g., ethynyl, propynyl, 2-propynyl, butynyl (including all isomeric forms), pentyl (including all isomeric forms), and the like.

"Amino" means a -NH₂

"Alkylamino" means a -NHR radical where R is alkyl as defined above, e.g., 30 methylamino, ethylamino, propylamino, or 2-propylamino, and the like.

"Alkoxy" means a -OR radical where R is alkyl as defined above, e.g., methoxy, ethoxy, propoxy, or 2-propoxy, *n-, iso-,* or *tert*-butoxy, and the like.

"Alkylthio" means a -SR radical where R is alkyl as defined above, e.g., methylthio, and the like.

""Aryl" means a monovalent monocyclic or bicyclic aromatic hydrocarbon radical of 6 to 10 ring atoms e.g., phenyl or naphthyl. The aryl ring can optionally be substituted with one, two or three substituents independently selected from alkyl, hydroxy, carboxy, alkoxy, halo, haloalkyl, cyano, alkylamino, dialkylamino, haloalkoxy, hydroxyalkyl or haloalkoxy.

"Aralkyl" means a -(alkylene)-R radical where R is aryl as defined above.

"Benzyl" means the "-CH₂-C₆ H₅" group with may be substituted with one, two or three substituents independently selected from alkyl, hydroxy, carboxy, alkoxy, halo, haloalkyl, cyano, alkylamino, dialkylamino, haloalkoxy, hydroxyalkyl or haloalkoxy.

"Cycloalkyl" means a cyclic saturated monovalent hydrocarbon radical of three to ten carbon atoms, e.g., cyclopropyl, cyclobutyl, cyclopentyl, or cyclohexyl, and the like.

Cycloalkylalkyl" means a -(alkylene)-R radical where R is cycloalkyl as defined above; e.g., cyclopropylmethyl, cyclobutylmethyl, cyclopentylethyl, or cyclohexylmethyl, and the like.

"Carboxy" means -COOH.

"Dialkylamino" means a -NRR' radical where R and R' are independently alkyl as defined herein, e.g., dimethylamino, and the like.

"Halo" means fluoro, chloro, bromo, or iodo, preferably fluoro or chloro.

"Haloalkyl" means alkyl radical as defined above, which is substituted with one or more 25 halogen atoms, preferably one to five halogen atoms, preferably fluorine or chlorine, including those substituted with different halogens, e.g., -CH₂Cl, -CF₃, - CHF₂, -CH₂CF₃, -CF₂CF₃, - CF(CH₃)₂, and the like. When the alkyl is substituted with only fluoro, it is referred to in this Application as fluoroalkyl.

"Haloalkoxy" means a -OR radical where R is haloalkyl as defined above e.g., - OCF₃,- OCHF₂, and the like. When R is haloalkyl where the alkyl is substituted with only fluoro, it is referred to in this Application as fluoroalkoxy.

"Hydroxyalkyl" means a linear monovalent hydrocarbon radical of one to six carbon atoms or a branched monovalent hydrocarbon radical of three to six carbons substituted with one or two hydroxy groups, provided that if two hydroxy groups are present they are not both on the same carbon atom. Representative examples include, but are not limited to, hydroxymethyl, 2-hydroxyethyl, 2-hydroxypropyl, 3-hydroxypropyl, 1-(hydroxymethyl)-2- methylpropyl, 2-hydroxybutyl, 3-hydroxybutyl, 4-hydroxybutyl, 2,3-dihydroxypropyl, 1- (hydroxymethyl)-2-hydroxyethyl, 2,3-dihydroxybutyl, 3,4-dihydroxybutyl and 2- (hydroxymethyl)-3-hydroxypropyl, preferably 2-hydroxyethyl, 2,3-dihydroxypropyl, and 1- (hydroxymethyl)-2-hydroxyethyl.

"Hydroxyalkoxy" or "hydroxyalkyloxy" means a -OR radical where R is hydroxyalkyl as defined above.

"Heterocyclyl" or "heterocycloalkyl" means a saturated or unsaturated monovalent monocyclic group of 4 to 6 ring atoms in which one or three ring atoms are heteroatom selected from N, O, or S(0)ₙ, where n is an integer from 0 to 2, the remaining ring atoms being C.

Additionally, one or two ring carbon atoms in the heterocyclyl ring can optionally be replaced by a -CO- group. More specifically the term heterocyclyl includes, but is not limited to, pyrrolidino, piperidino, homopiperidino, 2-oxopyrrolidinyl, 2-oxopiperidinyl, morpholino, piperazino, tetrahydropyranyl, thiomorpholino, and the like. When the heterocyclyl ring is unsaturated it can contain one or two ring double bonds provided that the ring is not aromatic. When the heterocyclyl group contains at least one nitrogen atom, it is also referred to herein as heterocycloamino and is a subset of the heterocyclyl group. The heterocyclyl ring can optionally be substituted with one, two or three substituents independently selected from alkyl, hydroxy, carboxy, alkoxy, halo, haloalkyl, cyano, alkylamino, dialkylamino, haloalkoxy, hydroxyalkyl, or haloalkoxy.

Heterocyclylalkyl" means a -(alkylene)-R radical where R is heterocyclyl ring as defined above e.g., tetraydrofuranylmethyl, piperazinylmethyl, morpholinylethyl, and the like.

"Heteroaryl" means a monovalent monocyclic or bicyclic aromatic radical of 3 to 6 ring atoms where one or more, preferably one, two, or three, ring atoms are heteroatom selected from N, O, or S, the remaining ring atoms being carbon. Representative examples include, but are not limited to, pyrrolyl, thienyl, thiazolyl, imidazolyl, furanyl, indolyl, isoindolyl, oxazolyl, isoxazolyl, benzothiazolyl, benzoxazolyl, quinolinyl, isoquinolinyl, pyridinyl, pyrimidinyl, pyrazinyl, pyridazinyl, triazolyl, tetrazolyl, and the like. The heteroaryl ring can optionally be substituted with one, two or three substituents independently selected from alkyl, hydroxy, carboxy, alkoxy, halo, haloalkyl, cyano, alkylamino, dialkylamino, haloalkoxy, hydroxyalkyl, or haloalkoxy.

"Heteroaralkyl" means a -(alkylene)-R radical where R is heteroaryl as defined above.

"Substituted alkyl" means a alkyl radical as defined above in which one, two, or three hydrogen atoms are independently replaced by hydroxyl, alkoxy, or halo.

The term "oxo", when used as a substitutent, means the =0 group, which is typically attached to a carbon atom.

"Optional" or "optionally" means that the subsequently described event or circumstance may but need not occur, and that the description includes instances where the event or circumstance occurs and instances in which it does not. For example, "heterocyclyl group optionally substituted with an alkyl group" means that the alkyl may but need not be present, and the description includes situations where the heterocyclyl group is substituted with an alkyl group and situations where the heterocyclyl group is not substituted with alkyl.

A "pharmaceutically acceptable salt" of a compound means a salt that is pharmaceutically acceptable and that possesses the desired pharmacological activity of the parent compound. Such salts include: acid addition salts, formed with inorganic acids such as hydrochloric acid, hydrobromic acid, sulfuric acid, nitric acid, phosphoric acid, and the like; or formed with organic acids such as formic acid, acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4- hydroxybenzoyl)benzoic acid, cinnamic acid, mandelic acid, methanesulfonic acid, ethanesulfonic acid, 1,2-ethanedisulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, glucoheptonic acid, 4,4'-methylenebis-(3-hydroxy-2-ene-1-carboxylic acid), 3-phenylpropionic acid, trimethylacetic acid, tertiary butylacetic acid, lauryl sulphuric acid, gluconic acid, glutamic acid, hydroxynaphthoic acid, salicylic acid, stearic acid, muconic acid, and the like; or salts formed when an acidic proton present in the parent compound either is replaced by a metal ion, e.g., an alkali metal ion, an alkaline earth ion, or an aluminum ion; or coordinates with an organic base such as ethanolamine, diethanolamine, triethanolamine, tromethamine, A -mcthylglucaminc, and the like. It is understood that the pharmaceutically acceptable salts are non-toxic. Additional information on suitable pharmaceutically acceptable salts can be found in Remington's Pharmaceutical Sciences, 17th ed., Mack Publishing Company, Easton, PA, 1985, which is incorporated herein by reference.

The term "pharmaceutically acceptable" means that the referenced substance, such as a compound of Formula I or a salt of a compound of Formula lor a formulation containing a compound of Formula I or a particular excipent, are suitable for administration to a patient.

The term "excipient" means any pharmaceutically acceptable additive, carrier, diluent, adjuvant, or other ingredient, other than the active pharmaceutical ingredient (API), which is typically included for formulation and/or administration to a patient.

Treating" or "treatment" of a disease includes:
preventing the disease, i.e. causing the clinical symptoms of the disease not to develop in a mammal that may be exposed to or predisposed to the disease but does not yet experience or display symptoms of the disease;
inhibiting the disease, i.e., arresting or reducing the development of the disease or its clinical symptoms; or
relieving the disease, i.e., causing regression of the disease or its clinical symptoms. A "therapeutically effective amount" means the amount of a compound of Formula (I) or (II) that, when administered to a mammal for treating a disease, is sufficient to effect such treatment for the disease. The "therapeutically effective amount" will vary depending on the compound, the disease and its severity and the age, weight, etc., of the mammal to be treated.

The term "prodrug" means compounds that are transformed in vivo to yield a compound of the present invention. The transformation may occur by various mechanisms, such as through hydrolysis in blood. A discussion of the use of prodrugs is provided by T.

Higuchi and W. Stella, "Pro-drugs as Novel Delivery Systems," Vol. 14 of the A.C.S. Symposium Series, and in Bioreversible Carriers in Drug Design, ed. Edward B. Roche, American Pharmaceutical Association and Pergamon Press, 1987.

To illustrate, if the compound of the invention contains a carboxylic add functional 5 group, a prodrug can comprise an ester formed by the replacement of the hydrogen atom of the add group with a group such as (C₁-C₈ alkyl, (C₂-Cl₂)alkanoyloxymethyl, 1- (alkanoyloxy)ethyl having from 4 to 9 carbon atoms, 1-methyl-1-(alkanoyloxy)ethyl having from 5 to 10 carbon atoms, alkoxycarbonyloxy-methyl having from 3 to 6 carbon atoms, 1- (alkoxycarbonyloxy)ethyl having from 4 to 7 carbon atoms, 1-methyl-1-(alkoxycarbonyloxy)- 10 ethyl having from 5 to 8 carbon atoms, N-(alkoxycarbonyl)aminomethyl having from 3 to 9 carbon atoms, 1-(N-(alkoxycarbonyl)aminomethyl having from 4 to 10 carbon atoms, 3- phthalidyl, 4-crotonolactonyl, gamma-butyrolacton-4-yl, di-N,N-(C₁-C₂)alkylamino(C₂- C₃) alkyl (such as P-dimethylaminoethyl), carbamoyl-(Ci-C2)alkyl, N,N-di(Ci-C2)alkylcarbamoyl-(Ci-C2)alkyl and piperidino-, pyrrolidino- or morpholino(C2-3)alkyl.

Similarly, if a compound of the present invention comprises an alcohol functional group, a prodrug can be formed by the replacement of the hydrogen atom of the alcohol group with a group such as (C₁-C₆)alkanoyloxymethyl, 1-((C₁-C₆)alkanoyloxy)ethyl, 1-methyl-1- ((C₁-C₆)alkanoyloxy)ethyl, (C₁-C₆)alkoxycarbonyloxymethyl, N-(C₁-C₆)alkoxycarbonyl- aminomethyl, succinoyl, (C₁-C₆)alkanoyl, α-amino(C₁-C₄)alkanoyl, arylacyl and α-aminoacyl, or α-aminoacyl-α-aminoacyl, where each α-aminoacyl group is independently selected from the naturally occurring L-amino acids, -P(O)(OH)₂, -P(O)(O(C₁-C₆)alkyl)₂ or glycosyl (the radical resulting from the removal of a hydroxyl group of the hemiacetal form of a carbohydrate).

The compounds of the present invention may exist in unsolvated as well as solvated forms with pharmaceutical acceptable solvents such as water (hydrate), ethanol, and the like. The present invention contemplates and encompasses both the solvated and unsolvated forms.

It is also possible that compounds of the present invention may exist in different tautomeric forms. All tautomers of compounds of the present invention are contemplated. For example, all of the tautomeric forms of the imidazole moiety are included in this invention. Also, for example, all keto-enol or imine-enamine forms of the compounds are included in this invention.

Those skilled in the art will recognize that the compound names and structures contained herein may be based on a particular tautomer of a compound. While the name or structure for only a particular tautomer may be used, it is intended that all tautomers are encompassed by the present invention, unless stated otherwise.

The present invention also includes isotopically-labelled compounds, which are identical to those recited herein, but for the fact that one or more atoms are replaced by an atom having an atomic mass or mass number different from the atomic mass or mass number usually found in nature. Examples of isotopes that can be incorporated into compounds of the 20 invention include isotopes of hydrogen, carbon, nitrogen, oxygen, phosphorous, fluorine and chlorine, such as ²H, ³H, ¹³C, ¹⁴C, ¹⁵N, ¹⁶0, ¹⁷0, ³¹P, ³²P, ³⁵S, ¹⁸F, and ³⁶Cl.

Compounds of the present invention that contain the aforementioned isotopes and/or other isotopes of other atoms are within the scope of this invention. Certain isotopically- labelled compounds of the present invention, for example those into which radioactive isotopes such as ³H and ¹⁴C are incorporated, are useful in drug and/or substrate tissue distribution assays. Tritiated, i.e., ³H, and carbon-¹⁴, i.e., ¹⁴C, isotopes are particularly preferred for their ease of preparation and detection. Further, substitution with heavier isotopes such as deuterium, i.e., ²H, can afford certain therapeutic advantages resulting from greater metabolic stability, for example increased in vivo half-life or reduced dosage requirements and, hence, may be preferred in some circumstances. Isotopically labelled compounds of this invention can generally be prepared by substituting a readily available isotopically labelled reagent for a non-isotopically labelled reagent.

The residues X and Y are independently chosen from each other and may be the same or different. The residues can be subsituted in ortho, meta or para position at the ring.

In the spirit of the invention, all residues are considered combinable one with each other unless stated otherwise in the definition of the residues. All conceivable subgroups thereof shall be considered disclosed.

In particular preferred embodiments the residues are
X = 2-propynyl, methyl, ethyl, propyl, cyclopropyl orcyclopropylmethyl
Y = H, methyl, ethyl or propyl
Z = aryl, benzyl (single or multiple substituted or unsubstituted)

The most preferred compounds are: 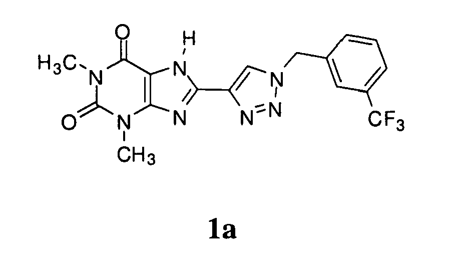 1,3-Dimethyl-8-(1-(3-(trifluoromethyl)benzyl)-1*H*-1,2,3-triazol-4-yl)-1*H*-purine-2,6(3*H*,7*H*)-dione (**1a**) 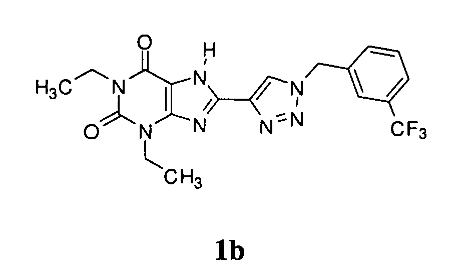 1,3-Diethyl-8-(1-(3-trifluoromethyl)benzyl)-1*H*-1,2,3-triazol-4-yl)-1*H*-purine-2,6,(3*H*,7*H*)-dione (**1b**) 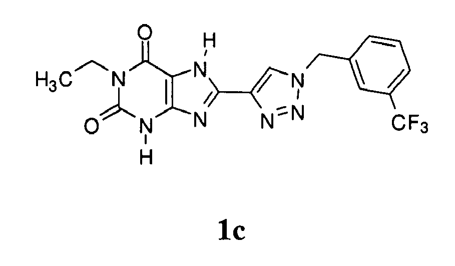 1-Ethyl-8-(1-(3-trifluoromethyl)benzyl)-1*H*-1,2,3-triazol-4-yl)-1*H*-purine-2,6,(3*H*,7*H*)-dione (**1c**) 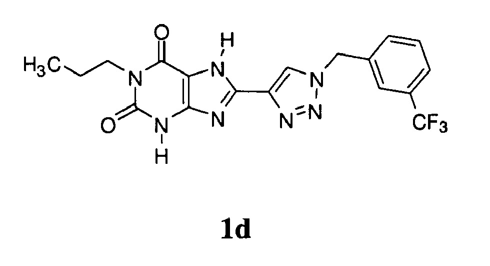 1-Propyl-8-(1-(3-trifluoromethyl)benzyl)-1*H*-1,2,3-triazol-4-yl)-1*H*-purine-2,6,(3*H*,7*H*)-dione (**1d**) 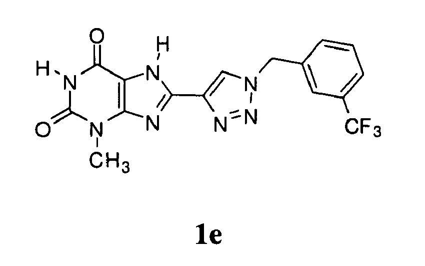 3-Methyl-8-(1-(3-(trifluoromethyl)benzyl)-1*H*-1,2,3-triazol-4-yl)-1*H*-purine-2,6(3*H*,7*H*)-dione (**1e**) 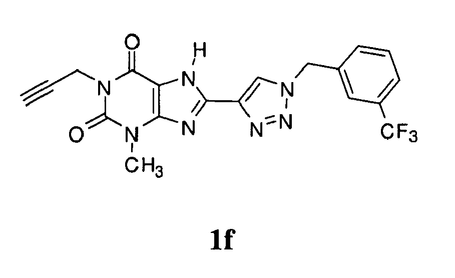 3-Methyl-1-(prop-2-ynyl)-8-(1-(3-(trifluoromethyl)benzyl)-1*H*-1,2,3-triazol-4-yl)-1*H-*purine-2,6,(3*H*,7*H*)-dione (**1f**) 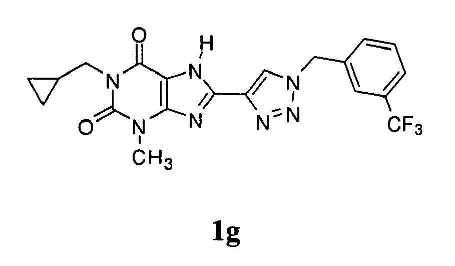 1-(Cyclopropylmethyl)-3-methyl-8-(1-(3-(trifluoromethyl)benzyl)-1*H*-1,2,3-triazol-4-yl)-1*H*-purine-2,6(3*H*,7*H*)-dione (**1g**) 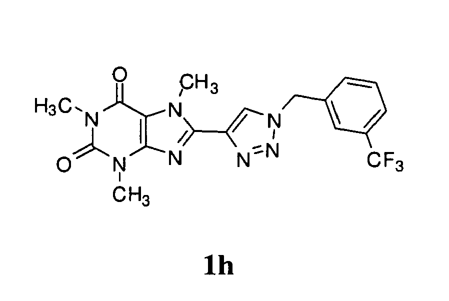 1,3,7-Trimethyl-8-(1-(3-(trifluoromethyl)benzyl)-1*H*-1,2,3-triazol-4-yl)-1*H*-purine-2,6(3*H*,7*H*)-dione (**1h**) 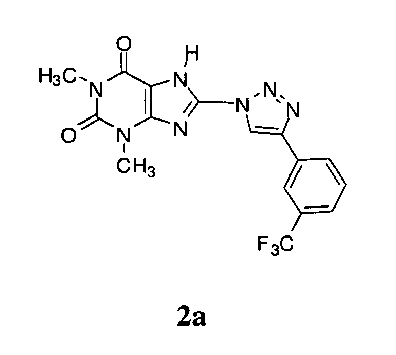 1,3-Dimethyl-8-(4-(3-(trifluoromethyl)phenyl)-1*H*-1,2,3-triazol-1-yl)-1*H*-purine-2,6(3*H*,7*H*)-dione (**2a**) 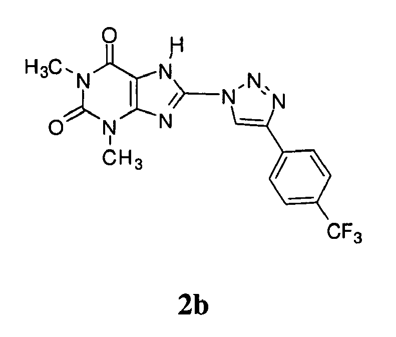 1,3-Dimethyl-8-(4-(4-(trifluoromethyl)phenyl)-1*H*-1,2,3-triazol-1-yl)-1*H*-purine-2,6(3*H*,7*H*)-dione (**2b**) 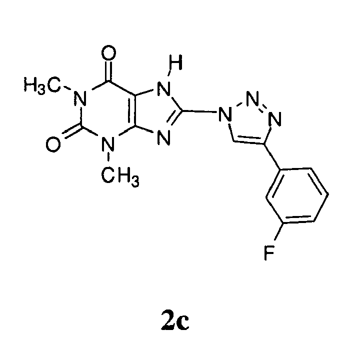 8-(4-(3-Fluorophenyl)-1*H*-1,2,3-triazol-1-yl)-1,3-dimethyl-1*H*-purine-2,6(3*H*,7*H*)-dione (**2c**) 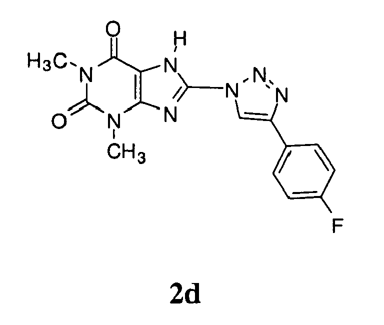 8-(4-(4-Fluorophenyl)-1*H*-1,2,3-triazol-1-yl)-1,3-dimethyl-1*H*-purine-2,6(3*H*,7*H*)-dione (**2d**) 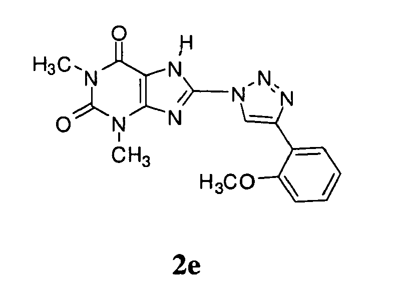 8-(4-(2-Methoxyphenyl)-1*H*-1,2,3-triazol-1-yl)-1,3-dimethyl-1*H*-purine-2,6(3*H*,7*H*)-dione (**2e**)

The invention also relates to physiologically compatible salts or prodrugs of the compounds of general formulae **I** and **II.** The physiologically compatible salts are obtained generally by the reaction of basic compounds of general formulae **I** and **II** with inorganic or organic acids. Hydrochloric acid, sulphuric acid or nitric acid are preferably used as inorganic acids and e.g. formic acid, acetic acid, propionic acid, benzoic acid, ascorbic acid or amino acids are preferably used as organic acids.

### Administration and pharmaceutical composition

The compounds according to the invention can be administered in different ways, e.g. orally, parenterally, cutaneously, subcutaneously, intravenously, by infusion, intramuscularly or rectally. The intravenous or oral administrations are preferred. The compound is given to a patient who needs a therapy for a disease coming under the indication spectrum of the compounds according to the invention over a period to be determined by a physician. The compound can be administered to both humans and other mammals.

The dosage of the compounds according to the invention is determined by the physician on the basis of the patient-specific parameters, such as age, weight, sex, severity of the disease, etc. The dosage is preferably from 0.001 mg/kg to 1000 mg/kg body weight, preferably from 0.01 to 500 mg/kg body weight and most preferably from 0.1 to 100 mg/kg body weight.

Corresponding to the kind of administration, the medicament is suitably formulated, e.g. in the form of solutions or suspensions, simple tablets or dragees, hard or soft gelatine capsules, suppositories, ovules, preparations for injection, which are prepared according to common galenic methods.

The compounds according to the invention can be formulated, where appropriate, together with further active substances and with excipients common in pharmaceutical compositions, e.g. - depending on the preparation to be produced - talcum, gum arabic, lactose, starch, magnesium stearate, cocoa butter, aqueous and nonaqueous carriers, fatty bodies of animal or vegetable origin, paraffin derivatives, glycols (in particular polyethylene glycol), various plasticizers, dispersants or emulsifiers, preservatives.

In order to produce liquid preparations, additives, such as sodium chloride solution, ethanol, sorbitol, glycerine, olive oil, almond oil, propylene glycol or ethylene glycol, can be used.

When solutions for infusion or injection are used, they are preferably aqueous solutions or suspensions, it is possible to produce them prior to use, e.g. from lyophilized preparations which contain the active substance as such or together with a carrier, such as mannitol, lactose, glucose, albuimin and the like. The ready made solutions are sterilized and, where appropriate, mixed with excipients, e.g. with preservatives, stabilizers, emulsifiers, solubilizers, buffers and/or salts for regulating the osmotic pressure. The sterilization can be obtained by sterile filtration using filters having a small pore size according to which the composition can be lyophilized, where appropriate. Small amounts of antibiotics can also be added to ensure the maintenance of sterility.

The invention also relates to pharmaceutical preparations which contain a therapeutically active amount of the active ingredients (compound according to the invention of formulae **I** and **II**) together with organic or inorganic solid or liquid, pharmaceutically compatible carriers which are suited for the intended administration and which interact with the active ingredients without drawbacks.

The invention also relates to processes for producing pharmaceutical preparations in which the compound according to the invention is mixed with a pharmaceutically compatible carrier.

The compounds according to the invention are also suited for combination therapies with previously known active substances for the treatment of the above mentioned diseases. In this connection, surprising synergy effects are to be used to increase the therapeutic effectiveness of the substances according to the invention. The combination may be, on the one hand, to offer a single pharmaceutical composition which contains at least one of the compounds according to the invention in combination with one or more of the below active substances or several preparations which contain one or more of the below active substances are administered to the patient simultaneously or time-staggered with respect to the pharmaceutical composition according to the invention. It is noted that the additional pharmaceutically active compounds/agents may be a traditional small organic chemical molecules or can be macromolecules such as a proteins, antibodies, peptibodies, DNA, RNA or fragments of such macromolecules

Depending on the development of the disease and the underlying symptoms, the ratio between the compounds according to the invention and the other active substances in combination can be 1:10,000 to 10,000:1, preferably 1:1,000 to 1,000:1, most preferably 1:10 to 10:1.

For the substances according to the invention dose-effect curves were established using ELISA data and the EC₅₀ values for every substance were calculated. The EC₅₀ values for the substances according to the invention are between 0.1 nM and 1 µM.

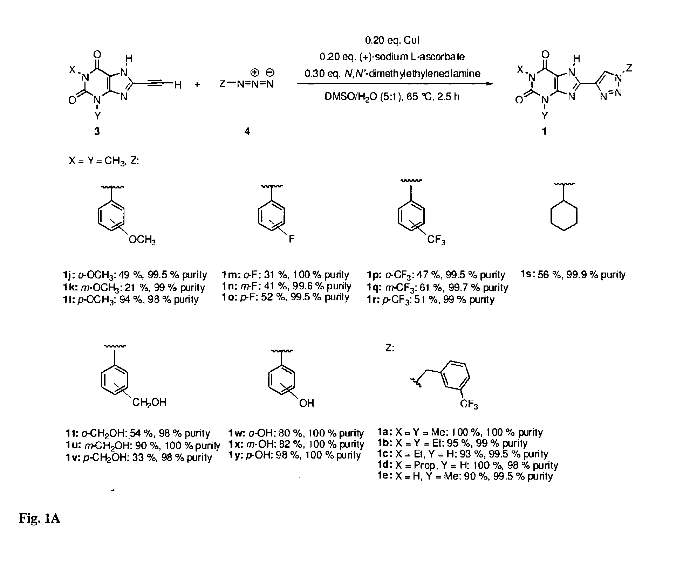

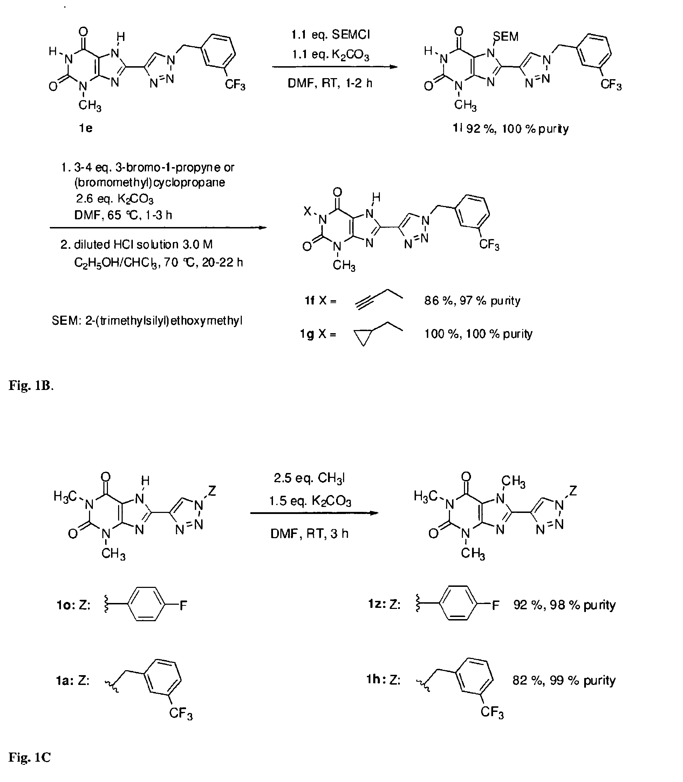

The claimed compounds have been identified to be suitable A_{2B} antagonists by testing their binding affinities to the human adenosine A_{2B} receptor as well as to other adenosine receptor subtypes. In this regard reference is made to Example 51-53, Figure 1-4 and Figure 6-7. In this regard it has to be emphasized that the claimed compounds have an antagonistic effect in the nanomolar concentration range on the A_{2B} receptor.

The compounds according to general formulae **I** and **II** may be synthesized by synthetic methods starting from building blocks that are commercially available.

In a further aspect, the invention relates to methods of preparing compounds of general formulae **I** and **II.**

In a preferred embodiment, the method comprises the preparation of 8-(1*H-*1,2,3-triazol-4-yl)-1*H*-purine-2,6(3*H*,7*H*)-dione derivatives of general formula **I** 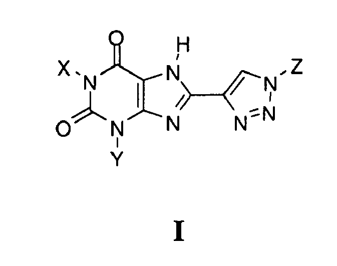 by reacting 8-ethynyl-1*H*-purine-2,6(3*H*,7*H*)-dione derivatives of general formula **III** with azides of general formula **IV.** 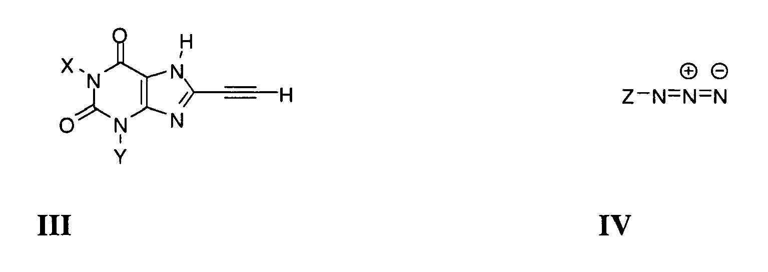 wherein X, Y and Z are defined above.

The invention also relates to processes for the production of the compounds according to the invention.

The processes according to the invention for the production of the compounds of general formula **I** with the above listed meanings of X, Y and Z are characterized by the following procedures:

8-Ethynyl-1*H*-purine-2,6(3*H*,7*H*)-dione derivatives of general formula **III** were reacted with azides of general formula **IV** (Scheme 1).

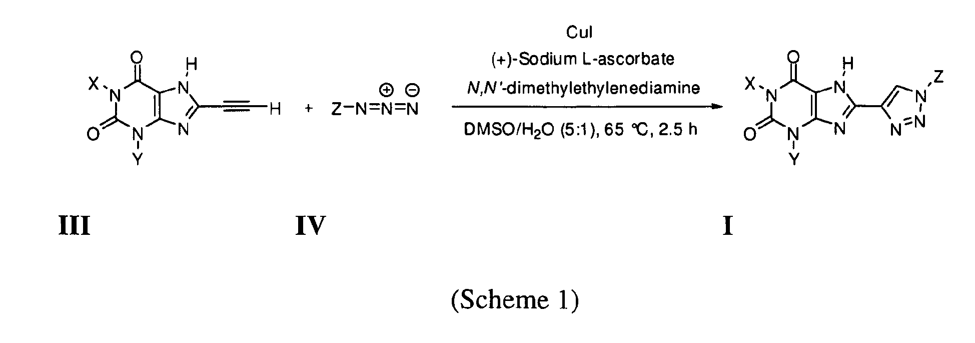

In addition a selectively alkylation of position 7 with 2-(trimethylsilyl)ethoxymethyl chloride (SEMCl) as protective group is possible (Scheme 2).

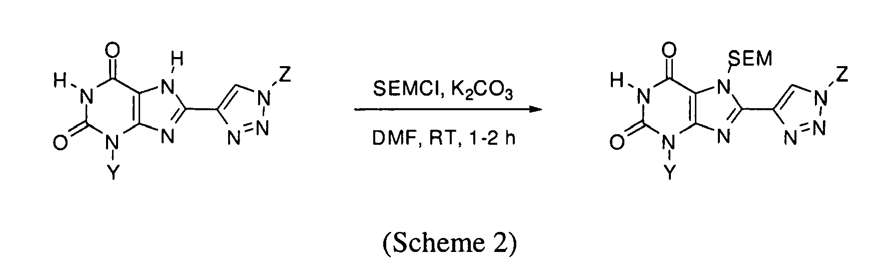

Further alkylation of position 1 and subsequent removal of the protective group under acidic conditions lead to more compounds of general formula **I** (Scheme 3).

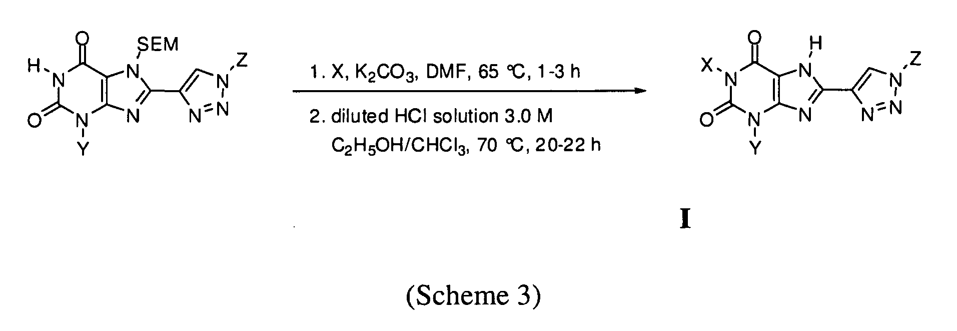

In a second preferred embodiment, the method comprises the preparation of 8-(1*H*-1,2,3-triazol-1-yl)-1*H*-purine-2,6(3*H*,7*H*)-dione derivatives of general formula **II** 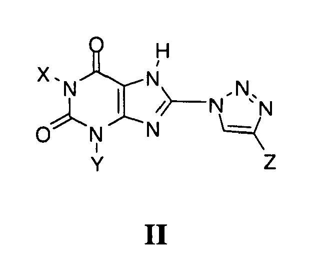 by reacting 8-azido-1*H*-purine-2,6(3*H*,7*H*)-dione derivatives of general formula **V** with alkynes of general formula **VI.** 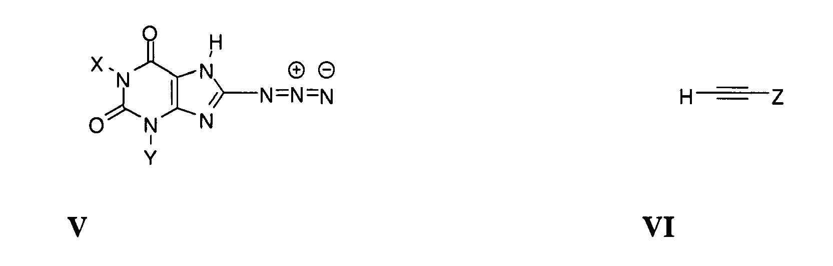 wherein X, Y and Z are defined as above.

The invention also relates to processes for the production of the compounds according to the invention.

The processes according to the invention for the production of the compounds of general formula **II** with the above listed meanings of X, Y and Z are characterized by the following procedures:

8-Azido-1*H*-purine-2,6(3*H*,7*H*)-dione derivatives of general formula **V** were prepared from 8-bromo-1*H*-purine-2,6(3*H*,7*H*)-dione derivatives by alkylation of position 7 with iodomethane, or 2-(trimethylsilyl)ethoxymethyl chloride (SEMCl) as a protective group, respectively, followed by reaction with sodium azide at position 8 and subsequent removal of the protective group under acidic conditions (Scheme 4).

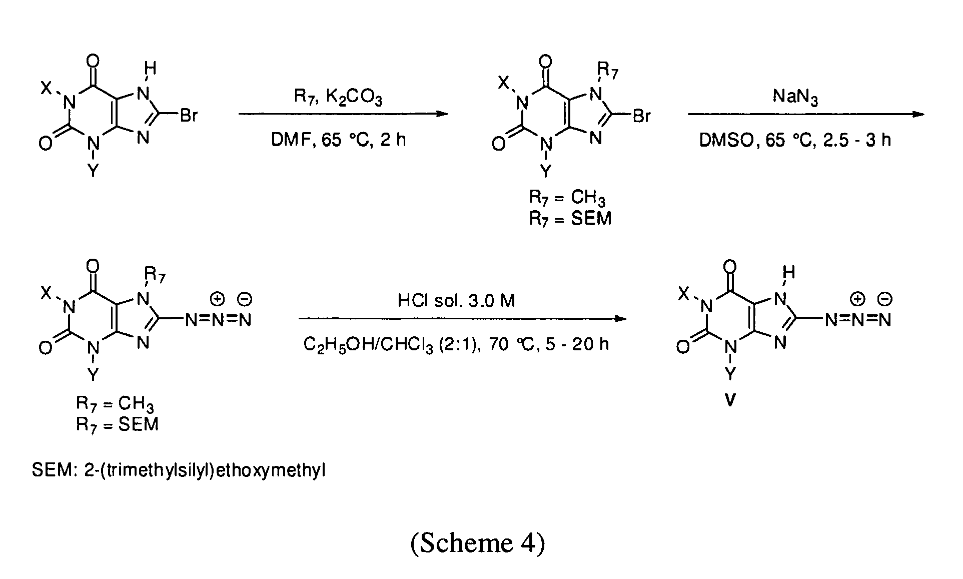

8-Azido-1*H*-purine-2,6(3*H*,7*H*)-dione derivatives of general formula **V** were reacted with azides of general formula **VI** (Scheme 5).

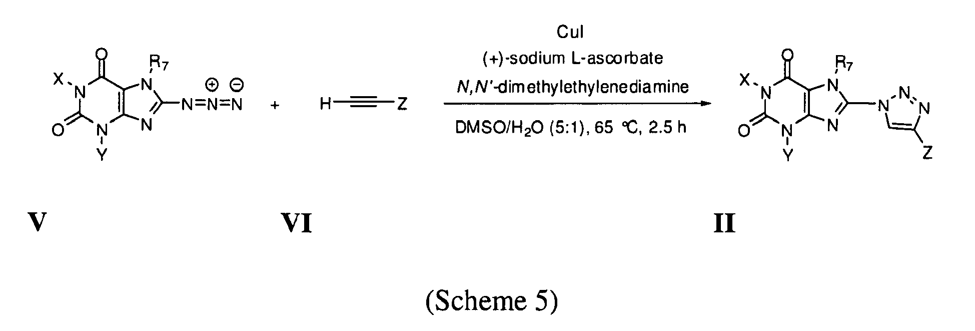

### Tables and Figures

The invention was further described with reference to the figures which show:
- Fig. 1:: Synthesis of 8-(1*H*-1,2,3-triazol-4-yl)-1*H*-purine-2,6(3*H*,7*H*)-dione derivatives **1**
- Fig. 2:: Synthesis of 8-(1*H*-1,2,3-triazol-1-yl)-1*H*-purine-2,6(3*H*,7*H*)-dione derivatives **2**
- Fig. 3:: Concentration-dependent inhibition of [³H]PSB-603 (A_{2B} antagonist radioligand) binding by compound **1a** at the human adenosine A_{2B} receptor
- Fig. 4A:: cAMP assays in HEK cells recombinantly expressing the human A_{2B} receptor
- Fig. 4B:: cAMP assays in CHO cells recombinantly expressing the human adenosine A_{2B} receptor
- Fig. 5:: ELISA assay to investigate the effects of adenosine receptor antagonists
- Fig. 6A:: Effect of adenosine on the TNFα expression in ESdM cells following LPS stimulation
- Fig. 6B:: Effect of NECA on the TNFα expression in ESdM cells following LPS stimulation
- Fig. 7A:: Antagonistic effect of the A_{2B} antagonist **1a**
- Fig. 7B:: Inhibitory potential of the A_{2B} antogonist **1a**
- Fig. 8A:: Antagonistic effect of the A_{2B} antagonist **1b**
- Fig. 8B:: Inhibitory potential of the A_{2B} antogonist **1b**
- Fig 9A:: Antagonistic effect of the A_{2B} antagonist **1d**
- Fig. 9B:: Inhibitory potential of the A_{2B} antogonist **1d**
- Fig. 10A:: Antagonistic effect of the A_{2B} antagonist **1f**
- Fig. 10B:: Inhibitory potential of the A_{2B} antogonist **1f**
- Fig. 11A:: Antagonistic effect of the A_{2B} antagonist **1g**
- Fig. 11B:: Inhibitory potential of the A_{2B} antogonist **1g**.

Table 1: Affinities of selected compounds for rat (rat cortex and striatum) and human adenosine receptors recombinantly expressed in CHO cells. The values are presented as means of Kᵢ values in nM of three separate experiments each run in triplicate. For experimental conditions see Müller et al. (J. Med. Chem. 2009, 52, 3994-4006).
Table 2: Determined EC₅₀ value of the different compounds in ELISA assays. The values are represented as means of several experiments.

More precisely Fig. 1 to 10 show the following:

For synthesis of 8-(1*H*-1,2,3-triazol-4-yl)-1*H*-purine-2,6(3*H*,7*H*)-dione derivatives **1** in a first step, 8-ethynyl-1*H*-purine-2,6(3*H*,7*H*)-dione derivatives **3,** under the reaction conditions of Liang et al. (Synlett 2005, 14, 2209-2213), were reacted with azides **4** in a Huisgen reaction with copper(I) iodide, (+)-sodium L-ascorbate and *N*,*N*'-dimethylethylenediamine in an aqueous medium (see Fig. 1A).

In the case of compound **1f** and **1g** a further synthetic pathway is described as follows: compound **1e** was selectively alkylated similar to a method described by Zablocki, Kalla and Scammells et al. (Bioorg. Med. Chem. Lett. 2002, 12, 3179-3182, Bioorg. Med. Chem. Lett. 2005, 15, 609-612) in position 7 with 2-(trimethylsilyl)ethoxymethyl chloride (SEMCl) as protective group. Dialkylated product received as a by-product (8 %) and could be successfully removed by column chromatography. Then, position 1 was alkylated with 3-bromo-1-propyne, or with (bromomethyl)cyclopropane, respectively. In the last step the protective group was removed under acidic conditions resulting to compounds **1f** and **1g** (see Fig. 1B).
For Alkylation of position 7 in xanthine derivatives 8-(1*H*-1,2,3-Triazol-4-yl)-1*H-*purine-2,6(3*H*,7*H*)-dione derivatives **1** can be alkylated with alkylating reagents, for example iodomethane similar to a described method (Müller et al., Heterocycles, 2002, 57, 871-879) resulting to compounds **1z** and **1h** (see Fig. 1C).

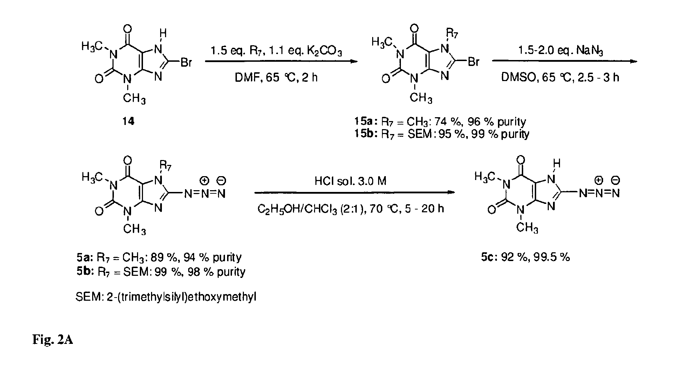

For synthesis of 8-(1-1,2,3-triazol-1-yl)-1H-purine-2,6(3H,7H)-dione derivatives **2** first, 8-azido-1*H*-purine-2,6(3*H*,7*H*)-dione derivative **5a** or **5c** were synthesized from 8-bromo-1*H*-purine-2,6(3*H*,7*H*)-dione derivatives by alkylation with iodomethane (Müller et al., Heterocycles, 2002, 57, 871-879), or 2-(trimethylsilyl)ethoxymethyl chloride at position 7 according to a method previously described by Scammells et al. (Bioorg. Med. Chem. Lett. 2002, 12, 3179-3182) and Kalla et al. (J. Med. Chem. 2006, 49, 3682-3692, Bioorg. Med. Chem. Lett. 2008, 18, 1397-1401), respectively. According to a method previously described by Rybár et al. (Synthesis 1989, 681-683) and Sutherland and Pickard (J. Heterocycl. Chem. 1974, 11, 457-458), a reaction with sodium azide to **5a** or **5b,** followed by hydrolysis of the SEM-protective group, yielded 8-azido-1*H*-purine-2,6(3*H*,7*H*)-dione derivative **5c** (see Fig. 2A).

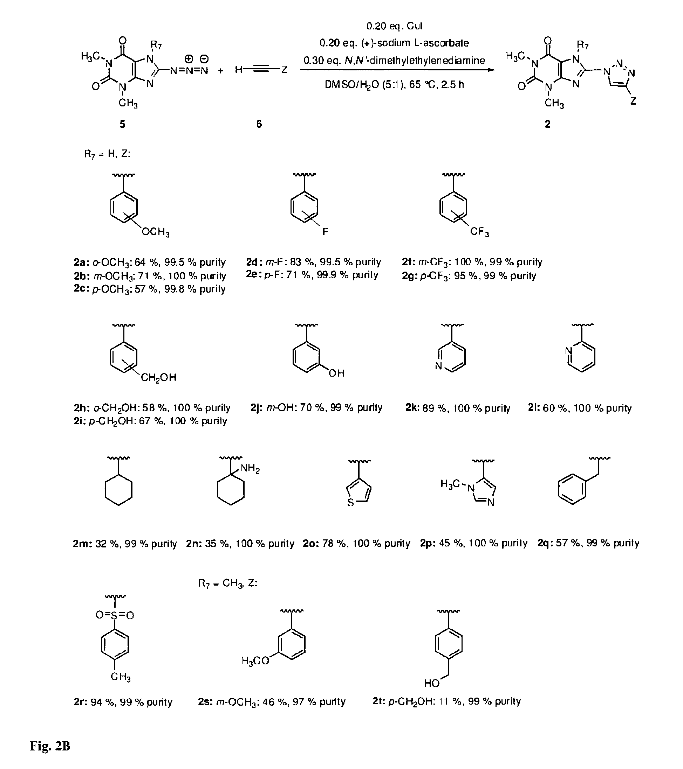

Using the reaction conditions described by Liang et al. (Synlett 2005, 14, 2209-2213), 8-azido-1,3-dimethyl-1*H*-purine-2,6(3*H*,7*H*)-dione (5c), or 8-azido-1,3,7-trimethyl-1*H*-purine-2,6(3*H*,7*H*)-dione (5a), respectively, were reacted with various commercially available alkynes **6** in a Huisgen reaction with copper(I) iodide, sodium L-ascorbate and *N*,*N*'-dimethylethylenediamine in an aqueous medium (see Fig. 2B).

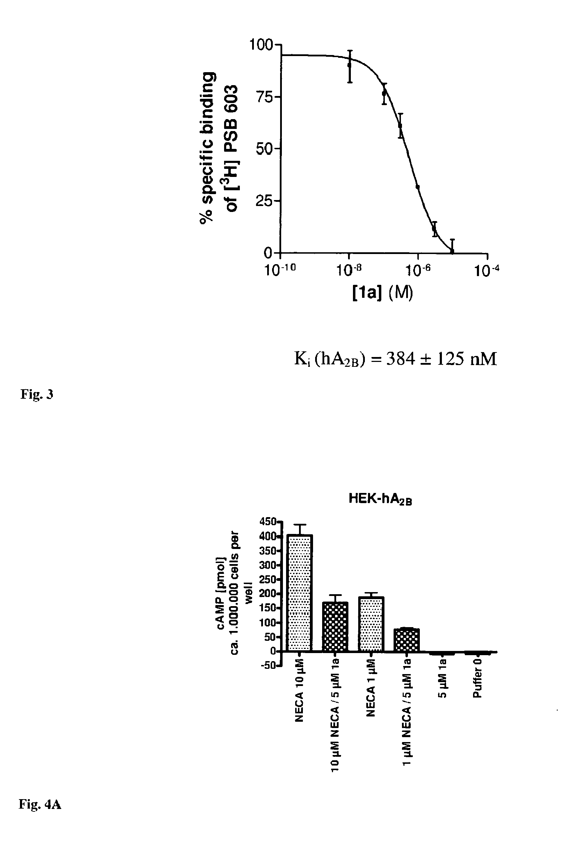

Fig. 3 shows the concentration-dependent inhibition curve of [3H]PSB-603 (A_{2B} antagonist radioligand) binding by compound 1a at the human adenosine A_{2B} receptor. Concentration-inhibition curves were recorded at various concentrations from 0.01-10 µM of three separate experiments each run in triplicate. From each curve, mean values and the Kᵢ value were calculated, which is given in nM ± SEM (n = 3).

Fig. 4A shows cAMP assays in HEK cells recombinantly expressing the human A_{2B} rceptor. NECA was used as an agonist in concentrations of 10 µM and 1 µM in combination with the new antagonist **1a** in a concentration of 5 µM.The A_{2B} agonist NECA leads to a concentration-dependent increase in cAMP formation, which was inhibited by compound **1a**. Compound **1a** itself showed no effect on cAMP levels.

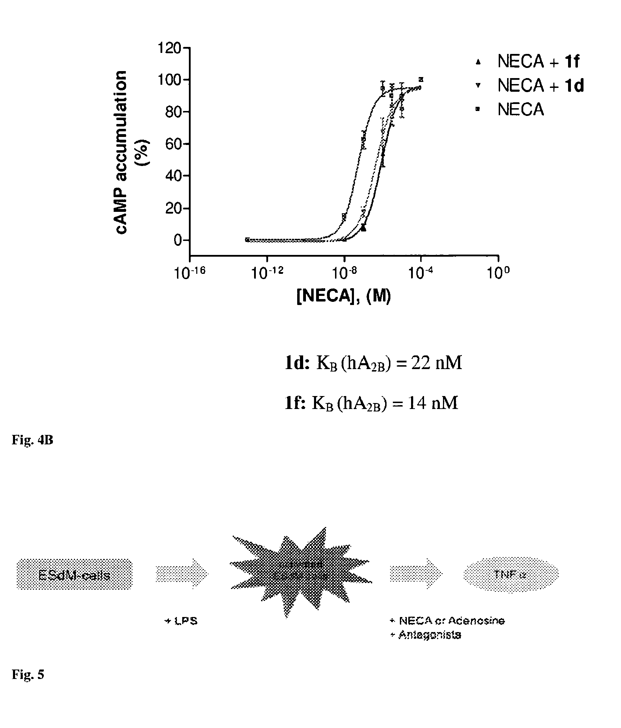

Fig. 4B shows cAMP assays in CHO cells recombinantly expressing the human adenosine A_{2B} receptor. Dose-response curves for the agonist NECA were determined in the absence and in the presence of a single concentration of the new antagonists **1d** and **1f,** using a ∼ 5-fold concentration of the Kᵢ-values. The A_{2B} agonist NECA led to a concentration-dependent increase in cAMP formation, which was inhibited by the new antagonists **1d** and **1f**. The NECA curve shifted to the right by the antagonists indicating competitive antagonism for the human A_{2B} adenosine receptors.

With the ELISA assay in Fig. 5 the effects of adenosine receptor antagonists were investigated. ESdM cells were activated with lipopolysaccharide (LPS) and the adenosine receptors were stimulated with adenosine or the synthetic adenosine receptor agonist NECA, which resulted in an expression of the proinflammatory cytokine TNFα. In the presence of the antagonists to be investigated, the influence on the TNFα expression was analyzed. The cytokine expression was determined in the ELISA.

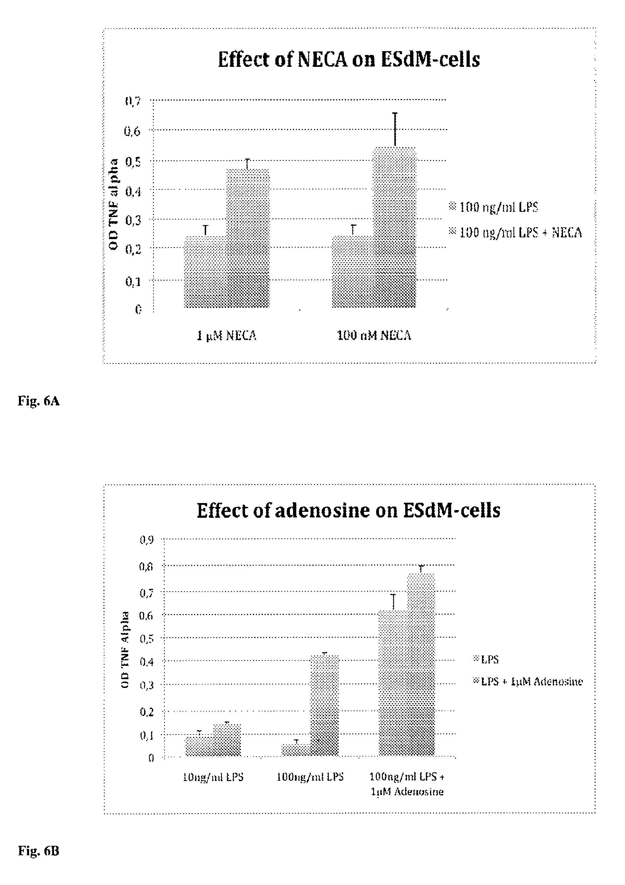

The Effect of adenosine on the TNFα expression in ESdM cells following LPS stimulation is shown in Fig. 6A. ESdM cells are sensitive for the stimulation with 1 µM adenosine. Best results are achieved with a LPS concentration of 100 ng/ml, whereas 500 ng/ml of LPS provide better detectable TNFα values.

The Effect of NECA on the TNFα expression in ESdM cells following LPS stimulation in shown in Fig. 6B. ESdM cells are sensitive for the stimulation with 1 µM and 100 nM of NECA, wherein 100 nM of NECA provide the better detectable TNFα values.

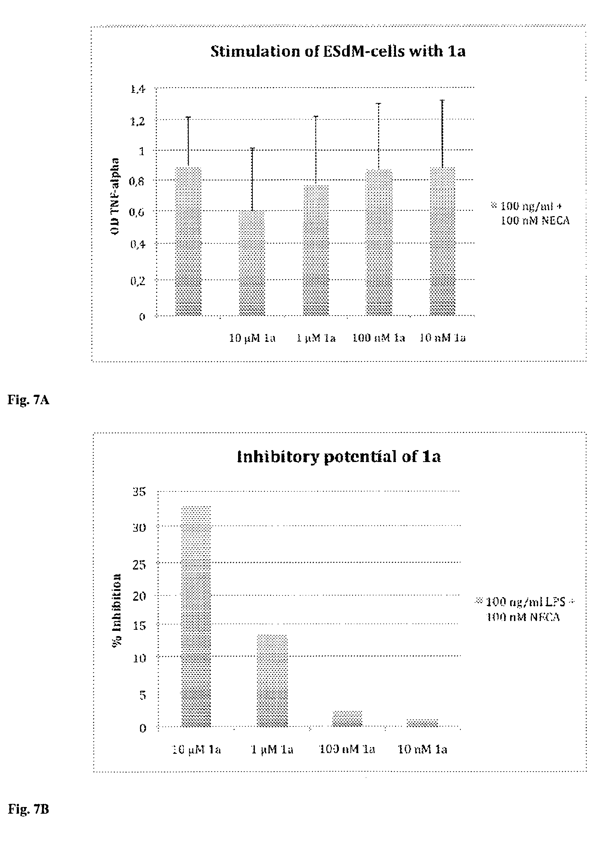

The A_{2B} antagonist **1a** showed an antagonistic effect in the nanomolar range (see Fig. 7A). The determined EC₅₀ concentration amounted to 60 nM. ESdM cells were stimulated with 100 nM of NECA and 100 ng/ml of LPS, and the A_{2B} antagonist **1a** was added to the stimulated cells at various concentrations for 24 h. The supernatants of the cells were investigated for TNFα expression in an ELISA.

Shown in Fig. 7B is the antagonistic effect of different concentrations of the compound **1a** on the A_{2B} receptor in percent.

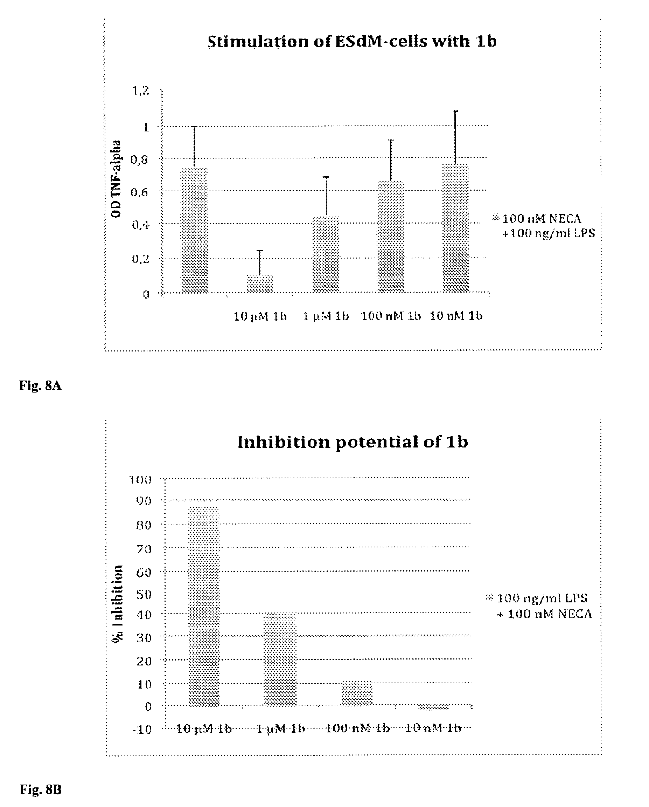

The A_{2B} antagonist **1b** showed an antagonistic effect on LPS-induced TNFα expression in the presence of NECA with an EC₅₀ value in a micromolar range (EC₅₀ = 1.09 µM) (see Fig. 8A).

Shown in Fig. 8B is the inhibitory potential of the A_{2B} antogonist **1b**, in particular the antagonistic effect of different concentrations of the compound **1b** on the A_{2B} receptor in percent.

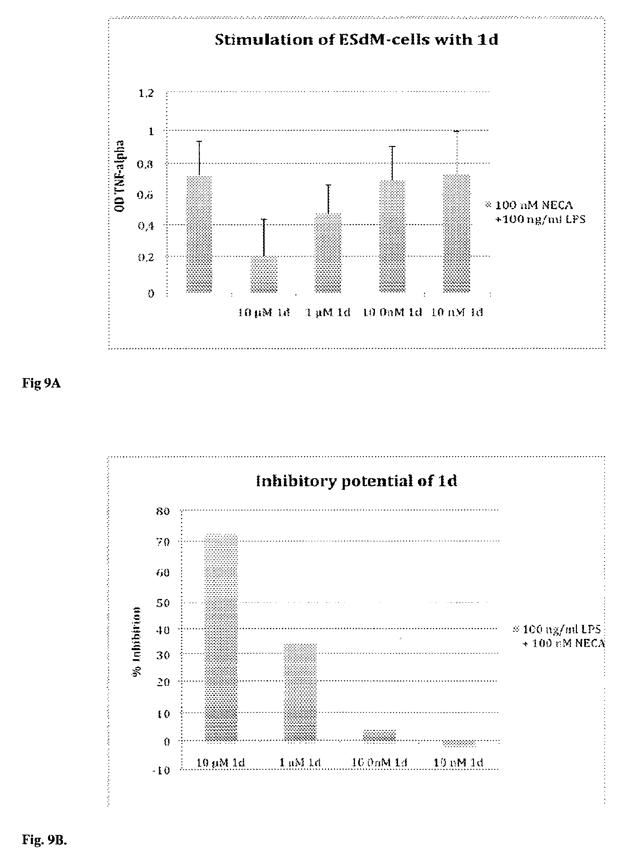

The A_{2B} antagonist 1d showed an antagonistic effect on LPS-induced TNFα expression in the presence of NECA with an EC₅₀ value in a micromolar range (EC₅₀ = 1.11 µM) (see Fig. 9A).

Shown in Fig. 9B is the inhibitory potential of the A_{2B} antogonist **1d,** in particular the antagonistic effect of different concentrations of the compound **1d** on the A_{2B} receptor in percent.

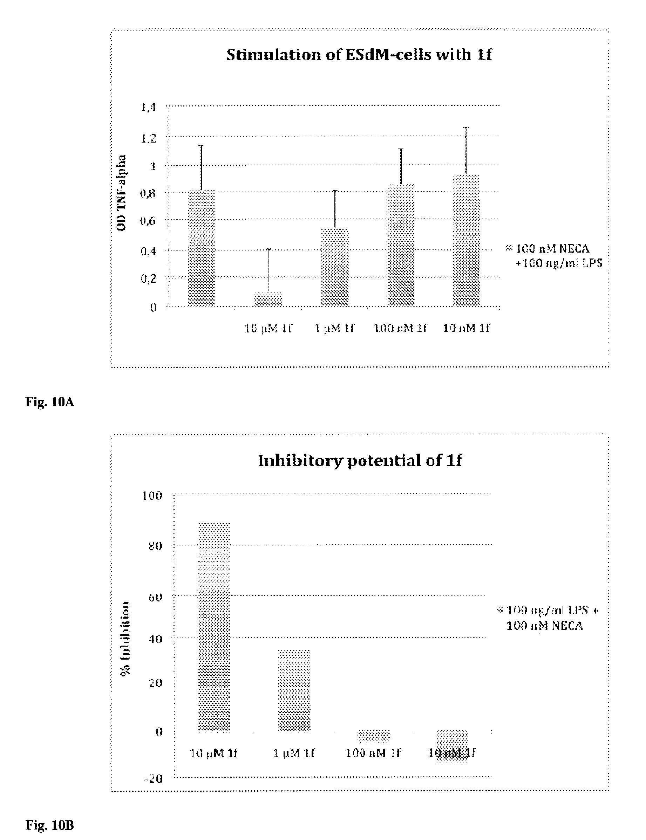

The A_{2B} antagonist **1f** showed an antagonistic effect on LPS-induced TNFα expression in the presence of NECA with an EC₅₀ value in a micromolar range (EC₅₀ = 1.36 µM) (see Fig. 10A).

Fig. 10B shows the inhibitory potential of the A_{2B} antogonist **1f.** in particular the antagonistic effect of different concentrations of the compound **1f** on the A_{2B} receptor in percent.

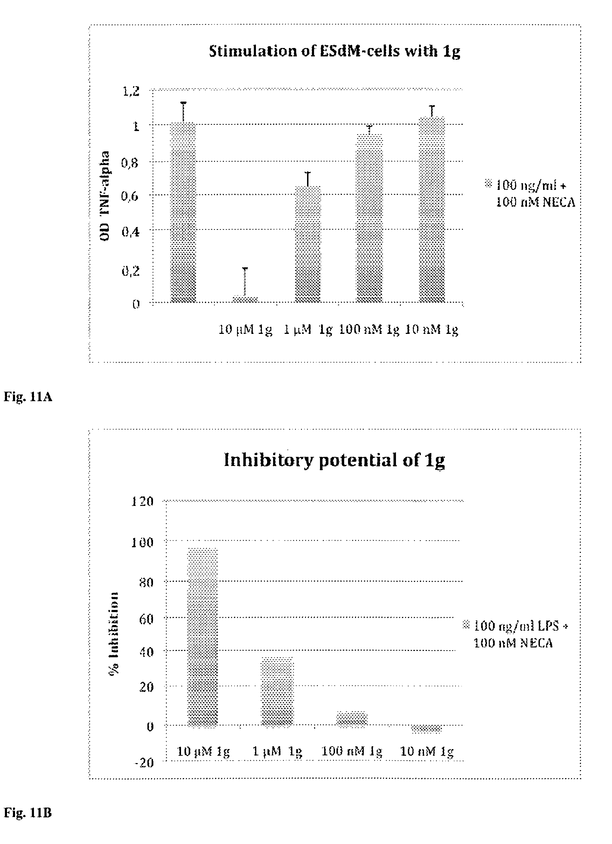

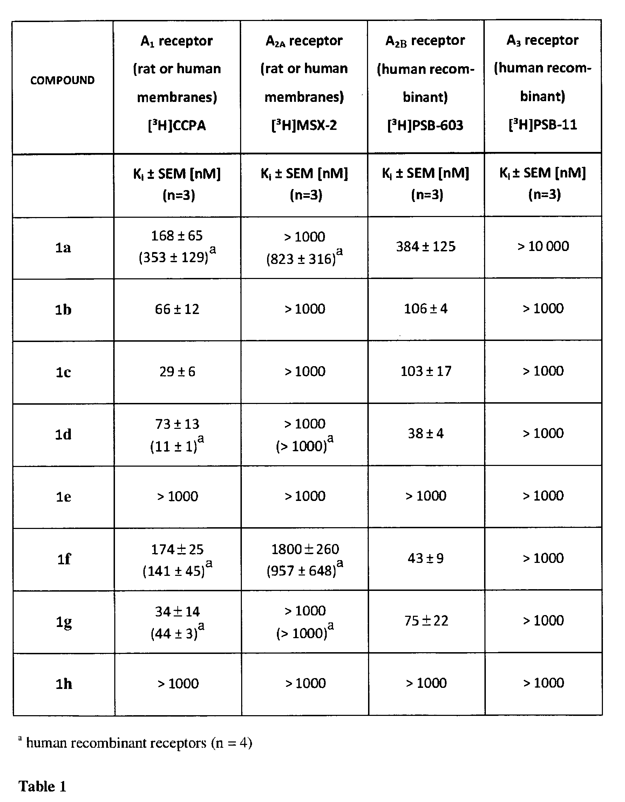

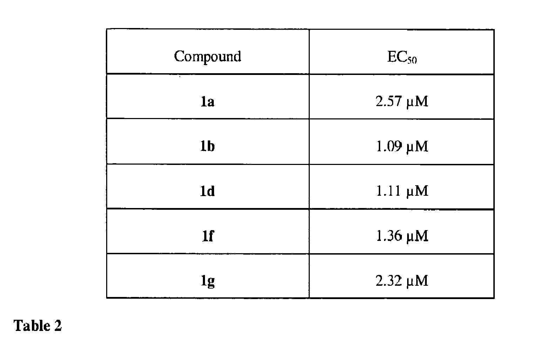

The A_{2B} antagonist **1g** showed an antagonistic effect on LPS-induced TNFα expression in the presence of NECA with an EC₅₀ value in a micromolar range (EC₅₀ = 2.32 µM) (see Fig. 11A).

Fig. 11B shows the inhibitory potential of the A_{2B} antogonist **1g,** in particular the antagonistic effect of different concentrations of the compound **1g** on the A_{2B} receptor in percent.

### Synthetic procedure

**General**: All commercially available reagents and solvents were obtained from various producers (Aldrich, Acros, Fluka, Fluorochem, Alfa Aesar, Merck, VWR, Prolabo, IRIS Biotech, Biesterfeld, Grüssing) and used without further purification or drying. Thin-layer chromatography (TLC) was performed using TLC aluminium sheets with silica gel 60 F₂₅₄ (Merck). Column chromatography was carried out with silica gel 0.040-0.063 mm, 230-400 mesh ASTM (Macherey-Nagel). ESI-mass spectra were obtained on an LC-MS instrument (Applied Biosystems API 2000 LCMS/MS, HPLC Agilent 1100) with an ionisation energy of 70 eV, using the following procedure: the compounds were dissolved at a concentration of 0.5 mg/ml in H₂O : MeOH =9:1, containing 2 mM NH₄CH₃COO. Then, 10 µL of the sample was injected into an HPLC column (Phenomenix Luna 3 µ C18, 50 X 2.00 mm). Elution was performed with a gradient of water : methanol (containing 2 mM NH₄CH₃COO) from 90 : 10 to 0 : 100 for 30 min at a flow rate of 250 µl/min, starting the gradient after 10 min. UV absorption was detected from 200 to 950 nm using a diode array detector. Purity of the compounds was determined at 254 nm. ¹H-, and ¹³C-NMR spectra were obtained on a Bruker Avance 500 MHz NMR spectrometer at 500 MHz (¹H), or 126 MHz (¹³C), respectively. CDCl₃ or DMSO-d₆ were used as solvents as indicated below. Chemical shifts (δ) are reported in parts per million (ppm) relative to the deuterated solvent. Coupling constants (J) are given in Hertz (Hz) and spin multiplicities are given as s (singlet), d (doublet), t (triplet), q (quartet), m (multiplet), br (broad). NMR spectra were recorded at room temperature. FT-IR spectra were recorded on an FT-IR-spectrometer Tensor 27 (Bruker) and analyzed by the software Opus. The samples were measured as potassium bromide pellets. Data are reported as: wave number of absorption (cm⁻¹), intensity of absorption (s = strong, m = medium, w = weak). Melting points were determined on a Büchi B-545 melting point apparatus and are uncorrected. Reactions were monitored by TLC on silica gel 60 F₂₅₄ (Merck) aluminium plates.

### Synthesis of intermediate products for compounds of general formulae I

The preparation of key intermediate products and those not previously described in the literature are described.

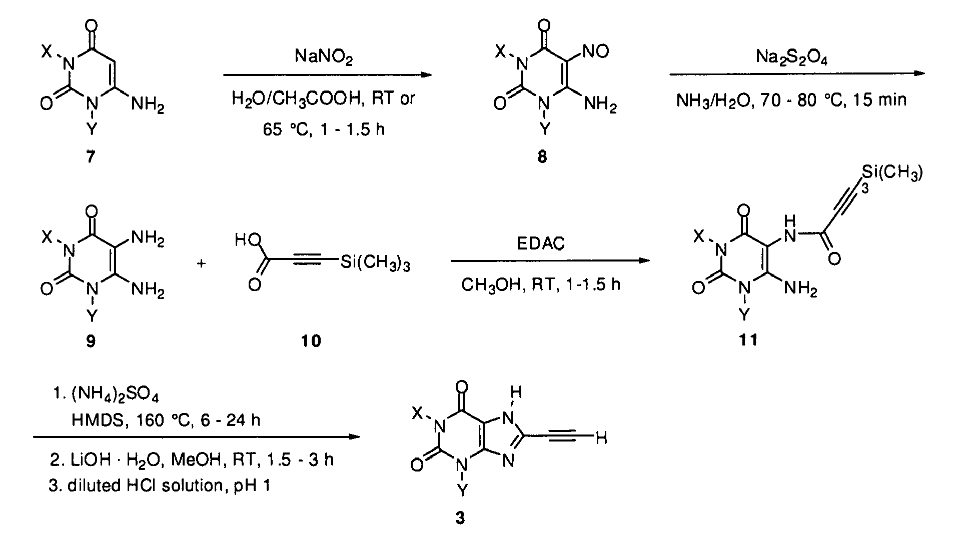

For the synthesis of 8-(1*H*-1,2,3-triazol-4-yl)-1*H*-purine-2,6(3*H*,7*H*)-dione derivatives of formula **1,** first the 8-ethynyl-1*H*-purine-2,6(3*H*,7*H*)-dione derivatives of formula **3** and the azides of formula **4** were prepared.

The 8-ethynyl-1*H*-purine-2,6(3*H*,7*H*)-dione derivatives of formula **2** were synthesized starting from the uracil derivatives **7** by nitrosation at position 5, followed by reduction to the diamines **9** and conversion with 3-(trimethylsilyl)propynoic acid (**10**) to the amide derivatives **11.** After ring closure and hydrolysis of the TMS-protective group, the 8-ethynyl-1*H*-purine-2,6(3*H*,7*H*)-dione derivatives **3** were obtained.

6-Amino-1,3-diethylpyrimidine-2,4(1*H*,3*H*)-dione (**7a**)

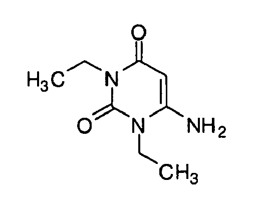

The uracil derivative **7a** was prepared as a white solid in 100 % yield (9.18 g) according to a method previously described by Speer and Raymond (J. Am. Chem. Soc. 1953, 75, 114-115).
Analytical data: **mp** 195 °C. **TLC:** R_{f} = 0.54 (silica gel, dichloromethane). **LC-MS (m/z):** 154.1 [M-C₂H₆]⁻ + 182.1 [M-H]⁻. Purity by **HPLC-UV (254 nm)-ESI-MS:** 99.7 %. **¹H-NMR (500 MHz, DMSO):** δ = 1.01-1.04 (t; ³J_{H,H} = 7.0 Hz, 3H), 1.09-1.12 (t; ³J_{H,H} = 7.0 Hz, 3H), 3.72-3.76 (q; ³J_{H,H} = 7.0 Hz, 2H), 3.81-3.85 (q; ³J_{H,H} = 7.0 Hz, 2H), 4.65 (s; 1H), 6.76 (s; 2H). **¹³C-NMR (126 MHz, DMSO):** δ = 13.0, 13.1, 34.6, 36.8, 75.0, 150.7, 154.0, 160.9.

6-Amino-3-ethylpyrimidine-2,4(1*H*,3*H*)-dione **(7b)**

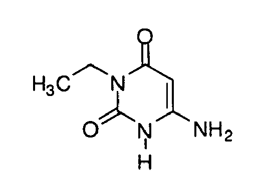

The uracil derivative **7b** was prepared as a dark yellow solid in 92 % yield (11.4 g) according to a method previously described by Müller (Tetrahedron Lett. 1991, 32, 6539-6540).
Analytical data: **mp** 285 °C. **TLC:** R_{f} = 0.64 (silica gel, dichloromethane/methanol 3 : 1). **LC-MS (m/z):** 154.1 [M-H]⁻. Purity by **HPLC-UV (254 nm)-ESI-MS:** 90 %. **¹H-NMR (500 MHz, DMSO):** δ = 1.00-1.03 (t; ³J_{H,H} = 7.0 Hz, 3H), 3.67-3.71 (q; ³J_{H,H} = 7.0 Hz, 2H), 4.52 (s; 1H), 6.30 (s; 2H), 10.74 (s; 1H). **¹³C-NMR (126 MHz, DMSO):** δ = 13.3, 33.5, 74.1, 151.6, 154.4, 162.8.

6-Amino-1,3-dimethyl-5-nitrosopyrimidine-2,4(1*H*,3*H*)-dione **(8a)**

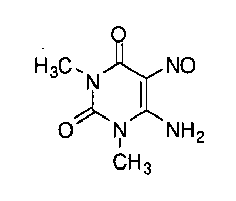

The nitroso derivative **8a** was prepared as a pink solid in 100 % yield (23.9 g) according to a methode previously described by Blicke and Godt Jr. (J. Am. Chem. Soc. 1954, 76, 2798-2800).
Analytical data: **mp** 251 °C. **TLC:** R_{f} = 0.76 (silica gel, methanol). **LC-MS (m/z):** 185.3 [M+H]⁺ + 156.1 [M-NO+2H]⁻ + 183.4 [M-H]⁻. Purity by **HPLC-UV (254 nm)-ESI-MS:** 99.9 %. **¹H-NMR (500 MHz, DMSO):** δ = 3.25 (s; 3H), 3.27 (s; 3H), 9.04 (s; 1H), 12.95 (s; 1H). **¹³C-NMR (126 MHz, DMSO):** δ = 27.7, 28.5, 139.0, 146.0, 149.3, 160.1.

6-Amino-3-ethyl-5-nitrosopyrimidine-2,4(1*H*,3*H*)-dione **(8c)**

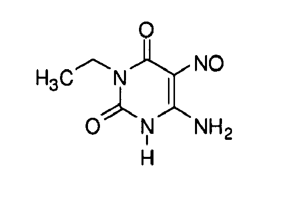

The nitroso derivative **8c** was prepared as a yellow solid in 85 % yield (5.09 g) according to a method described by Müller (Synthesis 1993, 125-128).
Analytical data: **mp** > 400 °C. **TLC:** R_{f} = 0.56 (silica gel, dichloromethane/methanol 3 : 1). **LC-MS (m/z):** 139.9 [M-C₂H₅-NH₂]⁻ + 155.9 [M-C₂H₅]⁻ + 183.1 [M-H]⁻. Purity by **HPLC-UV (254 nm)-ESI-MS:** 100 %. **¹H-NMR (500 MHz, DMSO):** δ = 1.15-1.17 (t; ³J_{H,H} = 7.1 Hz, 3H), 3.86-3.90 (q; ³J_{H,H} = 7.1 Hz, 2H), 7.94 (s; 1H), 11.43 (s; 1H). **¹³C-NMR (126 MHz, DMSO):** δ = 12.9, 34.8, 139.7, 148.6, 160.7.

5,6-Diamino-1,3-dimethylpyrimidine-2,4(1*H*,3*H*)-dione **(9a)**

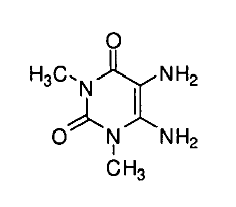

The diamine derivative **9a** was prepared as a light yellow solid in 95 % yield (4.53 g) according to a method described by Müller (Synthesis 1993, 125-128).
Analytical data: **TLC:** R_{f} = 0.33 (silica gel, dichloromethane/methanol 7 : 1). **¹H-NMR (500 MHz, DMSO):** δ = 3.13 (s; 3H), 3.29 (s; 3H), 6.12 (s; 2H). **¹³C-NMR (126 MHz, DMSO):** δ = 27.4, 29.6, 95.9, 144.5, 149.6, 158.8.

5,6-Diamino-3-ethylpyrimidine-2,4(1*H*,3*H*)-dione **(9c)**

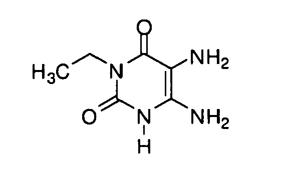

The diamine derivative **9c** was prepared as a light yellow solid in 97 % yield (1.78 g) according to a method described by Müller (Synthesis 1993, 125-128).
Analytical data: **TLC:** R_{f}= 0.34 (silica gel, dichloromethane/methanol 3 : 1). **¹H-NMR (500 MHz, DMSO):** δ = 1.02-1.05 (t; ³J_{H,H} = 7.0 Hz, 3H), 3.72-3.76 (q; ³J_{H,H} = 7.0 Hz, 2H), 5.54 (s; 2H). **¹³C-NMR (126 MHz, DMSO):** δ = 13.2, 34.3, 95.4, 142.2, 148.7, 159.9.

N-(6-Amino-1,3-dimethyl-2,4-dioxo-1,2,3,4-tetrahydropyrimidin-5-yl)-3-(trimethylsilyl)propiolamide (**11a**)

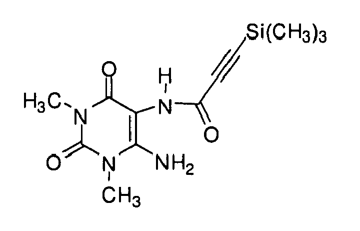

The amide derivative **11a** was prepared as an ocher solid in 80 % yield (5.98 g) according to a method described by Müller et al. and Hildebrand et al. (PCT Int. Appl. 2008, WO 2008077557 A1, Eur. Pat. Appl. 2008, EP 1939197 A1).
Analytical data: **mp** 230 °C. **TLC:** R_{f} = 0.56 (silica gel, dichloromethane/methanol 7 : 1). **LC-MS (m/z):** 295.1 [M+H]⁺ + 179.9 [M-C≡C-Si(CH₃)₃-OH]- + 195.1 [M-C≡C-Si(CH₃)₃-H]⁻ + 220.8 [M-Si(CH₃)₃]⁻ + 236.1 [M-Si(CH₃)₂]⁻ + 293.0 [M-H]⁻. Purity by **HPLC-UV (254 nm)-ESI-MS:** 85 %. **¹H-NMR (500 MHz, DMSO):** δ = 0.04 (s; 3H), 0.25 (s; 9H), 3.10 (s; 3H), 3.29 (s; 3H), 6.72 (s; 2H), 9.05 (s; 1H). **¹³C-NMR (126 MHz, DMSO):** δ = - 1.1, - 0.8, 27.4, 29.8, 85.7, 87.9, 89.7, 92.3, 98.1, 99.9, 150.3, 151.9, 152.3, 153.0, 157.0, 158.6, 159.8.

N-(6-Amino-1,3-diethyl-2,4-dioxo-1,2,3,4-tetrahydropyrimidin-5-yl)-3-(trimethylsilyl)propiolamide (**11b**)

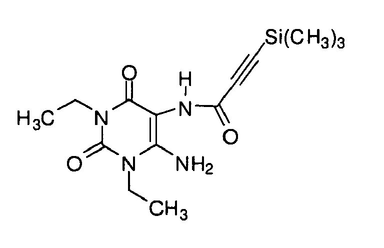

The amide derivative **11b** was prepared as a brown solid in 100 % yield (4.56 g) according to a method described by Müller et al. and Hildebrand et al. (PCT Int. Appl. 2008, WO 2008077557 A1, Eur. Pat. Appl. 2008, EP 1939197 A1).
Analytical data: **mp** 124 °C. **TLC:** R_{f} = 0.50 (silica gel, dichloromethane/methanol 7 : 1). **LC-MS (m/z):** 305.4 [M-17.0]⁺ + 323.3 [M+H]⁺ + 345.3 [M+Na]⁺+ 151.9 [M-CO-C≡C-Si(CH₃)₃-C₂H₅-NH₂-3H]⁻ + 195.1 [M-CO-C≡C-Si(CH₃)₃-2H]⁻ + 223.1 [M-Si(CH₃)₃-C₂H₂]⁻ + 250.3 [M-Si(CH₃)₃+H]⁻ + 293.1 [M-C₂H₅]⁻ + 321.1 [M-H]⁻. Purity by **HPLC-UV (254 nm)-ESI-MS:** 94 %. **¹H-NMR (500 MHz, DMSO):** δ = 0.00 (s; 3H), 0.04 (s; 5H), 0.05 (s; 2H), 0.23 (s; 9H), 1.02-1.07 (m; 6H), 1.08-1.12 (m; 6H), 3.74-3.78 (q; ³J_{H,H} = 7.0 Hz, 2H), 3.86-3.90 (q; ³J_{H,H} = 7.0 Hz, 2H), 6.72 (s; 2H), 9.01 (s; 1H). **¹³C-NMR (126 MHz, DMSO):** δ = - 1.0, - 0.8, 13.0, 13.1, 35.2, 37.5, 78.9, 85.8, 87.9, 89.7, 91.8, 98.0, 99.9, 149.6, 151.1, 152.2, 152.3, 157.0, 158.2, 159.4.

N-(6-Amino-3-ethyl-2,4-dioxo-1,2,3,4-tetrahydropyrimidin-5-yl)-3-(trimethylsilyl)propiolamide (**11c**)

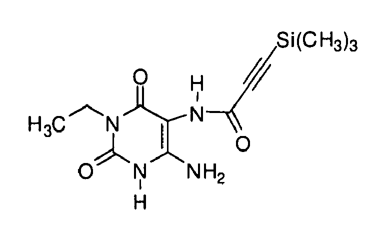

The amide derivative **11c** was prepared as a white solid in 69 % yield (2.00 g) according to a method described by Müller et al. and Hildebrand et al. (PCT Int. Appl. 2008, WO 2008077557 A1, Eur. Pat. Appl. 2008, EP 1939197 A1).
Analytical data: **mp** 264 °C. **TLC:** R_{f} = 0.46 (silica gel, dichloromethane/methanol 7 : 1). **LC-MS (m/z):** 295.0 [M+H]⁺ + 151.9 [M-CO-C≡C-Si(CH₃)₃-NH-2H]⁻ + 195.1 [M-C≡C-Si(CH₃)₃-2H]⁻ + 222.1 [M-Si(CH₃)₃+H]⁻ + 293.1 [M-H]⁻. Purity by **HPLC-UV (254 nm)-ESI-MS:** 99 %. **¹H-NMR (500 MHz, DMSO):** δ = 0.08 (s; 3H), 0.24 (s; 6H), 1.02-1.06 (m; ³J_{H,H} = 7.0 Hz, 3H), 3.68-3.73 (m; ³J_{H,H} = 7.0 Hz, 2H), 6.09 (s; 2H), 9.03 (s; 1H), 10.43 (s; 1H). **¹³C-NMR (126 MHz, DMSO):** δ = - 1.0, - 0.9, 13.1, 13.2, 34.3, 85.4, 87.4, 89.8, 92.3, 98.0, 99.7, 149.4, 150.1, 151.3, 152.0, 156.9, 159.8, 160.9.

*N*-(6-Amino-2,4-dioxo-3-propyl-1,2,3,4-tetrahydropyrimidin-5-yl)-3-(trimethylsilyl)propiolamide (**11d**)

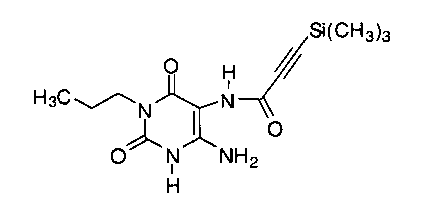

The amide derivative **11d** was prepared as a white solid in 79 % yield (2.41 g) according to a method described by Müller et al. and Hildebrand et al. (PCT Int. Appl. 2008, WO 2008077557 A1, Eur. Pat. Appl. 2008, EP 1939197 A1).
Analytical data: **mp** 306 °C. **TLC:** R_{f} = 0.56 (silica gel, dichloromethane/methanol 7 : 1). **LC-MS (m/z):** 149.9 [M-158.5]⁻ + 165.9 [M-CO-C≡C-Si(CH₃)₃-NH₃]⁻ + 209.1 [M-Si(CH₃)₃-C₂H₃]⁻ + 235.1 [M-Si(CH₃)₃+H]⁻ + 307.4 [M-H]⁻ + 309.3 [M+H]⁺. Purity by **HPLC-UV (254 nm)-ESI-MS:** 98 %. **¹H-NMR (500 MHz, DMSO):** δ = 0.08 (s; 3H), 0.24 (s; 6H), 0.80-0.85 (q; ³J_{H,H} = 7.4 Hz, 3H), 1.44-1.54 (m; ³J_{H,H} = 5.3 Hz, ³J_{H,H} = 7.4 Hz, 2H), 3.61-3.64 (m; ³J_{H,H} = 6.0 Hz, ³J_{H,H} = 7.4 Hz, 2H), 6.08 (s; 1H), 9.03 (s; 1H), 10.41 (s; 1H).**¹³C-NMR (126 MHz, DMSO):** δ = - 1.0, - 0.9, 11.0, 11.1, 20.8, 40.8, 85.3, 87.3, 89.8, 92.3, 98.0, 99.7, 149.6, 150.1, 151.3, 152.0, 156.9, 160.0, 161.1.

N-(6-Amino-1-methyl-2,4-dioxo-1,2,3,4-tetrahydropyrimidin-5-yl)-3-(trimethylsilyl)propiolamide (**11e**)

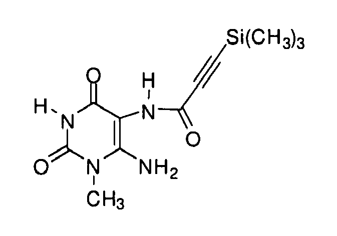

The amide derivative **11e** was prepared as a light yellow solid in 37 % yield (3.72 g) according to a method described by Müller et al. and Hildebrand et al. (PCT Int. Appl. 2008, WO 2008077557 A1, Eur. Pat. Appl. 2008, EP 1939197 A1).
Analytical data: **mp** 309 °C. **TLC:** R_{f} = 0.38 (silica gel, dichloromethane/methanol 7 : 1). **LC-MS (m/z):** 181.3 [M-C≡C-Si(CH₃)₃]⁻ + 207.1 [M-Si(CH₃)₃]⁻ + 236.3 [M-(CH₃)₃+H]⁻ + 279.5 [M-H]⁻ + 263.4 [M-NH₂-H]⁺ + 281.5 [M+H]⁺. Purity by **HPLC-UV (254 nm)-ESI-MS:** 100 %. **¹H-NMR (500 MHz, DMSO):** δ = 0.08 (s; 3H), 0.24 (s; 9H), 3.21 (s; 3H), 6.69 (s; 2H), 8.97 (s; 1H), 10.56 (s; 1H). **¹³C-NMR (126 MHz, DMSO):** δ = - 1.0, - 0.8, 28.9, 28.9, 85.9, 88.0, 89.7, 92.3, 98.2, 99.9, 149.9, 152.2, 153.1, 154.2, 157.0, 159.2, 160.3.

8-Ethynyl-1,3-dimethyl-1*H*-purine-2,6(3*H*,7*H*)-dione **(3a)**

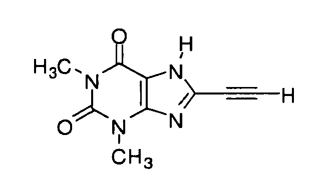

The 8-ethynyl-1*H*-purine-2,6(3*H*,7*H*)-dione derivative **3a** was prepared as a light green solid in 87 % yield (1.52 g) according to a method described by Müller et al. and Hildebrand et al. (PCT Int. Appl. 2008, WO 2008077557 A1, Eur. Pat. Appl. 2008, EP 1939197 A1).
Analytical data: **mp** 338 °C. **TLC:** R_{f} = 0.61 (silica gel, dichloromethane/methanol 9 : 1). **LC-MS (m/z):** 188.0 [M-2H-CH₃]⁻ + 203.1 [M-H]⁻. **Purity by HPLC-UV (254 nm)-ESI-MS:** 99.5 %. **¹H-NMR (500 MHz, DMSO):** δ = 3.22 (s; 3H), 3.40 (s; 3H), 4.70 (s; 1H). **¹³C-NMR (126 MHz, DMSO):** δ = 27.7, 29.6, 73.6, 84.1, 107.4, 131.8, 147.1, 150.9, 153.8.

1,3-Diethyl-8-ethynyl-1*H*-purine-2,6(3*H*,7*H*)-dione (**3b**)

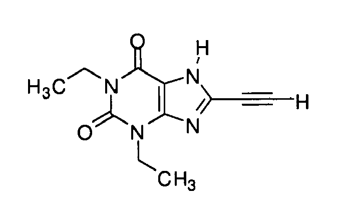

The 8-ethynyl-1H-purine-2,6(3H,7H)-dione derivative **3b** was prepared as an ocher solid in 97 % yield (3.08 g) according to a method described by Müller et al. and Hildebrand et al. (PCT Int. Appl. 2008, WO 2008077557 A1, Eur. Pat. Appl. 2008, EP 1939197 A1).
Analytical data: **mp** 254 °C. **TLC:** R_{f} = 0.55 (silica gel, dichloromethane/methanol 7 : 1). **LC-MS (m/z):** 158.9 [M-73.3]⁻ + 174.9 [M-2C₂H₅]⁻ + 203.1 [M-CH₂]⁻ + 231.4 [M-H]⁻. Purity by **HPLC-UV (254 nm)-ESI-MS:** 93 %. **¹H-NMR (500 MHz, DMSO):** δ = 1.10-1.13 (t; ³J_{H,H} = 7.0 Hz, 3H), 1.20-1.22 (t; ³J_{H,H} = 7.1 Hz, 3H), 3.89-3.93 (q; ³J_{H,H} = 7.0 Hz, 2H), 3.97-4.01 (q; ³J_{H,H} = 7.1 Hz, 2H), 4.71 (s; 1H), 14.28 (s; 1H). **¹³C-NMR (126 MHz, DMSO): δ** = 12.9, 12.9, 35.7, 38.0, 73.6, 84.2, 132.0, 146.6, 149.9, 153.4.

1-Ethyl-8-ethynyl-1H-purine-2,6(3*H*,7*H*)-dione **(3c)**

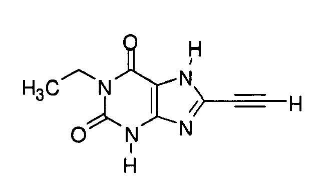

The 8-ethynyl-1*H*-purine-2,6(3*H*,7*H*)-dione derivative **3c** was prepared as a light yellow solid in 78 % yield (0.55 g) according to a method described by Müller et al. and Hildebrand et al. (PCT Int. Appl. 2008, WO 2008077557 A1, Eur. Pat. Appl. 2008, EP 1939197 A1).
Analytical data: **mp >** 400 °C. **TLC:** R_{f} = 0.46 (silica gel, dichloromethane/methanol 7 : 1). **LC-MS (m/z):** 174.9 [M-C₂H₅]⁻ + 203.1 [M-H]⁻. Purity by **HPLC-UV (254 nm)-ESI-MS:** 98 %. **¹H-NMR (500 MHz, DMSO):** δ=1.08-1.09 (t; ³J_{H,H} = 5.8 Hz, 3H), 3.85-3.86 (q; ³J_{H,H} = 6.3 Hz, 2H), 4.66 (s; 1H), 11.87 (s; 1H), 14.10 (s; 1H). **¹³C-NMR (126 MHz, DMSO):** δ = 12.9, 34.8, 73.6, 83.8, 131.9, 146.1, 150.4, 154.0.

8-Ethynyl-1-propyl-1*H*-purine-2,6(3*H*,7*H*)-dione (**3d**)

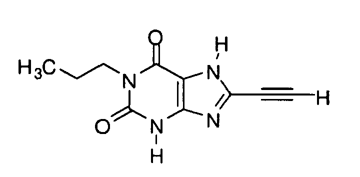

The 8-ethynyl-1*H*-purine-2,6(3*H*,7*H*)-dione derivative **3d** was prepared as a light brown solid in 88 % yield (0.62 g) according to a method described by Müller et al. and Hildebrand et al. (PCT Int. Appl. 2008, WO 2008077557 A1, Eur. Pat. Appl. 2008, EP 1939197 A1).
Analytical data: **mp** 390 °C. **TLC:** R_{f} = 0.37 (silica gel, dichloromethane/methanol 7: 1). **LC-MS (m/z):** 174.9 [M-C₃H₇]⁻ + 217.1 [M-H]⁻. Purity by **HPLC-UV (254 nm)-ESI-MS:** 99.7 %. **¹H-NMR (500 MHz, DMSO):** δ=0.84-0.87 (t; ³J_{H,H} = 7.5 Hz, 3H), 1.51-1.58 (m; ³J_{H,H} = 7.4 Hz, 2H), 3.76-3.79 (m; ³J_{H,H} = 6.0 Hz, ³J_{H,H} = 7.4 Hz, 2H), 4.65 (s; 1H), 11.86 (s; 1H), 14.08 (s; 1H). **¹³C-NMR (126 MHz, DMSO):** δ = 11.0, 20.7, 41.3, 73.7, 83.9, 132.0, 146.3, 150.7, 154.3.

8-Ethynyl-3-methyl-1*H*-purine-2,6(3*H*,7*H*)-dione (**3e**)

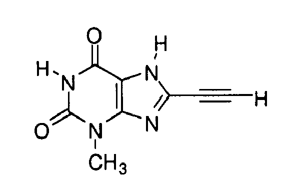

The 8-ethynyl-1*H*-purine-2,6(3*H*,7*H*)-dione derivative **3e** was prepared as a rose solid in 64 % yield (0.62 g) according to a method described by Müller et al. and Hildebrand et al. (PCT Int. Appl. 2008, WO 2008077557 A1, Eur. Pat. Appl. 2008, EP 1939197 A1).
Analytical data: **mp** > 400 °C. **TLC:** R_{f} = 0.44 (silica gel, dichloromethane/methanol 9.5 : 0.5). **LC-MS (m/z):** 174.1 [M-CH₃-H]⁻ + 189.1 [M-H]⁻. Purity by **HPLC-UV (254 nm)-ESI-MS:** 96 %. **¹H-NMR (500 MHz, DMSO):** δ = 3.33 (s; 3H), 4.69 (s; 1H), 11.19 (s; 1H), 14.19 (s; 1H). **¹³C-NMR (126 MHz, DMSO):** δ = 28.7, 73.6, 84.1, 107.7, 131.8, 148.7, 150.8, 154.0.

The azides of formula **4** were synthesized by reacting the corresponding anilines **12** with sodium nitrite and sodium azide under acidic conditions according to a method previously described by Moro and Tron et al. (Chem. Med. Chem. 2007, 2, 437).

General synthetic procedure for the preparation of azides of formula **4** 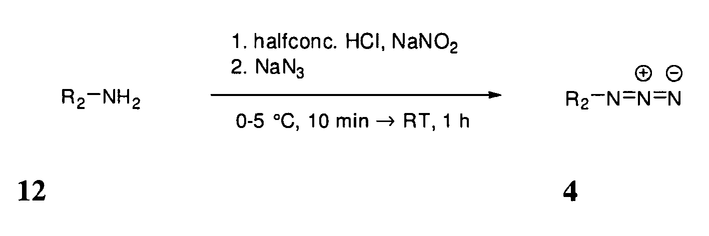 In a 50 mL round bottom flask with magnetic stirrer and reflux condenser, (10.0 mmol, 1.0 eq.) aniline derivative of formula **12** was suspended in 5 mL of semi-concentrated hydrochloric acid with cooling in an ice bath. After a short time, 0.69 g (10.0 mmol, 1.0 eq.) of sodium nitrite, dissolved in 10 mL water, was added dropwise. After stirring for 5 min, 0.78 g (12.0 mmol, 1.0 eq.) of sodium azide, dissolved in 10 mL water, was added dropwise. After 5 min under reaction with ice bath cooling, the reaction was subsequently stirred for 1 h at room temperature. Finally, the reaction mixture was extracted three times with diethyl ether and combined organic phases washed twice with saturated sodium chloride solution. The organic phase was separated and dried over sodium sulfate. The solvent was removed under reduced pressure.

1-Azido-2-(trifluoromethyl)benzene **(4p)**

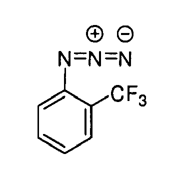

Reaction conditions: According to the general synthesis procedure 1.63 g (10.1 mmol, 1.0 eq.) 2-(trifluoromethyl)aniline **12p** was used. The product **4p** was isolated as a yellow liquid in 94 % yield (1.77 g).
Analytical data: **TLC:** R_{f} = 0.44 (silica gel, cyclohexane). **¹H-NMR (500 MHz, CDCl₃):** δ = 7.21-7.24 (m; 1H), 7.29-7.31 (m; 1H), 7.56-7.59 (m; 1H), 7.63-7.64 (m; 1H). **¹³C-NMR (126 MHz, CDCl₃):** δ = 119.5, 119.7 (¹J_{C,F} = 273.1 Hz), 121.1 (²J_{C,F} = 31.6 Hz), 121.3 (²J_{C,F} = 31.6 Hz), 121.9 (¹J_{C,F} = 273.1 Hz), 124.0 (¹J_{C,F} = 273.1 Hz), 124.4, 126.2 (¹J_{C,F} = 273.1 Hz), 127.4 (³J_{C,F} = 5.2 Hz), 127.4 (³J_{C,F} = 5.2 Hz), 127.5 (³J_{C,F} = 5.2 Hz), 127.5 (³J_{C,F} = 5.2 Hz), 133.1, 138.5. **FT-IR:** *ṽ* = 3442 [m], 2131 [s; v(N₃)], 2104 [s], 1606 [m], 1589 [m], 1495 [m], 1458 [m], 1427 [w], 1322 [s], 1296 [s], 1270 [m], 1168 [w], 1132 [s], 1116 [s], 1041 [s], 950 [w], 758 [s], 684 [w], 642 [w], 596 [w], 534 [w].

1-Azido-3-(trifluoromethyl)benzene **(4q)**

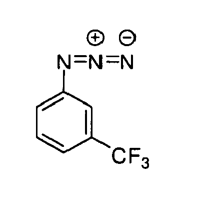

Reaction conditions: According to the general synthesis procedure 1.62 g (10.1 mmol, 1.0 eq.) 3-(trifluoromethyl)aniline **12q** was used. The product **4q** was isolated as a yellow liquid in 87 % yield (1.63 g).
Analytical data: **TLC:** R_{f} = 0.87 (silica gel, ethylacetate). **¹H-NMR (500 MHz, CDCl₃):** δ = 7.20-7.22 (m; 1H), 7.26 (m; 1H), 7.39-7.41 (m; 1H), 7.47-7.50 (m; 1H). **¹³C_NMR (126 MHz, CDCl₃):** δ = 116.0 (³J_{C,F} = 3.6 Hz), 116.0 (³J_{C,F} = 3.6 Hz), 116.1 (³J_{C,F} = 3.6 Hz), 121.5 (³J_{C,F} = 3.6 Hz), 121.6 (³J_{C,F} = 3.6 Hz), 121.6 (³J_{C,F} = 3.6 Hz), 121.6 (3_{JC,F} = 3.6 Hz), 122.2, 130.3, 141.0. **FT-IR:** ṽ = 3232 [w], 2402 [w], 2265 [w], 2165 [s], 2113 [s; ν(N₃)], 1615 [m], 1593 [m], 1489 [m], 1454 [s], 1330 [s], 1290 [m], 1280 [m], 1171 [s], 1133 [s], 1100 [m], 1067 [s], 1001 [w], 886 [m], 866 [m], 792 [s], 754 [w], 695 [s], 651 [w], 535 [w], 437 [w].

1-(Azidomethyl)-3-(trifluoromethyl)benzene **(4a)**

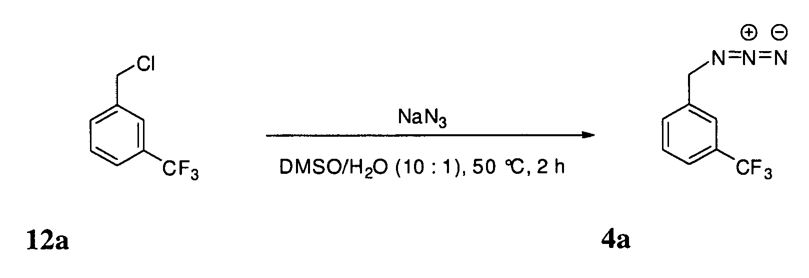

The azide **4a** was obtained as a colorless liquid in 94 % yield (3.01 g) according to a methode previously described by Amegadzie et al. (PCT Int. Appl. 2005, WO 2005000821 A1) by reacting 1-(chloromethyl)-3-(trifluoromethyl)benzene with sodium azide at 50 °C for 2 h.

The present inventions will be explained in more detail in the following examples. However, the examples are only used for illustration and do not limit the scope of the present invention.

### Examples

General synthesis procedure of 8-(1*H*-1,2,3-triazol-4-yl)-*1*H-purine-2,6(3*H*,7*H*)-dione derivatives **1** 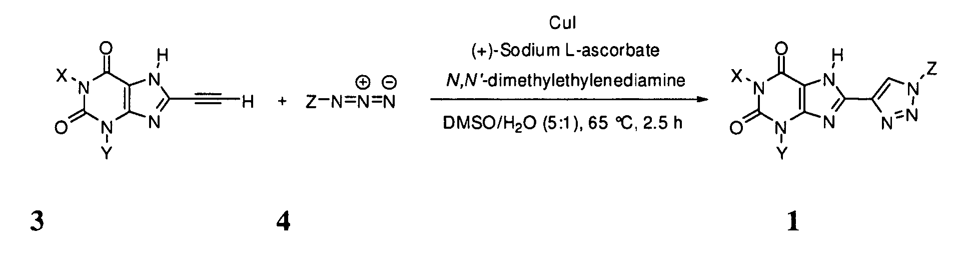 wherein X and Y are defined as above.

In a 50 mL round bottom flask with magnetic stirrer and reflux condenser, (0.10-0.20 mmol, 0.20 eq.) copper (I) iodide, (0.10-0.20 mmol, 0.20 eq.) (+)-sodium L-ascorbate and (0.50-1.00 mmol, 1.0 eq.) 8-ethynyl-1*H*-purine-2,6(3*H*,7*H*)-dione derivative **3** were suspended in 5.0 mL dimethyl sulfoxide and 1.0 mL water. Then, (0.50-1.00 mmol, 1.0-1.9 eq.) azido derivative of formula **4** and (0.15-0.30 mmol, 0.30 eq.) *N*,*N*'-dimethylethylenediamine were added and heated at 65 °C under argon atmosphere for 2.5 h. Then, it was cooled to room temperature and water was added. The precipitate was filtered off and washed with water. The solid was dried at 50-75 °C in a drying oven.

**Example 1:** 8-(1-(2-Methoxyphenyl)-1*H*-1,2,3-triazol-4-yl)-1,3-dimethyl-1*H-*purine-2,6(3*H*,7*H*)-dione (**1j**)

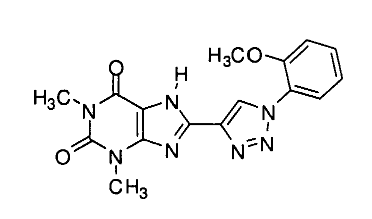

Reaction conditions: According to the general synthesis procedure 0.20 g (1.00 mmol, 1.0 eq.) 8-ethynyl-1,3-dimethyl-1*H*-purine-2,6(3*H*,7*H*)-dione **(3a)** and 0.15 g (1.00 mmol, 1.0 eq.) 1-azido-2-methoxybenzene **(4j)** were used. The crude product was purified by column chromatography on silica gel (eluent: dichloromethane/ethylacetate/acetone). The solid was dried at 50-75 °C in a drying oven. The product **1j** was isolated as a light yellow solid in 49 % yield (0.17 g).
Analytical data: **mp** 320 °C. **TLC:** R_{f} = 0.43 (silica gel, ethylacetate). **LC-MS (m/z):** 326.3 [M-N₂+H]⁺ + 354.3 [M+H]⁺ + 376.3 [M+Na]⁺ + 217.4 [M-C₆H₄-OCH₃-2CH₃+H]⁻ + 309.5 [M-N₂-CH₃-H]⁻ + 324.4 [M-N₂-H]⁻ + 352.1 [M-H]⁻. Purity by **HPLC-UV (254 nm)-ESI-MS:** 99.5 %. **¹H-NMR (500 MHz, DMSO):** δ = 3.22 (s; 3H), 3.46 (s; 3H), 3.88 (s; 3H), 7.14-7.17 (m; 1H), 7.31-7.33 (m; 1H), 7.51-7.54 (m; 1H), 7.66-7.68 (m; 1H). **¹³C-NMR (126 MHz, DMSO):** δ = 27.6, 30.2, 56.2, 113.1, 116.2, 121.0, 125.5, 125.8, 130.7, 144.7, 148.5, 150.5, 151.6, 151.8, 157.8.

**Example 2:** 8-(1-(3-Methoxyphenyl)-1*H*-1,2,3-triazol-4-yl)-1,3-dimethyl-1*H-*purine-2,6(3*H*,7*H*)-dione **(1k)**

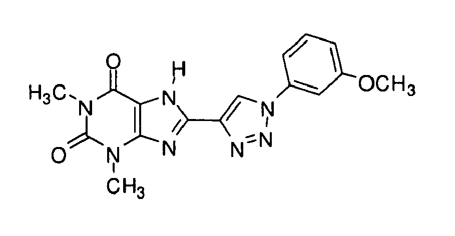

Reaction conditions: According to the general synthesis procedure 0.21 g (1.00 mmol, 1.0 eq.) 8-ethynyl-1,3-dimethyl-1*H*-purine-2,6(3*H*,7*H*)-dione **(3a)** and 0.15 g (1.00 mmol, 1.0 eq.) 1-azido-3-methoxybenzene **(4k)** were used. The crude product was purified by column chromatography on silica gel (eluent: dichloromethane/ethylacetate/acetone). The solid was dried at 50-75 °C in a drying oven. The product **1k** was isolated as a light yellow solid in 21 % yield (75.0 mg).
Analytical data: **mp** 315 °C. **TLC:** R_{f} = 0.51 (silica gel, ethylacetate). **LC-MS (m/z):** 354.3 [M+H]⁺ + 376.4 [M+Na]⁺ + 217.4 [M-C₆H₄-OCH₃-2CH₃+H]⁻ + 309.1 [M-N₂-CH₃-H]⁻ + 324.1 [M-N₂-H]⁻ + 352.0 [M-H]⁻. Purity by **HPLC-UV (254 nm)-ESI-MS:** 99 %. **¹H-NMR (500 MHz, DMSO):** δ = 3.27 (s; 3H), 3.51 (s; 3H), 3.88 (s; 3H), 7.09-7.11 (m; 1H), 7.51-7.58 (m; 3H), 9.38 (s; 1H), 14.17 (brs; 1H) **¹³C-NMR (126 MHz, DMSO): δ=27.7,** 29.8, 55.6, 105.7, 112.2, 114.9, 122.6, 130.7, 137.1, 138.8, 151.0, 154.0, 160.1.

**Example 3:** 8-(1-(4-Methoxyphenyl)-1*H*-1,2,3-triazol-4-yl)-1,3-dimethyl-1*H-*purine-2,6(3*H*,7*H*)-dione **(11)**

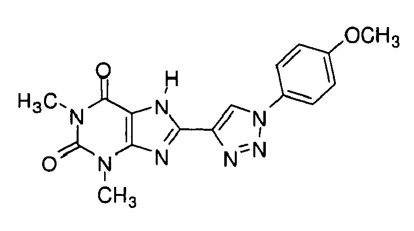

Reaction conditions: According to the general synthesis procedure 0.42 g (2.05 mmol, 1.0 eq.) 8-ethynyl-1,3-dimethyl-1*H*-purine-2,6(3*H*,7*H*)-dione **(3a)** and 0.31 g (2.05 mmol, 1.0 eq.) 1-azido-4-methoxybenzene **(41)** were used. The crude product was purified by column chromatography on silica gel (eluent: dichloromethane/ethylacetate/acetone). The solid was dried at 50-75 °C in a drying oven. The product **1l** was isolated as a light yellow solid in 94 % yield (0.68 g).
Analytical data: **mp** 319 °C. **TLC:** R_{f} = 0.42 (silica gel, ethylacetate). **LC-MS (m/z):** 354.3 [M+H]⁺ + 376.1 [M+Na]⁺ + 217.1 [M-C₆H₄-OCH₃-2CH₃+H]⁻ + 309.0 [M-N₂⁻CH₃-H]⁻ + 324.0 [M-N₂-H]⁻ + 351.8 [M-H]⁻. Purity by **HPLC-UV (254 nm)-ESI-MS:** 98 %. **¹H-NMR (500 MHz, DMSO):** δ = 3.27 (s; 3H), 3.50 (s; 3H), 3.85 (s; 3H), 7.15-7.17 (d; 2H), 7.88-7.89 (d; 2H), 9.22 (s; 1H), 14.12 (s; 1H). **¹³C-NMR (126 MHz, DMSO):** δ = 27.7, 29.7, 55.5, 107.1, 114.8, 121.9, 129.5, 138.6, 142.3, 148.3, 151.0, 153.9, 159.5.

**Example 4:** 8-(1-(2-Fluorophenyl)-1*H*-1,2,3-triazol-4-yl)-1,3-dimethyl-1*H-*purine-2,6(3*H*,7*H*)-dione (**1m**)

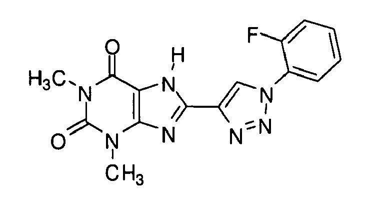

Reaction conditions: According to the general synthesis procedure 0.20 g (1.00 mmol, 1.0 eq.) 8-ethynyl-1,3-dimethyl-1*H*-purine-2,6(3*H*,7*H*)-dione **(3a)** and 0.14 g (1.00 mmol, 1.0 eq.) 1-azido-2-fluorobenzene **(4m)** were used. The crude product was purified by column chromatography on silica gel (eluent: dichloromethane/ethylacetate/acetone). The solid was dried at 50-75 °C in a drying oven. The product **1m** was isolated as a white solid in 31 % yield (0.10 g).
Analytical data: **mp** 337 °C. **TLC:** R_{f} = 0.44 (silica gel, ethylacetate). **LC-MS (m/z):** 314.5 [M-N₂+H]⁺ + 342.3 [M+H]⁺ + 217.4 [M-C₆H₄-F-2CH₃+H]⁻ + 297.4 [M-N₂-CH₃-H]⁻ + 312.3 [M-N₂-H]⁻ + 340.4 [M-H]⁻. Purity by **HPLC-UV (254 nm)-ESI-MS:** 100 %. **¹H-NMR (500 MHz, DMSO):** δ = 3.28 (s; 3H), 3.52 (s; 3H), 7.47-7.50 (m; 1H), 7.60-7.67 (m; 2H), 7.93-7.96 (m; 1H), 9.15 (s; 1H), 14.20 (s; 1H). **13C-NMR (126 MHz, DMSO):** δ = 27.6, 30.2, 99.6, 107.4, 116.4, 116.9, 117.2 (²J_{C,F} = 19.2 Hz), 117.4 (²J_{C,F} = 19.2 Hz), 121.9, 124.8 (³J_{C,F} = 10.7 Hz), 124.9 (³J_{C,F} = 10.7 Hz), 125.7, 130.9 (³J_{C,F} = 7.9 Hz), 131.0 (³J_{C,F} = 7.9 Hz), 145.6, 148.1, 150.5, 151.9, 157.7.

**Example 5:** 8-(1-(3-Fluorophenyl)-1*H*-1,2,3-triazol-4-yl)-1,3-dimethyl-1*H-*purine-2,6(3*H*,7*H*)-dione **(1n)**

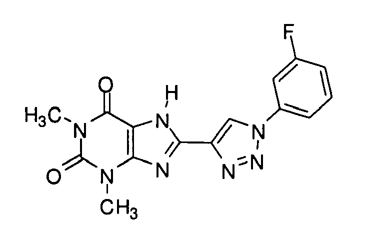

Reaction conditions: According to the general synthesis procedure 0.20 g (1.00 mmol, 1.0 eq.) 8-ethynyl-1,3-dimethyl-1*H*-purine-2,6(3*H*,7*H*)-dione **(3a)** and 0.14 g (1.00 mmol, 1.0 eq.) 1-azido-3-fluorobenzene **(4n)** were used. The crude product was purified by column chromatography on silica gel (eluent: dichloromethane/ethylacetate/acetone). The solid was dried at 50-75 °C in a drying oven. The product **1n** was isolated as a yellow solid in 41 % yield (0.14 g).
Analytical data: **mp** 325 °C. **TLC:** R_{f} = 0.55 (silica gel, ethylacetate). **LC-MS (m/z):** 314.4 [M-N₂+H]⁺ + 342.3 [M+H]⁺ + 217.4 [M-C₆H₄-F-2CH₃+H]⁻ + 297.4 [M-N₂-CH₃-H]⁻ + 312.3 [M-N₂-H]⁻ + 340.4 [M-H]⁻. Purity by **HPLC-UV (254 nm)-ESI-MS:** 99.6 %. **¹H-NMR (500 MHz, DMSO): δ** = 3.26 (s; 3H), 3.50 (s; 3H), 7.37-7.41 (m; 1H), 7.65-7.70 (m; 1H), 7.87-7.89 (m; 1H), 7.92-7.94 (m; 1H), 9.38 (s; 1H), 14.20 (s; 1H). **¹³C-NMR (126 MHz, DMSO):** δ = 27.7, 29.7, 107.3, 107.7 (²J_{C,F} = 26.8 Hz), 107.9 (²J_{C,F} = 26.8 Hz), 115.6 (²J_{C,F} = 21.1 Hz), 115.8 (²J_{C,F} = 21.1 Hz), 116.1 (⁴J_{C,F} = 2.8 Hz), 116.1 (⁴J_{C,F} = 2.8 Hz), 122.6, 131.7 (³J_{C,F} = 9.1 Hz), 131.8 (³J_{C,F} = 9.1 Hz), 137.2 (³J_{C,F} = 10.6 Hz), 137.3 (³J_{C,F} = 10.6 Hz), 138.9, 142.0, 148.3, 151.0, 153.9, 161.3 (¹J_{C,F} = 245.4 Hz), 163.2 (¹J_{C,F} =245.4 Hz).

**Example 6:** 8-(1-(4-Fluorophenyl)-1*H*-1,2,3-triazol-4-yl)-1,3-dimethyl-1*H-*purine-2,6(3*H*,7*H*)-dione (**1o**)

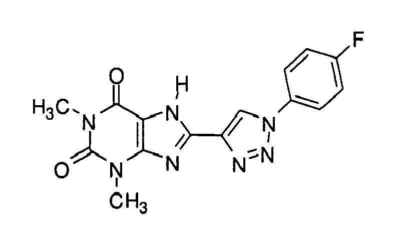

Reaction conditions: According to the general synthesis procedure 0.11 g (0.54 mmol, 1.0 eq.) 8-ethynyl-1,3-dimethyl-1*H*-purine-2,6(3*H*,7*H*)-dione (**3a**) and 0.13 g (0.93 mmol, 1.7 eq.) 1-azido-4-fluorobenzene **(4o)** were used. The crude product was purified by precipitation three times by dissolving in acetone and treatment with diluted hydrochloric acid. The precipitate was filtered off and washed with water. The solid was dried at 50-75 °C in a drying oven. The product **1o** was isolated as a yellow solid in 52 % yield (95.7 mg).
Analytical data: **mp** 344 °C. **TLC:** R_{f} = 0.49 (silica gel, ethylacetate). **LC-MS (m/z):** 342.2 [M+H]⁺ + 217.1 [M-C₆H₄-F-2CH₃+H]⁻ + 312.2 [M-N₂-H]⁻ + 340.2 [M-H]⁻. Purity by **HPLC-UV (254 nm)-ESI-MS:** 99.5 %. **¹H-NMR (500 MHz, DMSO):** δ = 3.27 (s; 3H), 3.50 (s; 3H), 7.47-7.50 (m; 2H), 8.02-8.05 (m; 2H), 9.31 (s; 1H), 14.16 (s; 1H). **¹³C-NMR (126MHz, DMSO):** δ = 27.7, 29.7, 107.3, 116.6 (²J_{C,F} = 23.3 Hz), 116.7 (²J_{C,F} = 23.3 Hz), 122.6 (³J_{C,F} = 8.8 Hz), 122.7 (³J_{C,F} = 8.8 Hz), 132.6 (⁴J_{C,F} = 2.6 Hz), 132.6 (⁴J_{C,F} = 2.6 Hz), 138.8, 142.0, 148.2, 151.0, 153.9, 160.8 (¹J_{C,F} = 246.4 Hz), 162.8 (¹J_{C,F} = 246.4 Hz).

**Example 7:** 1,3-Dimethyl-8-(1-(2-(trifluoromethyl)phenyl)-1*H*-1,2,3-triazol-4-yl)-1*H*-purine-2,6(3*H*,7*H*)-dione **(1p)**

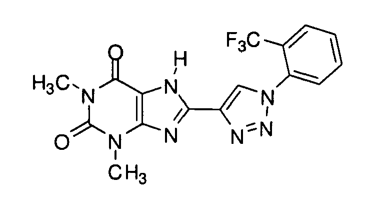

Reaction conditions: According to the general synthesis procedure 0.21 g (1.02 mmol, 1.0 eq.) 8-ethynyl-1,3-dimethyl-1*H*-purine-2,6(3*H*,7*H*)-dione **(3a)** and 0.19 g (1.02 mmol, 1.0 eq.) 1-azido-2-(trifluoromethyl)benzene **(4p)** were used. The crude product was purified by column chromatography on silica gel (eluent: dichloromethane/ethylacetate/acetone). The solid was dried at 50-75 °C in a drying oven. The product **1p** was isolated as a light yellow solid in 47 % yield (0.19 g).
Analytical data: **mp** 309 °C. **TLC:** R_{f} = 0.35 (silica gel, ethylacetate). **LC-MS (m/z):** 364.1 [M-N₂+H]⁺+ 392.3 [M+H]⁺ + 217.4 [M-C₆H₄-CF₃-2CH₃+H]⁻ + 342.4 [M-48.9]⁻ + 362.4 [M-N₂-H]⁻ + 390.3 [M-H]⁻. Purity by **HPLC-UV (254 nm)-ESI-MS:** 99.5 %. **¹H-NMR (500 MHz, DMSO):** δ = 3.27 (s; 3H), 3.50 (s; 3H), 7.84-7.86 (m; 1H), 7.89-7.93 (m; 1H), 7.96-7.99 (m; 1H), 8.06-8.08 (m; 1H), 9.09 (s; 1H), 14.20 (s; 1H). **¹³C-NMR (126 MHz, DMSO):** δ = 27.7, 29.7, 121.5 (¹J_{C,F} = 273.6 Hz), 123.7 (¹J_{C,F} = 273.6 Hz), 124.4 (²J_{C,F} = 31.4 Hz), 124.7 (²J_{C,F} = 31.4 Hz), 124.9 (²J_{C,F} = 31.4 Hz), 125.1 (²J_{C,F} = 31.4 Hz), 126.9, 127.3 (³J_{C,F} = 4.7 Hz), 127.4 (³J_{C,F} = 4.7 Hz), 127.4 (³J_{C,F} = 4.7 Hz), 127.5 (³J_{C,F} = 4.7 Hz), 129.3, 131.4, 133.5, 134.0, 138.1, 142.0, 148.3, 151.0, 154.0.

**Example 8:** 1,3-Dimethyl-8-(1-(3-(trifluoromethyl)phenyl)-1*H*-1,2,3-triazol-4-yl)-1*H*-purine-2,6(3*H*,7*H*)-dione **(1q)**

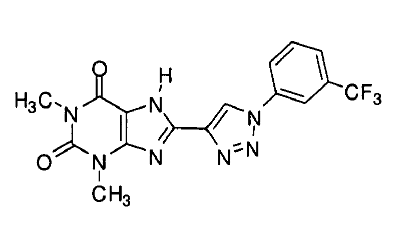

Reaction conditions: According to the general synthesis procedure 0.21 g (1.01 mmol, 1.0 eq.) 8-ethynyl-1,3-dimethyl-1*H*-purine-2,6(3*H*,7*H*)-dione **(3a)** and 0.19 g (1.01 mmol, 1.0 eq.) 1-azido-3-(trifluoromethyl)benzene **(4q)** were used. The crude product was purified by column chromatography on silica gel (eluent: dichloromethane/ethylacetate/acetone). The solid was dried at 50-75 °C in a drying oven. The product **1q** was isolated as a light yellow solid in 61 % yield (0.24 g).
Analytical data: **mp** 303 °C. **TLC:** R_{f} = 0.56 (silica gel, ethylacetate). **LC-MS (m/z):** 364.3 [M-N₂+H]⁺ + 392.4 [M+H]⁺ + 414.1 [M+Na]⁺ + 217.1 [M-C₆H₄-CF₃-2CH₃+H]⁻ + 342.1 [M-49.2]⁻ + 362.4 [M-N₂-H]⁻ + 390.4 [M-H]⁻. Purity by **HPLC-UV (254 nm)-ESI-MS:** 99.7 **%. ¹H-NMR (500 MHz, DMSO):** δ = 3.23 (s; 3H), 3.47 (s; 3H), 7.84 (m; 2H), 8.29 (m; 2H). **¹³C-NMR (126 MHz, DMSO):** δ = 27.7, 30.3, 116.6, 123.9, 125.1, 130.6 (²J_{C,F} = 32.6 Hz), 130.8 (²J_{C,F} = 32.6 Hz), 131.5, 137.2, 146.3, 148.1, 150.6, 152.0, 157.9.

**Example 9:** 1,3-Dimethyl-8-(1-(4-(trifluoromethyl)phenyl)-1*H*-1,2,3-triazol-4-yl)-1*H*-purine-2,6(3*H*,7*H*)-dione **(1r)**

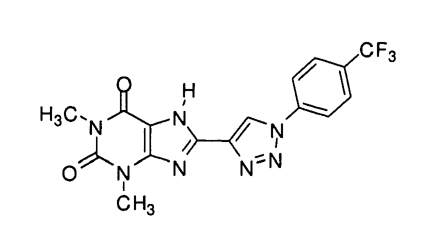

Reaction conditions: According to the general synthesis procedure 0.21 g (1.01 mmol, 1.0 eq.) 8-ethynyl-1,3-dimethyl-1*H*-purine-2,6(3*H*,7*H*)-dione **(3a)** and 0.19 g (1.01 mmol, 1.0 eq.) 1-azido-4-(trifluoromethyl)benzene **(4r)** were used. The crude product was purified by column chromatography on silica gel (eluent: dichloromethane/ethylacetate/acetone). The solid was dried at 50-75 °C in a drying oven. The product **1r** was isolated as a white solid in 51 % yield (0.20 g).
Analytical data: **mp** 297 °C. **TLC:** R_{f} = 0.57 (silica gel, ethylacetate). **LC-MS (m/z):** 364.1 [M-N₂+H]⁺ + 392.4 [M+H]⁺ + 414.3 [M+Na]⁺ + 217.1 [M-C₆H₄-CF₃-2CH₃+H]⁻ + 347.3 [M-N₂-CH₃-H]- + 362.4 [M-N₂-H]⁻ + 390.3 [M-H]⁻. Purity by **HPLC-UV (254 nm)-ESI-MS:** 99 %. **¹H-NMR (500 MHz, DMSO): δ** = 3.23 (s; 3H), 3.47 (s; 3H), 7.96-7.98 (m; 2H), 8.20-8.22 (m; 2H). **¹³C-NMR (126 MHz, DMSO):** δ = 27.6, 30.2, 116.4, 120.4, 122.8 (¹J_{C,F} = 272.2 Hz), 125.0 (¹J_{C,F} = 272.2 Hz), 127.1 (³J_{C,F} = 3.5 Hz), 127.1 (³J_{C,F} = 3.5 Hz), 127.2 (³J_{C,F} = 3.5 Hz), 127.3 (³J_{C,F} = 3.5 Hz), 128.5 (²J_{C,F} = 32.3 Hz), 128.7 (²J_{C,F} = 32.3 Hz), 139.4, 146.0, 147.9, 150.4, 151.8, 157.8.

**Example 10:** 8-(1-(2-(Hydroxymethyl)phenyl)-1*H*-1,2,3-triazol-4-yl)-1,3-dimethyl-1*H*-purine-2,6(3*H*,7*H*)-dione **(1s)**

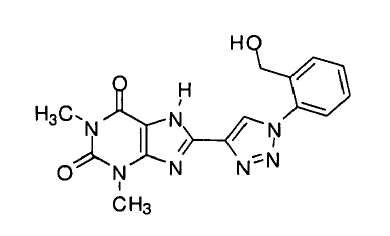

Reaction conditions: According to the general synthesis procedure 0.21 g (1.01 mmol, 1.0 eq.) 8-ethynyl-1,3-dimethyl-1*H*-purine-2,6(3*H*,7*H*)-dione **(3a)** and 0.15 g (1.01 mmol, 1.0 eq.) (2-azidophenyl)methanol **(4s)** were used. The crude product was purified by column chromatography on silica gel (eluent: dichloromethane/ethylacetate/acetone). The solid was dried at 50-75 °C in a drying oven. The product **1s** was isolated as a white solid in 54 % yield (0.19 g).
Analytical data: **mp** 278 °C. **TLC:** R_{f} = 0.56 (silica gel, acetone). **LC-MS (m/z):** 308.4 [M-CH₃-N₂-2H]⁺ + 326.3 [M-N₂+H]⁺ + 354.4 [M+H]⁺ + 296.5 [M-2CH₃-N₂+H]⁻ + 324.4 [M-N₂-H]⁻ + 352.3 [M-H]⁻. Purity by **HPLC-UV (254 nm)-ESI-MS:** 98 %. **¹H-NMR (500 MHz, DMSO):** δ = 3.27 (s; 3H), 3.51 (s; 3H), 4.45 (s; 2H), 5.37 (s; 1H), 7.52-7.64 (m; 3H), 7.73-7.74 (m; 1H), 9.03 (s; 1H), 14.15 (s; 1H). **¹³C-NMR (126 MHz, DMSO):** δ = 27.6, 29.7, 58.8, 107.1, 125.5, 128.0, 128.8, 129.9, 134.2, 136.8, 137.9, 142.3, 148.3, 151.0, 154.0.

**Example 11:** 8-(1-(3-(Hydroxymethyl)phenyl)-1*H*-1,2,3-triazol-4-yl)-1,3-dimethyl-1H-purine-2,6(3*H*,7*H*)-dione **(1t)**

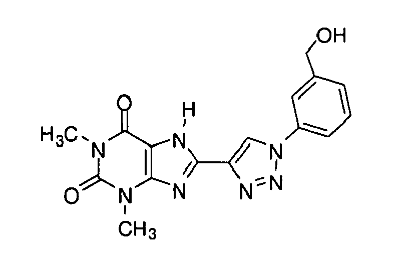

Reaction conditions: According to the general synthesis procedure 0.12 g (0.57 mmol, 1.0 eq.) 8-ethynyl-1,3-dimethyl-1*H*-purine-2,6(3*H*,7*H*)-dione **(3a)** and 0.14 g (0.96 mmol, 1.7 eq.) (3-Azidophenyl)methanol **(4t)** were used. The crude product was purified by precipitation twice by dissolving in acetone and treatment with diluted hydrochloric acid. The precipitate was filtered off and washed with water. The solid was dried at 50-75 °C in a drying oven. The product **1t** was isolated as a white solid in 90 % yield (0.18 g).
Analytical data: **mp** 317 °C. **TLC:** R_{f} = 0.71 (silica gel, acetone). **LC-MS** (m/z): 354.5 [M+H]⁺ + 217.1 [M-C₆H₄-CH₂OH-2CH₃+H]⁻ + 291.2 [M-2CH₃-CH₂OH-H]⁻ + 306.3 [M-2CH₃-OH]⁻ + 324.2 [M-N₂-H]⁻ + 352.1 [M-H]⁻. Purity by **HPLC-UV (254 nm)-ESI-MS:** 100 %. **¹H-NMR (500 MHz, DMSO):** δ = 3.26 (s; 3H), 3.50 (s; 3H), 4.62 (s; 2H), 5.41 (s; 1H), 7.46-7.48 (m; 1H), 7.56-7.59 (m; 1H), 7.82-7.84 (m; 1H), 7.94 (s; 1H), 9.30 (s; 1H), 14.13 (s; 1H). **¹³C-NMR (126 MHz, DMSO):** δ = 27.7, 29.7, 62.1, 107.0, 118.0, 118.4, 122.4, 126.8, 129.6, 136.0, 138.7, 142.1, 144.8, 148.3, 151.0, 153.9.

**Example 12:** 8-(1-(4-(Hydroxymethyl)phenyl)-1*H*-1,2,3-triazol-4-yl)-1,3-dimethyl-1*H*-purine-2,6(3*H*,7*H*)-dione **(1u)**

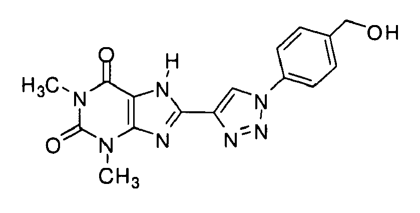

Reaction conditions: According to the general synthesis procedure 0.21 g (1.01 mmol, 1.0 eq.) 8-ethynyl-1,3-dimethyl-1*H*-purine-2,6(3*H*,7*H*)-dione (**3a**) and 0.15 g (1.01 mmol, 1.0 eq.) (4-Azidophenyl)methanol **(4u)** were used. The crude product was purified by column chromatography on silica gel (eluent: dichloromethane/ethylacetate/acetone). The solid was dried at 50-75 °C in a drying oven. The product **1u** was isolated as a light yellow solid in 33 % yield (0.12 g).
Analytical data: **mp** 318 °C. **TLC:** R_{f} = 0.64 (silica gel, acetone). **LC-MS (m/z):** 354.3 [M+H]⁺ + 217.4 [M-C₆H₄-CH₂OH-2CH₃+H]⁻ + 324.1 [M-N₂-H]⁻ + 352.0 [M-H]⁻. Purity by **HPLC-UV (254 nm)-ESI-MS:** 98 %. **¹H-NMR (500 MHz, DMSO):** δ = 3.27 (s; 3H), 3.51 (s; 3H), 4.60 (s; 2H), 5.35 (s; 1H), 7.55-7.56 (m; 2H), 7.92-7.94 (m; 2H), 9.30 (s; 1H), 14.14 (s; 1H). **¹³C-NMR (126 MHz, DMSO):** δ = 27.7, 29.7, 62.1, 107.4, 120.0, 122.3, 127.5, 134.7, 138.8, 142.2, 143.6, 148.3, 151.0, 154.0.

**Example 13:** 8-(1-(2-Hydroxyphenyl)-1*H*-1,2,3-triazol-4-yl)-1,3-dimethyl-1*H-*purine-2,6(3*H*,7*H*)-dione **(1v)**

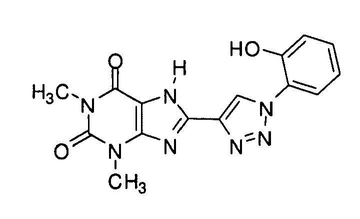

Reaction conditions: According to the general synthesis procedure 0.11g (0.53 mmol, 1.0 eq.) 8-ethynyl-1,3-dimethyl-1*H*-purine-2,6(3*H*,7*H*)-dione **(3a)** and 0.13 g (0.98 mmol, 1.9 eq.) 2-azidophenol **(4v)** were used. The crude product was purified by precipitation three times by dissolving in acetone and treatment with diluted hydrochloric acid. The precipitate was filtered off and washed with water. The solid was dried at 50-75 °C in a drying oven. The product **1v** was isolated as a light brown solid in 80 % yield (0.14 g).
Analytical data: **mp** 316 °C. **TLC:** R_{f} = 0.67 (silica gel, acetone). **LC-MS (m/z):** 340.2 [M+H]⁺ + 295.1 [M-N₂-CH₃-H]⁻ + 310.2 [M-N₂-H]⁻ + 338.2 [M-H]⁻. Purity by **HPLC-UV (254 nm)-ESI-MS:** 100 %. **¹H-NMR (500 MHz, DMSO):** δ = 3.28 (s; 3H), 3.52 (s; 3H), 7.02-7.05 (m; 1H), 7.15-7.17 (m; 1H), 7.38-7.41 (m; 1H), 7.70-7.72 (m; 1H), 9.05 (s; 1H), 10.69 (s; 1H), 14.10 (s; 1H). **¹³C-NMR (126 MHz, DMSO):** δ = 27.7, 29.7, 107.1, 117.0, 119.5, 123.9, 124.9, 125.6, 130.4, 137.6, 142.5, 148.4, 149.5, 151.1, 154.0.

**Example 14:** 8-(1-(3-Hydroxyphenyl)-1*H*-1,2,3-triazol-4-yl)-1,3-dimethyl-1*H-*purine-2,6(3*H*,7*H*)-dione **(1w)**

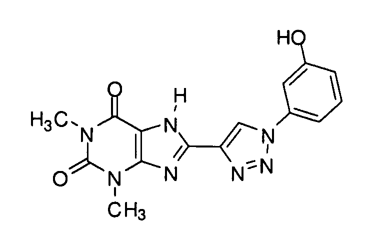

Reaction conditions: According to the general synthesis procedure 0.11 g (0.52 mmol, 1.0 eq.) 8-ethynyl-1,3-dimethyl-1*H*-purine-2,6(3*H*,7*H*)-dione **(3a)** and 87.5 mg (0.65 mmol, 1.3 eq.) 3-azidophenol **(4w)** were used. The crude product was purified by precipitation twice by dissolving in acetone and treatment with diluted hydrochloric acid. The precipitate was filtered off and washed with water. The solid was dried at 50-75 °C in a drying oven. The product **1w** was isolated as an ocher solid in 82 % yield (0.15 g).
Analytical data: **mp** > 400 °C. **TLC:** R_{f} = 0.71 (silica gel, acetone). **LC-MS (m/z):** 217.2 [M-C₆H₄-OH-2CH₃+H]⁻ + 295.2 [M-N₂-CH₃-H]⁻ + 310.2 [M-N₂-H]⁻ + 338.3 [M-H]-. Purity by **HPLC-UV (254 nm)-ESI-MS:** 100 %. **¹H-NMR (500 MHz, DMSO):** δ = 3.26 (s; 3H), 3.49 (s; 3H), 6.91-6.93 (m; 1H), 7.36-7.42 (m; 3H), 9.23 (s; **1H),** 10.07 (s; 1H), 14.10 (s; 1H). **¹³C-NMR (126 MHz, DMSO):** δ = 27.7, 29.7, 107.1, 110.6, 116.0, 122.3, 130.7, 137.0, 138.7, 142.2, 148.3, 151.0, 153.9, 158.4.

**Example 15:** 8-(1-(4-Hydroxyphenyl)-1*H*-1,2,3-triazol-4-yl)-1,3-dimethyl-1*H-*purine-2,6(3*H*,7*H*)-dione **(1x)**

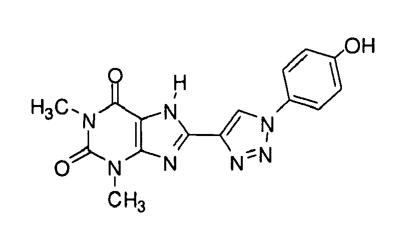

Reaction conditions: According to the general synthesis procedure 0.10 g (0.50 mmol, 1.0 eq.) 8-ethynyl-1,3-dimethyl-1*H*-purine-2,6(3*H*,7*H*)-dione **(3a)** and 0.12 g (0.86 mmol, 1.7 eq.) 4-azidophenol **(4x)** were used. The crude product was purified by precipitation three times by dissolving in acetone and treatment with diluted hydrochloric acid. The precipitate was filtered off and washed with water. The solid was dried at 50-75 °C in a drying oven. The product **lx** was isolated as a light brown solid in 98 % yield (0.17 g).
Analytical data: **mp** 389 °C. **TLC:** R_{f} = 0.74 (silica gel, acetone). **LC-MS (m/z):** 217.1 [M-C₆H₄-OH-2CH₃+H]⁻ + 310.2 [M-N₂-H]⁻ + 338.2 [M-H]⁻. Purity by **HPLC-UV (254 nm)-ESI-MS:** 100 %. **¹H-NMR (500 MHz, DMSO):** δ = 3.26 (s; 3H), 3.50 (s; 3H), 6.94-6.97 (m; 2H), 7.73-7.76 (m; 2H), 9.13 (s; 1H), 9.99 (s; 1H), 14.08 (s; 1H). **¹³C-NMR (126 MHz, DMSO):** δ = 27.7, 29.7, 107.1, 116.0, 122.1, 122.2, 128.2, 138.5, 142.4, 148.3, 151.0, 153.9, 158.0.

**Example 16:** 8-(1-Cyclohexyl-1*H*-1,2,3-triazol-4-yl)-1,3-dimethyl-1*H*-purine-2,6(3*H*,7*H*)-dione **(1y)**

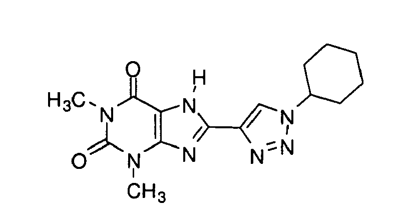

Reaction conditions: According to the general synthesis procedure 0.21 g (1.02 mmol, 1.0 eq.) 8-ethynyl-1,3-dimethyl-1*H*-purine-2,6(3*H*,7*H*)-dione **(3a)** and 0.13 g (1.02 mmol, 1.0 eq.) azidocyclohexane **(4y)** were used. The crude product was purified by column chromatography on silica gel (eluent: dichloromethane/ethylacetate/acetone). The solid was dried at 50-75 °C in a drying oven. The product **1y** was isolated as a light yellow solid in 56 % yield (0.19 g).
Analytical data: **mp** 332 °C. **TLC:** R_{f} = 0.41 (silica gel, ethylacetate). **LC-MS (m/z):** 302.4 [M-N₂+H]⁺ + 330.5 [M+H]⁺ + 352.3 [M+Na]⁺ + 217.6 [M-C₆H₁₁-2CH₃+H]⁻ + 300.4 [M-2CH₃+H]⁻ + 328.4 [M-H]⁻. Purity by **HPLC-UV (254 nm)-ESI-MS:** 99.9 %. **¹H-NMR (500 MHz, DMSO):** δ = 1.22-1.31 (m; 1H), 1.42-1.50 (m; 2H), 1.67-1.70 (m; 1H), 1.79-1.87 (m; 4H), 2.11-2.14 (m; 2H), 3.25 (s; 3H), 3.48 (s; 3H), 4.55-4.60 (s; 1H), 8.72 (s; 1H), 13.97 (s; 1H). **¹³C-NMR (126 MHz, DMSO):** δ = 24.4, 24.5, 27.6, 29.6, 32.5, 59.3, 106.9, 122.6, 137.5, 143.0, 148.3, 151.0, 153.9.

**Example 17:** 1,3-Dimethyl-8-(1-(3-(trifluoromethyl)benzyl)-1*H*-1,2,3-triazol-4-yl)-1*H*-purine-2,6(3*H*,7*H*)-dione **(1a)**

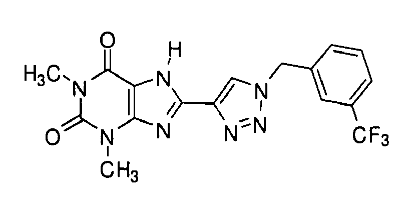

Reaction conditions: According to the general synthesis procedure 0.21 g (1.05 mmol, 1.0 eq.) 8-ethynyl-1,3-dimethyl-1*H*-purine-2,6(3*H*,7*H*)-dione **(3a)** and 0.21 g (1.05 mmol, 1.0 eq.) 1-(azidomethyl)-3-(trifluoromethyl)benzene **(4a)** were used. The crude product was purified by precipitation by dissolving in acetone and treatment with diluted hydrochloric acid and petroleum ether. A white precipitate resulted at the interface. The precipitate was filtered off and washed with water and petroleum ether. The solid was dried at 50-75 °C in a drying oven. The product **1a** was isolated as a white solid in 100 % yield (0.43 g).
Analytical data: **mp** 275 °C. **TLC:** R_{f} = 0.79 (silica gel, acetone). **LC-MS (m/z):** 406.3 [M+H]⁺ + 217.2 [M-CH₂-C₆H₄-CF₃-2CH₃+H]⁻ + 404.3 [M-H]⁻. Purity by **HPLC-UV (254 nm)-ESI-MS:** 100 %. **¹H-NMR (500 MHz, DMSO):** δ = 3.24 (s; 3H), 3.47 (s; 3H), 5.83 (s; 2H), 7.63-7.69 (m; 2H), 7.73-7.74 (m; 1H), 7.82 (s; 1H), 8.82 (s; 1H), 14.01 (s; 1H). **¹³C-NMR (126 MHz, DMSO):** δ = 27.6, 29.6, 52.3, 107.0, 120.6 (¹J_{C,F} = 272.4 Hz), 122.8 (¹J_{C,F} = 272.4 Hz), 124.8 (³J_{C,F} = 3.7 Hz), 124.8 (³J_{C,F} = 3.7 Hz), 125.0 (³J_{C,F} = 3.5 Hz), 125.0 (³J_{C,F} = 3.5 Hz), 127.1 (¹J_{C,F} = 272.4 Hz), 129.0 (²J_{C,F} = 31.8 Hz), 129.2 (²J_{C,F} = 31.8 Hz), 129.5 (²J_{C,F} = 31.8 Hz), 129.7 (²J_{C,F} = 31.8 Hz), 129.9, 132.2, 136.8, 138.1, 142.5, 148.3, 151.0, 153.9.

**Example 18:** 1,3-Diethyl-8-(1-(3-(trifluoromethyl)benzyl)-1*H*-1,2,3-triazol-4-yl)-1*H*-purine-2,6(3*H*,7*H*)-dione **(1b)**

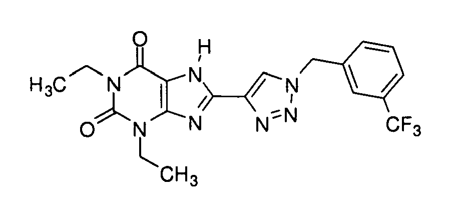

Reaction conditions: According to the general synthesis procedure 0.12 g (0.51 mmol, 1.0 eq.) 1,3-diethyl-8-ethynyl-1*H*-purine-2,6(3*H*,7*H*)-dione **(3b)** and 0.10g (0.51 mmol, 1.0 eq.) 1-(azidomethyl)-3-(trifluoromethyl)benzene (**4a**) were used. The product **1b** was isolated as a white solid in 95 % yield (0.21 g).
Analytical data: **mp** 217 °C. **TLC:** R_{f} = 0.36 (silica gel, dichloromethane/methanol 7 : 1). **LC-MS (m/z):** 434.3 [M+H]⁺ + 245.3 [M-CH₂-C₆H₄-CF₃-C₂H₅]⁻ + 404.4 [M-C₂H₅]⁻ + 432.1 [M-H]⁻. Purity by **HPLC-UV (254 nm)-ESI-MS:** 99 %. **¹H-NMR (500 MHz, DMSO):** δ = 1.12-1.15 (t; ³J_{H,H} = 7.0 Hz, 3H), 1.24-1.27 (t; ³J_{H,H} = 7.1 Hz, 3H), 3.92-3.96 (q; ³J_{H,H} = 7.1 Hz, 2H), 4.05-4.09 (q; ³J_{H,H} = 7.0 Hz, 2H), 5.83 (s; 2H), 7.63-7.70 (m; 2H), 7.73-7.75 (m; 1H), 7.82 (s; 1H), 8.84 (s; 1H), 14.03 (s; 1H). ¹³C-**NMR (126 MHz, DMSO):** δ = 13.0, 35.6, 37.9, 52.3, 107.2, 120.6 (¹J_{C,F} = 272.6 Hz), 122.8 (¹J_{C,F} = 272.6 Hz), 124.8 (³J_{C,F} = 3.7 Hz), 124.8 (³J_{C,F} = 3.7 Hz), 125.0 (³J_{C,F} = 3.5 Hz), 125.0 (³J_{C,F} = 3.5 Hz), 127.1 (¹J_{C,F} = 272.6 Hz), 129.0 (²J_{C,F} = 31.9 Hz), 129.2 (²J_{C,F} = 31.9 Hz), 129.5 (²J_{C,F} = 31.9 Hz), 129.7 (²J_{C,F} = 31.9 Hz), 129.9, 132.3, 136.8, 138.1, 142.7, 147.8, 150.0, 153.5.

**Example 19:** 1-Ethyl-8-(1-(3-(trifluoromethyl)benzyl)-1*H*-1,2,3-triazol-4-yl)-1*H*-purine-2,6(3*H*,7*H*)-dione **(1c)**

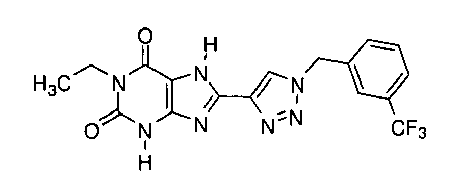

Reaction conditions: According to the general synthesis procedure 56.3 mg (0.28 mmol, 1.0 eq.) 1-ethyl-8-ethynyl-1*H*-purine-2,6(3*H*,7*H*)-dione **(3c)** and 0.11 g (0.55 mmol, 2.0 eq.) 1-(azidomethyl)-3-(trifluoromethyl)benzene **(4a)** were used. The crude product was purified by precipitation by dissolving in acetone and treatment with diluted hydrochloric acid and petroleum ether. A white precipitate resulted at the interface. The precipitate was filtered off and washed with water and petroleum ether. The solid was dried at 50-75 °C in a drying oven. The product **1c** was isolated as a white solid in 93 % yield (0.10 g).
Analytical data: **mp** 389 °C. **TLC:** R_{f} = 0.63 (silica gel, dichloromethane/methanol 7 : 1). **LC-MS (m/z):** 378.5 [M-N₂+H]⁺ + 406.1 [M+H]⁺ + 428.3 [M+Na]⁺ + 217.3 [M-CH₂-C₆H₄-CF₃-C₂H₅]⁻ + 349.0 [M-C₂H₅-N₂+H]⁻ + 376.1 [M-C₂H₅]⁻ + 404.3 [M-H]⁻. Purity by **HPLC-UV (254 nm)-ESI-MS:** 99.5 %. **¹H-NMR (500 MHz, DMSO):** δ = 1.11-1.14 (t; ³J_{H,H} = 6.9 Hz, 3H), 3.87-3.91 (q; ³J_{H,H} = 6.8 Hz, 2H), 5.83 (s; 2H), 7.63-7.68 (m; 2H), 7.73-7.74 (m; 1H), 7.81 (s; 1H), 8.77 (s; 1H), 11.86 (s; 1H). **¹³C-NMR (126 MHz, DMSO):** δ = 13.1, 34.8, 52.3, 107.1, 122.8, 124.7 (³J_{C,F} = 5.2 Hz), 124.7 (³J_{C,F} = 5.2 Hz), 125.0 (³J_{C,F} = 3.5 Hz), 125.0 (³J_{C,F} = 3.5 Hz), 129.2 (²J_{C,F} = 31.7 Hz), 129.5 (²J_{C,F} = 31.7 Hz), 129.9, 132.2, 136.8, 138.2, 142.7, 147.3, 150.6, 154.3.

**Example 20:** 1-Propyl-8-(1-(3-(trifluoromethyl)benzyl)-1*H*-1,2,3-triazol-4-yl)-1*H*-purine-2,6(3*H*,7*H*)-dione **(1d)**

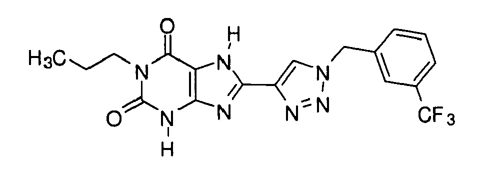

Reaction conditions: According to the general synthesis procedure 56.9 mg (0.26 mmol, 1.0 eq.) 8-ethynyl-1-propyl-1*H*-purine-2,6(3*H*,7*H*)-dione **(3d)** and 0.11 g (0.52 mmol, 2.0 eq.) 1-(azidomethyl)-3-(trifluoromethyl)benzene **(4a)** were used. The crude product was purified by precipitation by dissolving in acetone and treatment with diluted hydrochloric acid and petroleum ether. A white precipitate resulted at the interface. The precipitate was filtered off and washed with water and petroleum ether. The solid was dried at 50-75 °C in a drying oven. The product **1d** was isolated as a white solid in 100 % yield (0.11 g).
Analytical data: **mp** 386 °C. **TLC:** R_{f} = 0.65 (silica gel, dichloromethane/methanol 7 : 1). **LC-MS (m/z):** 420.3 [M+H]⁺ + 231.3 [M-CH₂-C₆H₄-CF₃-C₂H₅]⁻ + 418.4 [M-H]⁻. Purity by **HPLC-UV (254 nm)-ESI-MS:** 98 %. **¹H-NMR (500 MHz, DMSO):** δ = 0.85-0.88 (t; ³J_{H,H} = 7.4 Hz, 3H), 1.53-1.60 (m; ³J_{H,H} = 7.4 Hz, 2H), 3.79-3.82 (t; ³J_{H,H} = 7.3 Hz, 2H), 5.83 (s; 2H), 7.65-7.66 (m; 2H), 7.73-7.75 (m; 1H), 7.81 (s; 1H), 8.76 (s; 1H), 11.86 (s; 1H), 13.83 (s; 1H). **¹³C-NMR (126 MHz, DMSO):** δ = 11.0, 20.7, 41.3, 52.3, 106.9, 122.8, 124.7 (³J_{C,F} = 3.6 Hz), 124.7 (³J_{C,F} = 3.6 Hz), 125.0 (³J_{C,F} = 3.6 Hz), 128.9 (²J_{C,F} = 31.7 Hz), 129.2 (²J_{C,F} = 31.7 Hz), 129.5 (²J_{C,F} = 31.7 Hz), 129.7, 129.9, 132.2, 136.8, 138.2, 142.8, 147.4, 150.8, 154.5.

**Example 21:** 3-Methyl-8-(1-(3-(trifluoromethyl)benzyl)-1*H*-1,2,3-triazol-4-yl)-1*H*-purine-2,6(3*H*,7*H*)-dione **(1e)**

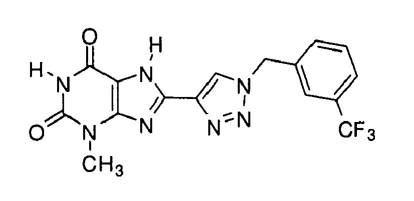

Reaction conditions: According to the general synthesis procedure 0.20 g (1.04 mmol, 1.0 eq.) 8-ethynyl-3-methyl-1*H*-purine-2,6(3*H*,7*H*)-dione **(3e)** and 0.42 g (2.08 mmol, 2.0 eq.) 1-(azidomethyl)-3-(trifluoromethyl)benzene **(4a)** were used. The crude product was purified by precipitation by dissolving in acetone and treatment with diluted hydrochloric acid and petroleum ether. A white precipitate resulted at the interface. The precipitate was filtered off and washed with water and petroleum ether. The solid was dried at 50-75 °C in a drying oven. The product **1e** was isolated as a rose solid in 90 % yield (0.37 g).
Analytical data: **mp** 337 °C. **TLC:** R_{f} = 0.61 (silica gel, dichloromethane/methanol 9 : 1). **LC-MS (m/z):** 392.5 [M+H]⁺ + 203.1 [M-N₂-CH₂-C₆H₄-CF₃-H]⁻ + 390.0 [M-H]⁻. Purity by **HPLC-UV (254 nm)-ESI-MS:** 99.5 %. **¹H-NMR (500 MHz, DMSO):** δ = 3.40 (s; 3H), 5.83 (s; 2H), 7.63-7.69 (m; 2H), 7.73-7.74 (m; 1H), 7.81 (s; 1H), 8.81 (s; 1H), 11.09 (s; 1H), 13.97 (s; 1H). **¹³C-NMR (126 MHz, DMSO):** δ = 28.7, 52.3, 107.4, 122.8, 124.7 (³J_{C,F} = 3.8 Hz), 124.8 (³J_{C,F} = 3.8 Hz), 125.0 (³J_{C,F} = 3.4 Hz), 125.0 (³J_{C,F} = 3.4 Hz), 127.1, 129.0 (²J_{C,F} = 31.8 Hz), 129.2 (²J_{C,F} = 31.8 Hz), 129.5 (²J_{C,F} = 31.8 Hz), 129.7 (²J_{C,F} = 31.8 Hz), 129.9, 132.2, 136.8, 138.2, 142.6, 150.0, 151.0, 154.2.

**Example 22:** 1,3,7-Trimethyl-8-(1-(3-(trifluoromethyl)benzyl)-1*H*-1,2,3-triazol-4-yl)-1*H*-purine-2,6(3*H*,7*H*)-dione (1h)

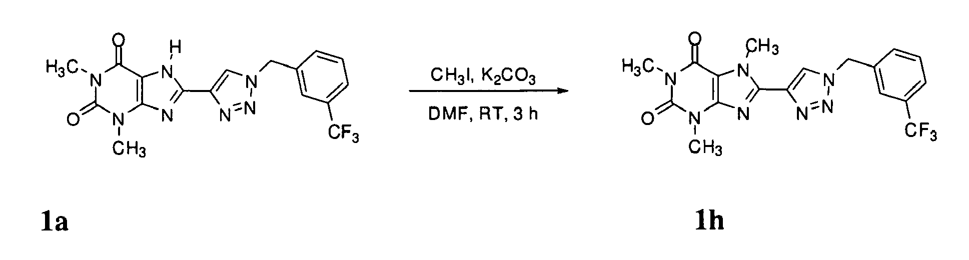

In a 50 mL round bottom flask with magnetic stirrer and reflux condenser, 0.11 g (0.26 mmol, 1.0 eq.) 1,3-dimethyl-8-(1-(3-(trifluoro-methyl)benzyl)-1*H*-1,2,3-triazol-4-yl)-1*H*-purine-2,6(3*H*,7*H*)-dione **(**1a) and 54.0 mg (0.39 mmol, 1.5 eq.) potassium carbonate were suspended in 10 mL dimethylformamide. Then, 92.0 mg (0.65 mmol, 2.5 eq.) iodomethane was added dropwise. After stirring for 3 h at room temperature water was added. The precipitate was filtered off and washed with water. The crude product was purified by precipitation by dissolving in acetone and treatment with diluted hydrochloric acid and petroleum ether. The precipitate was filtered off and washed with water and petroleum ether. The solid was dried at 50-75 °C in a drying oven. The product **1h** was isolated as a white solid in 82 % yield (89.1 mg). Analytical data: **mp** 223 °C. **TLC:** R_{f} = 0.54 (silica gel, dichloromethane/acetone 9 : 2). **LC-MS (m/z):** 420.1 [M+H]⁺ + 217.1 [M-CH₂-C₆H₄-CF₃-3CH₃+2H]⁻ + 363.3 [M-56.1]⁻ + 375.0 [M-N₂-CH₃-H]⁻ + 390.3 [M-N₂-H]⁻ + 418.4 [M-H]⁻ + 478.3 [M+CH₃COO]⁻. Purity by **HPLC-UV (254 nm)-ESI-MS:** 99 %. **¹H-NMR (500 MHz, DMSO):** δ = 3.21 (s; 3H), 3.42 (s; 3H), 4.24 (s; 3H), 5.82 (s; 2H), 7.63-7.66 (m; 1H), 7.69-7.74 (m; 2H), 7.85 (s; 1H), 8.96 (s; 1H). **¹³C-NMR (126 MHz, DMSO):** δ = 27.4, 29.2, 33.6, 52.3, 107.3, 120.6 (¹J_{C,F} = 272.4 Hz), 122.8 (¹J_{C,F} = 272.4 Hz), 124.8 (³J_{C,F} = 3.8 Hz), 124.8 (³J_{C,F} = 3.8 Hz), 125.0 (³J_{C,F} = 3.6 Hz), 125.0 (³J_{C,F} = 3.6 Hz), 126.6, 127.1 (¹J_{C,F} = 272.4 Hz), 128.9 (²J_{C,F} = 31.8 Hz), 129.2 (²J_{C,F} = 31.8 Hz), 129.4 (²J_{C,F} = 31.8 Hz), 129.7 (²J_{C,F} = 31.8 Hz), 129.9, 132.2, 136.7, 138.2, 142.4, 147.5, 150.7, 154.3.

**Example 23:** 8-(1-(4-Fluorophenyl)-1*H*-1,2,3-triazol-4-yl)-1,3,7-trimethyl-1*H-*purine-2,6(3*H*,7*H*)-dione **(1z)**

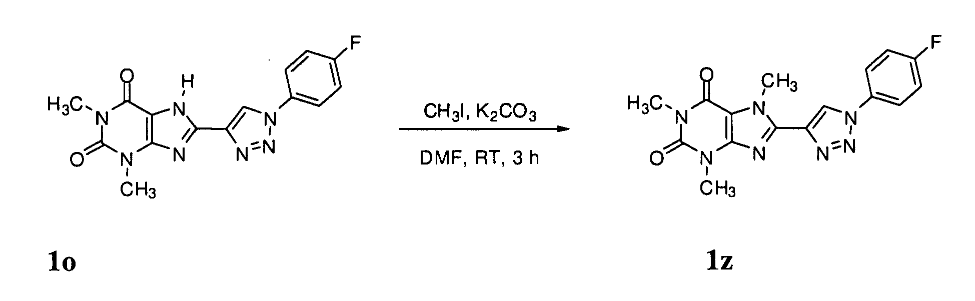

Reaction conditions: According to the synthesis procedure for **1h** 0.15 g (0.44 mmol, 1.0 eq.) 8-(1-(4-fluorophenyl)-1H-1,2,3-triazol-4-yl)-1,3-dimethyl-1*H-*purine-2,6(3*H*,7*H*)-dione **(1o)** was used. The product **1z** was isolated as an ocher solid in 92 % yield (0.15 g).
Analytical data: **mp** 268 °C. **TLC:** R_{f} = 0.74 (silica gel, acetone). **LC-MS (m/z):** 328.1 [M-N₂+H]⁺ + 356.1 [M+H]⁺ + 378.3 [M+Na]⁺ + 299.3 [M-N₂-2CH₃+2H]⁻ + 312.1 [M-N₂-CH_{3]}⁻ + 326.0 [M-N₂-H]⁻ + 414.0 [M+CH₃COO]⁻. Purity by **HPLC-UV (254 nm)-ESI-MS:** 98 %. **¹H-NMR (500 MHz, DMSO):** δ = 3.25 (s; 3H), 3.48 (s; 3H), 4.31 (s; 3H), 7.48-7.51 (m; 2H), 8.08-8.10 (m; 2H), 9.44 (s; 1H). **¹³C-NMR (126 MHz, DMSO):** δ=27.5, 29.4, 33.7, 107.6, 116.5 (²J_{C,F} = 23.3 Hz), 116.7 (²J_{C,F} = 23.3 Hz), 122.7 (³J_{C,F} = 8.9 Hz), 122.8 (³J_{C,F} = 8.9 Hz), 124.3, 132.5, 138.7, 142.1, 147.6, 150.8, 154.4, 160.9 (¹J_{C,F} = 246.6 Hz), 162.8 (¹J_{C,F} = 246.6 Hz).

**Synthesis procedure of selective alkylation** 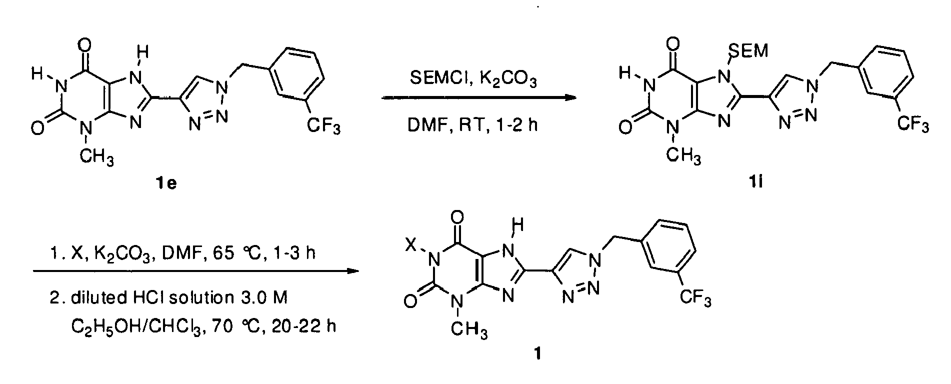 wherein X is defined as above.

**Part A:** In a 50 mL round bottom flask with magnetic stirrer and reflux condenser, 0.14 g (0.35 mmol, 1.0 eq.) 3-methyl-8-(1-(3-(trifluoromethyl)benzyl)-1*H-*1,2,3-triazol-4-yl)-1*H*-purine-2,6(3*H*,7*H*)dione **(1e)** and 48.7 mg (0.35 mmol, 1.0 eq.) potassium carbonate were suspended in 2.5 mL dimethylformamide. After stirring for a short time (5 - 10 min) 61.4 mg (0.37 mmol, 1.1 eq.) 2-(trimethylsilyl)ethoxymethyl chloride (SEMCl), dissolved in 2.5 mL dimethylformamide, was added slowly. After stirring for 1-2 h at room temperature ice water was added. The precipitate was filtered off and washed with cold water. The solid was dried at 50-75 °C in a drying oven. The crude product was purified four times by column chromatography on silica gel (eluent: dichloromethane/ methanol (9.5 : 0.5).

**Part B:** In a 50 mL round bottom flask with magnetic stirrer and reflux condenser, 0.15 g (0.30 mmol, 1.0 eq.) 3-methyl-8(1-(3-(trifluoromethyl)benzyl)-1*H-*1,2,3-triazol-4-yl)-7-((2-(trimethyl-silyl)ethoxy)methyl)-1*H*-purine-2,6(3*H*,7*H*)-dione (**1i**) and 0.11 g (0.78 mmol, 2.6 eq.) potassium carbonate were suspended in 4 mL dimethylformamide. Alkylating reagent (3-4 eq.) was added and heated for 1-3 h at 65 °C. Then, it was cooled to room temperature. 3 mL chloroform, 5 mL ethanol and 4 mL of diluted hydrochloric acid solution 3.0 M were added successively. After 20-22 h at 70 °C it was cooled to room temperature and water was added. After constriction of the solvent the remaining precipitate was filtered off and washed with water. The solid was dried at 50-75 °C in a drying oven.

**Example 24:** 3-Methyl-8-(1-(3-(trifluoromethyl)benzyl)-1*H*-1,2,3-triazol-4-yl)-7-((2-(trimethylsilyl)ethoxy)methyl)-1*H*-purine-2,6(3*H*,7*H*)-dione (**1i**)

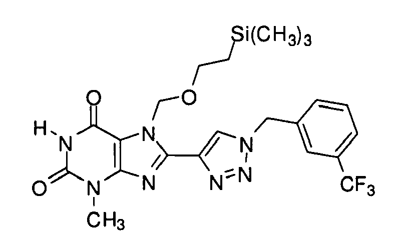

Reaction conditions: According to the synthesis procedure of selective alkylation (Part A) the product **1i** was isolated as a white solid in 92 % yield (0.17 g).
Analytical data: **mp** 292 °C. **TLC:** R_{f} = 0.42 (silica gel, dichloromethane/methanol 9.5 : 0.5). **LC-MS (m/z):** 344.3 [M-177.3]⁺ + 364.3 [M-CF₃-CH₂-C₆H₄+2H]⁺ + 391.3 [M-CH₂-O-2CH₂-Si(CH₃)₃+H]⁺ + 404.4 [M-O-2CH₂-Si(CH₃)₃]⁺ + 420.4 [M-2CH₂-Si(CH₃)₃]⁺ + 436.4 [M-CH₂-Si(CH₃)₃+2H]⁺ + 464.2 [M-2CH₃-N₂+H]⁺ + 492.4 [M-2CH₃+H]⁺ + 522.6 [M+H]⁺ + 544.3 [M+Na]⁺ + 232.1 [M-CF₃-CH₂-C₆H₄+H]⁻ + 333.1 [M-CF₃-CH₂-C₆H₄-N₂]⁻ + 362.3 [M-CF₃-CH₂-C₆H₄]⁻ + 492.3 [M-N₂-H]⁻ + 520.4 [M-H]⁻. Purity by **HPLC-UV (254 nm)-ESI-MS:** 100 %. **¹H-NMR (500 MHz, DMSO):** δ = -0.22 (s; 9H), 0.74-0.77 (t; ³J_{H,H} = 7.9 Hz, 2H), 3.40 (s; 3H), 3.53-3.56 (t; ³J_{H,H} = 7.9 Hz, 2H), 5.84 (s; 2H), 6.10 (s; 2H), 7.63-7.66 (m; 1H), 7.70-7.74 (m; 2H), 7.82 (s; 1H), 8.95 (s; 1H), 11.24 (s; 1H). **¹³C-NMR (126 MHz, DMSO):** δ = - 1.8, 16.9, 28.4, 52.3, 65.4, 73.5, 107.4, 122.8, 124.8 (³J_{C,F} = 3.7 Hz), 124.8 (³J_{C,F} = 3.7 Hz), 125.0 (³J_{C,F} = 3.6 Hz), 125.0 (³J_{C,F} = 3.6 Hz), 126.7, 129.0 (²J_{C,F} = 31.9 Hz), 129.2 (²J_{C,F} = 31.9 Hz), 129.5 (²J_{C,F} = 31.9 Hz), 129.7 (²J_{C,F} = 31.9 Hz), 129.9, 132.3, 136.7, 137.8, 143.0, 149.5, 150.6, 154.5.

**Example 25:** 3-Methyl-1-(prop-2-ynyl)-8-(1-(3-(trifluoromethyl)-benzyl)-1*H-*1,2,3-triazol-4-yl)-1*H*-purine-2,6(3*H*,7*H*)-dione **(If)**

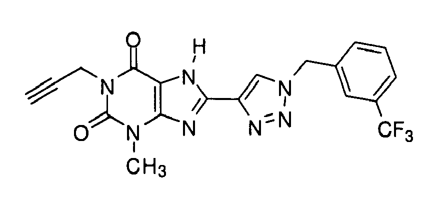

Reaction conditions: According to the synthesis procedure of selective alkylation (Part B) 0.17 g (1.15 mmol, 3.9 eq.) 3-bromo-1-propyne was used. The product **If** was isolated as a white solid in 86 % yield (0.11 g).
Analytical data: **mp** 263 °C. **TLC:** R_{f} = 0.35 (silica gel, dichloromethane/methanol 9.5 : 0.5). **LC-MS (m/z):** 430.2 [M+H]⁺ + 241.1 [M-N₂-CH₂-C₆H₄-CF₃-H]⁻ + 373.3 [M-C₃H₃-CH₃-2H]⁻ + 400.1 [M-N₂-H]⁻ + 428.2 [M-H]⁻. Purity by **HPLC-UV (254 nm)-ESI-MS:** 97 %. **¹H-NMR (500 MHz, DMSO):** δ = 3.07-3.08 (t; ⁴J_{H,H} = 2.4 Hz, 1H), 3.49 (s; 3H), 4.62 (d; ⁴J_{H,H} = 2.4 Hz, 2H), 5.83 (s; 2H), 7.64-7.70 (m; 2H), 7.73-7.75 (m; 1H), 7.82 (s; 1H), 8.84 (s; 1H), 14.14 (s; 1H). **¹³C-NMR (126 MHz, DMSO): δ** = 29.7, 30.1, 52.3, 72.6, 79.6, 106.8, 120.6 (¹J_{C,F} = 272.1 Hz), 122.8 (¹J_{C,F} = 272.1 Hz), 124.8 (³J_{C,F} = 3.7 Hz), 124.8 (³J_{C,F} = 3.7 Hz), 125.0 (³J_{C,F} = 3.7 Hz), 125.0 (³J_{C,F} = 3.7 Hz), 127.1 (¹J_{C,F} = 272.1 Hz), 129.0 (²J_{C,F} = 31.8 Hz), 129.2 (²J_{C,F} = 31.8 Hz), 129.5 (²J_{C,F} = 31.8 Hz), 129.7 (²J_{C,F} = 31.8 Hz), 129.9, 132.3, 136.8, 138.0, 143.1, 148.7, 150.2, 152.8.

**Example 26:** 1-(Cyclopropylmethyl)-3-methyl-8-(1-(3-(trifluoromethyl)benzyl)-1*H*-1,2,3-triazol-4-yl)-1*H*-purine-2,6(3*H*,7*H*)-dione **(1g)**

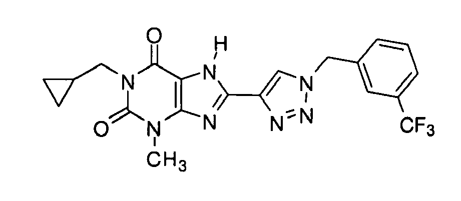

According to the synthesis procedure of selective alkylation (Part B) 0.18 g (1.31 mmol, 4.4 eq.) (bromomethyl)cyclopropane was used. The product **1g** was isolated as a white solid in 100 % yield (0.13 g).
Analytical data: **mp** 237 °C. **TLC:** R_{f} = 0.40 (silica gel, dichloromethane/methanol 9.5 : 0.5). **LC-MS (m/z):** 446.2 [M+H]⁺ + 468.4 [M+Na]⁺ + 242.3 [M-N₂-CH₂-C₆H₄-CF₃-H-CH₃]⁻ + 257.2 [M-N₂-CH₂-C₆H₄-CF₃-H]⁻ + 416.2 [M-N₂-H]⁻ + 444.2 [M-H]⁻. Purity by **HPLC-UV (254 nm)-ESI-MS:** 100 %. **¹H-NMR (500 MHz, DMSO):** δ = 0.33-0.36 (m; 2H), 0.40-0.43 (m; 2H), 1.16-1.22 (m; 1H), 3.49 (s; 3H), 3.77-3.79 (d; ³J_{H,H} = 7.1 Hz, 2H), 5.83 (s; 2H), 7.63-7.70 (m; 2H), 7.73-7.75 (m; 1H), 7.82 (s; 1H), 8.83 (s; 1H), 14.03 (s; 1H). **¹³C-NMR (126 MHz, DMSO):** δ = 3.4, 9.9, 29.6, 31.8, 44.8, 52.3, 107.1, 120.6 (¹J_{C,F} = 272.3 Hz), 122.8 (¹J_{C,F} = 272.3 Hz), 124.8 (³J_{C,F} = 3.7 Hz), 124.8 (³J_{C,F} = 3.7 Hz), 125.0 (³J_{C,F} = 3.6 Hz), 125.0 (³J_{C,F} = 3.6 Hz), 127.1 (¹J_{C,F} = 272.3 Hz), 129.0 (²J_{C,F} = 31.8 Hz), 129.2 (²J_{C,F} = 31.8 Hz), 129.5 (²J_{C,F} = 31.8 Hz), 129.7 (²J_{C,F} = 31.8 Hz), 129.9, 132.3, 135.3, 136.8, 138.1, 142.7, 148.4, 151.0, 153.9.

### Synthesis of intermediate products for compounds of general formulae II

The preparation of the key intermediate products **5a** and **5c** and those which are not previously described in the literature.

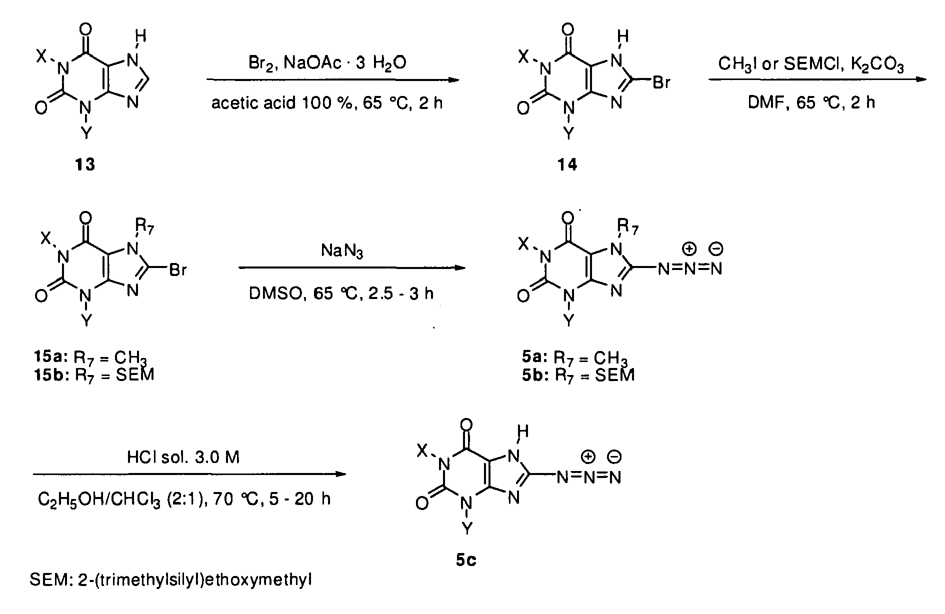

For the synthesis of 8-(1*H*-1,2,3-triazol-1-yl)-1*H*-purine-2,6(3*H*,7*H*)-dione derivatives of formula **2,** first the 8-azido-1*H*-purine-2,6(3*H*,7*H*)-dione derivatives of formula **5** were prepared.

The 8-azido-1*H*-purine-2,6(3*H*,7*H*)-dione derivatives of formula **5** were synthesized starting from theophylline (**13**) by bromination at position 8, followed by alkylation with iodomethane or 2-(trimethylsilyl)ethoxymethyl chloride at position 7 to **15a** or **15b** and subsequent reaction with sodium azide to **5a** or **5b.** After hydrolysis of the SEM-protective group, the 8-azido-1*H*-purine-2,6(3H,7H)-dione derivative **5c** was obtained.

**Example 27:** 8-Azido-1,3,7-trimethyl-1*H*-purine-2,6(3H,7*H*)-dione **(5a)**

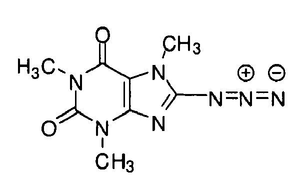

In a 50 mL round bottom flask with magnetic stirrer and reflux condenser, 0.78 g (2.86 mmol, 1.0 eq.) 8-bromo-1,3,7-trimethyl-1*H*-purine-2,6(3*H*,7*H*)-dione **(15a)** was dissolved in 8 mL dimethyl sulfoxide. Then, 0.28 g (4.29 mmol, 1.5 eq.) sodium azide was added and the reaction stirred for 2 h at 65 °C. After cooling to room temperature water was added. The precipitate was filtered off and washed with water. The solid was dried at 50-75 °C in a drying oven. The product **5a** was isolated as a light yellow solid in 89 % yield (0.60 g).
Analytical data: **mp** 264 °C. **TLC:** R_{f} = 0.55 (silica gel, dichloromethane/methanol 9.5 : 0.5). **LC-MS (m/z):** 208.1 [M-N₂+H]⁺ + 236.1 [M+H]⁺ + 163.9 [M-N₃-2CH₃]⁻ + 179.1 [M-N₃-CH_{2]}⁻ + 194.1 [M-N₃+H]⁻ + 205.9 [M-N₂-H]⁻. Purity by **HPLC-UV (254 nm)-ESI-MS:** 94 %. **¹H-NMR (500 MHz, DMSO):** δ = 3.21 (s; 3H), 3.39 (s; 3H), 3.63 (s; 3H). **¹³C-NMR (126 MHz, DMSO):** δ = 27.3, 29.4, 30.8, 105.4, 122.6, 146.5, 150.6, 153.8. **FT-IR:** ν̃ = 2955 [w], 2172 [s; ν(N₃)], 1708 [s], 1610 [w], 1546 [m], 1516 [s], 1443 [s], 1288 [w], 1242 [w], 1219 [m], 1035 [w], 980 [w], 756 [w], 745 [m], 711 [w], 674 [w], 646 [w], 511 [w], 411 [w].

**Example 28:** 8-Bromo-1,3-dimethyl-7-((2-(trimethylsilyl)ethoxy)-methyl)-1*H-*purine-2,6(3*H*,7*H*)-dione **(15b)**

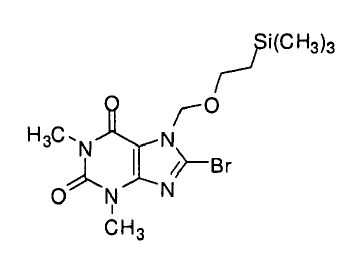

In a 250 mL round bottom flask with magnetic stirrer and reflux condenser, 2.53 g (9.78 mmol, 1.0 eq.) of 8-bromo-1,3-dimethyl-1*H*-purine-2,6(3*H*,7*H*)-dione **(14)** and 1.49 g (10.8 mmol, 1.1 eq.) of potassium carbonate were suspended in 50 mL dimethylformamide. After stirring for a short time (5 - 10 min) 2.44 g (14.7 mmol, 1.5 eq.) 2-(trimethylsilyl)-ethoxymethyl chloride (SEMCl) was added slowly. After stirring for 2 h at 65 °C the reaction was cooled to room temperature and water was added. The precipitate was filtered off and washed with water. The solid was dried at 50-75 °C in a drying oven. The product **15b** was isolated as a white solid in 95 % yield (3.62 g).
Analytical data: **mp** 84 °C. **TLC:** R_{f} = 0.68 (silica gel, dichloromethane/methanol 9.5 : 0.5). **LC-MS (m/z):** 214.1 [M-68.3]⁺ + 259.3 [M-23.1]⁺ + 273.0 [M-CH₂-O-2CH₂-Si(CH₃)₃+NH₄]⁺ + 333.3 [M+50.9]⁺ + 361.5 [M+79.1]⁺ + 389.4 [M+H]⁺+ 413.1 [M+Na]⁺ + 200.1 [M-82.3]⁻ + 225.3 [M-57.1]⁻ + 244.1 [M-CH₂-O-2CH₂-Si(CH₃)₃+CH₃]⁻ + 251.1 [M-31.3]⁻ + 265.0 [M-17.4]⁻. Purity by **HPLC-UV (254 nm)-ESI-MS:** 99 %. **¹H-NMR (500 MHz, DMSO):** δ = - 0.05 (s; 9H), 0.84-0.88 (t; ³J_{H,H} = 8.0 Hz, 2H), 3.23 (s; 3H), 3.41 (s; 3H), 3.61-3.64 (t; ³J_{H,H} = 8.0 Hz, 2H), 5.63 (s; 2H). **¹³C-NMR (126 MHz, DMSO):** δ = - 1.53, 17.0, 27.5, 29.5, 65.9, 74.3, 108.2, 128.7, 147.6, 150.5, 153.4.

**Example 29:** 8-Azido-1,3-dimethyl-7-((2-(trimethylsilyl)ethoxy)-methyl)-1*H-*purine-2,6(3*H*,7*H*)-dione **(5b)**

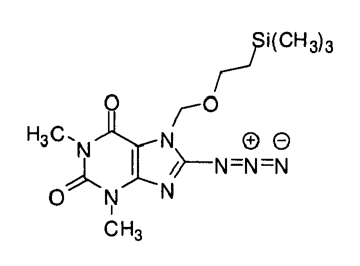

In a 100 mL round bottom flask with magnetic stirrer and reflux condenser, 3.45 g (8.87 mmol, 1.0 eq.) 8-bromo-1,3-dimethyl-7-((2-(trimethylsilyl)ethoxy)methyl)-1*H*-purine-2,6(3*H*,7*H*)-dione (**15b**) was dissolved in 30 mL dimethyl sulfoxide. Then, 1.15 g (17.7 mmol, 2.0 eq.) sodium azide was added and the reaction stirred for 2 h at 65 °C. After cooling to room temperature water was added. The precipitate was filtered off and washed with water. The solid was dried at 50-75 °C in a drying oven. The product **5b** was isolated as a light yellow solid in 99 % yield (3.09 g).
Analytical data: **mp** 122 °C. **TLC:** R_{f} = 0.75 (silica gel, dichloromethane/methanol 9.5 : 0.5). **LC-MS (m/z):** 206.1 [M-O-2CH₂-Si(CH₃)₃-2CH₃+2H]⁺ + 266.5 [M-CH₂-Si(CH₃)₃+2H]⁺ + 290.5 [M-60.9]⁺ + 324.3 [M-N₂+H]⁺ + 374.5 [M+Na]⁺ + 179.1 [M-172.3]⁻ + 194.1 [M-157.3]⁻. Purity by **HPLC-UV (254 nm)-ESI-MS:** 98 %. **¹H-NMR (500 MHz, DMSO):** δ = - 0.05 (s; 9H), 0.83-0.86 (t; ³J_{H,H} = 8.0 Hz, 2H), 3.22 (s; 3H), 3.41 (s; 3H), 3.59-3.62 (t; ³J_{H,H} = 8.0 Hz, 2H), 5.41 (s; 2H). **¹³C-NMR (126 MHz, DMSO):** δ = - 1.57, 17.0, 27.4, 29.5, 65.9, 72.0, 104.9, 145.9, 146.8, 150.6, 153.5. **FT-IR:** ν̃ = 2951 [m], 2897 [w], 2138 [s; ν(N₃)], 1702 [s], 1609 [w], 1547 [m], 1487 [s], 1441 [s], 1378 [m], 1296 [m], 1249 [m], 1213 [m], 1118 [m], 1092 [s], 1032 [w], 978 [w], 961 [w], 947 [m], 928 [m], 862 [m], 835 [s], 760 [m], 752 [m], 744 [s], 715 [w], 688 [m], 655 [w], 608 [w], 527 [w], 507 [m], 407 [m].

**Example 30:** 8-Azido-1,3-dimethyl-1*H*-purine-2,6(3*H*,7*H*)-dione (**5c**)

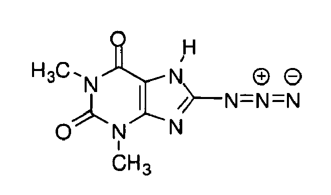

In a 250 mL round bottom flask with magnetic stirrer and reflux condenser, 3.08 g (8.77 mmol, 1.0 eq.) 8-azido-1,3-dimethyl-7-((2-(trimethylsilyl)ethoxy)methyl)-1*H*-purine-2,6(3H,7*H*)-dione (**5b**) was dissolved in 27 mL chloroform. Then, 53 mL ethanol and 85 mL of diluted hydrochloric acid solution 7.7 M were added successively. After stirring for 87 h at 70 °C the reaction was cooled to room temperature and water was added. After constriction of the solvent the remaining precipitate was filtered off and washed with less water. The solid was dried at 50-75 °C in a drying oven. The product **5c** was isolated as a light yellow solid in 92 % yield (1.77 g).
Analytical data: **mp** 201 °C. **TLC:** R_{f} = 0.32 (silica gel, dichloromethane/methanol 9.5 : 0.5). **LC-MS (m/z):** 222.3 [M+H]⁺ + 220.1 [M-H]⁻ + 269.0 [M+47.8]⁻ + 312.1 [M+90.9]⁻. Purity by **HPLC-UV (254 nm)-ESI-MS:** 99.5 %. **¹H-NMR (500 MHz, DMSO):** δ = 3.21 (s; 3H), 3.38 (s; 3H), 13.48 (s; 1H). **¹³C-NMR (126 MHz, DMSO):** δ = 27.5, 29.7, 105.1, 145.1, 147.0, 150.8, 153.4. **FT-IR:** ν̃ = 2191 [s; ν(N₃)], 2148 [s; ν(N₃)], 1697 [s], 1665 [s], 1606 [w], 1557 [w], 1542 [w], 1508 [s], 1499 [s], 1458 [w], 1436 [w], 1391 [s], 1278 [m], 1221 [m], 1174 [m], 1102 [w], 1054 [w], 1008 [w], 983 [m], 817 [w], 789 [m], 757 [m], 745 [m], 712 [w], 676 [m], 500 [s], 410 [m].

The present invention will be explained in more detail in the following examples. However, the examples are only used for illustration and do not limit the scope of the present invention.

### Examples

General synthesis procedure of 8-(1*H*-1,2,3-triazol-1-yl)-1*H*-purine-2,6(3*H*,7*H*)-dione derivatives **2** 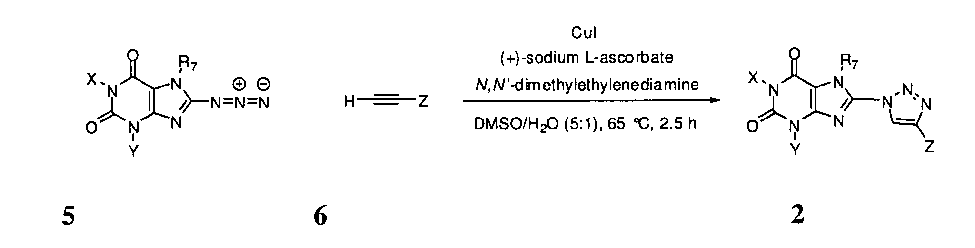 wherein X and Y are defined as above.

In a 50 mL round bottom flask with magnetic stirrer and reflux condenser, 19.0 mg (0.10 mmol, 0.20 eq.) copper (I) iodide, 19.8 mg (0.10 mmol, 0.20 eq.) (+)-sodium L-ascorbate and (0.50 mmol, 1.0 eq.) 8-azido-1H-purine-2,6(3*H*,7*H*)-dione derivative **5** were suspended in 5.0 mL dimethyl sulfoxide and 1.0 mL water. Then, (0.76-1.62 mmol, 1.5-3.2 eq.) alkyne of general formula **6** and 17.0 µL (0.15 mmol, 0.30 eq.) *N,N*'-dimethylethylenediamine were added and heated at 65 °C under argon atmosphere for 2.5 h. Then, the mixture was cooled to room temperature and water and diluted hydrochloric acid were added. The precipitate was filtered off and washed with water. The solid was dried at 50-75 °C in a drying oven.

**Example 31:** 1,3-Dimethyl-8-(4-(3-(trifluoromethyl)phenyl)-1*H*-1,2,3-triazol-1-yl)-1*H*-purine-2,6(3*H*,7*H*)-dione **(2a)**

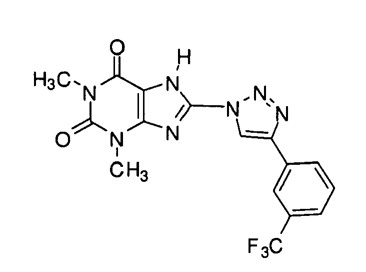

Reaction conditions: According to the general synthesis procedure 0.11 g (0.50 mmol, 1.0 eq.) 8-azido-1,3-dimethyl-1*H*-purine-2,6(3*H*,7*H*)-dione **(5a)** and 0.15 g (0.87 mmol, 1.7 eq.) 1-ethynyl-3-(trifluoromethyl)benzene **(6a)** were used. The product **2a** was isolated as a light yellow solid in 100 % yield (0.20 g).
Analytical data: **mp** 294 °C. **TLC:** R_{f} = 0.41 (silica gel, dichloromethane/methanol 9 : 1). **LC-MS (m/z):** 364.1 [M-N₂+H]⁺ + 392.5 [M+H]⁺ + 414.1 [M+Na]⁺ + 347.1 [M-N₂-CH₃-H]⁻ + 362.3 [M-N₂-H]- + 390.1 [M-H]⁻. Purity by **HPLC-UV (254 nm)-ESI-MS:** 99 %. **¹H-NMR (500 MHz, DMSO):** δ = 3.24 (s; 3H), 3.46 (s; 3H), 7.70-7.71 (m; 2H), 8.28-8.29 (m; 1H), 8.33 (s; 1H). **¹³C-NMR** (**126 MHz, DMSO):** δ = 27.5, 30.0, 114.4, 121.6 (³J_{C,F} = 3.7 Hz), 121.6 (³J_{C,F} = 3.7 Hz), 123.1 (¹J_{C,F} = 272.2 Hz), 124.2 (³J_{C,F} = 3.6 Hz), 124.3 (³J_{C,F} = 3.6 Hz), 125.2 (¹J_{C,F} = 272.2 Hz), 129.1, 129.6 (²J_{C,F} = 31.8 Hz), 129.9 (²J_{C,F} = 31.8 Hz), 130.0, 131.7, 144.2, 147.6, 149.1, 151.7, 157.5.

**Example 32:** 1,3-Dimethyl-8-(4-(4-(trifluoromethyl)phenyl)-1*H*-1,2,3-triazol-1-yl)-1*H*-purine-2,6(3*H*,7*H*)-dione (**2b**)

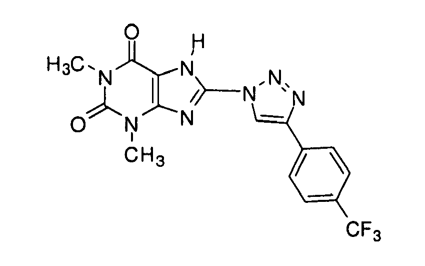

Reaction conditions: According to the general synthesis procedure 0.11 g (0.51 mmol, 1.0 eq.) 8-azido-1,3-dimethyl-1*H*-purine-2,6(3*H*,7*H*)-dione **(5a)** and 0.13 g (0.76 mmol, 1.5 eq.) 1-ethynyl-4-(trifluoromethyl)benzene **(6b)** were used. The product **2b** was isolated as a light yellow solid in 95 % yield (0.19 g).
Analytical data: mp 296 °C. **TLC:** R_{f} = 0.51 (silica gel, dichloromethane/methanol 9 : 1). **LC-MS** (m/z): 364.1 [M-N₂+H]⁺ + 392.4 [M+H]⁺ + 347.1 [M-N₂-CH₃-H]⁻ + 362.3 [M-N₂-H]⁻ + 390.0 [M-H]⁻. Purity by **HPLC-UV** (254 **nm)-ESI-MS:** 99 %. **¹H-NMR (500 MHz, DMSO):** δ = 3.24 (s; 3H), 3.46 (s; 3H), 7.81-7.82 (m; 2H), 8.20-8.21 (m; 2H). **¹³C-NMR (126 MHz, DMSO):** δ=27.4, 29.9, 114.4, 123.1 (¹J_{C,F} = 272.0 Hz), 125.3 (¹J_{C,F} = 272.0 Hz), 125.8, 127.8 (²J_{C,F} = 31.7 Hz), 128.1 (²J_{C,F} = 31.7 Hz), 134.6, 144.2, 147.6, 149.1, 151.7, 157.5.

**Example 33**: 8-(4-(3-Fluorophenyl)-1*H*-1,2,3-triazol-1-yl)-1,3-dimethyl-1*H-*purine-2,6(3*H*,7*H*)-dione (2c)

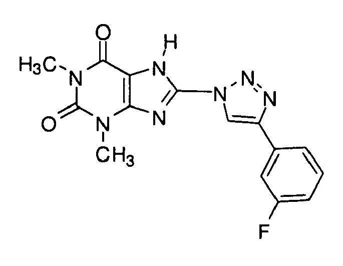

Reaction conditions: According to the general synthesis procedure 0.11 g (0.50 mmol, 1.0 eq.) 8-azido-1,3-dimethyl-1*H*-purine-2,6(3H,7*H*)-dione (**5a**) and 0.10 g (0.86 mmol, 1.7 eq.) 1-ethynyl-3-fluorobenzene (**6c**) were used. The product **2c** was isolated as a light yellow solid in 83 % yield (0.14 g).
Analytical data: mp 288 °C. **TLC**: R_{f} = 0.28 (silica gel, dichloromethane/methanol 9 : 1). **LC-MS (m/z)**: 314.4 [M-N₂+H]⁺ + 342.3 [M+H]⁺ + 364.3 [M+Na]⁺ + 297.3 [M-N₂-CH₃-H]⁻ + 312.1 [M-N₂-H]⁻ + 340.3 [M-H]⁻. Purity by HPLC-UV (254 nm)-ESI-MS: 99.5 %. ¹H-NMR (500 MHz, DMSO): δ = 3.24 (s; 3H), 3.46 (s; 3H), 7.15-7.19 (m; 1H), 7.49-7.53 (m; 1H), 7.77-7.84 (m; 2H). ¹³C-NMR (126 MHz, DMSO): δ = 27.4, 29.9, 111.8 (²J_{C,F} = 23.0 Hz), 111.9 (²J_{C,F} = 23.0 Hz), 114.4 (²J_{C,F} = 20.7 Hz), 114.5 (²J_{C,F} = 20.7 Hz), 121.3, 130.9 (³J_{C,F} = 8.5 Hz), 130.9 (³J_{C,F} = 8.5 Hz), 133.0 (³J_{C,F} = 8.6 Hz), 133.0 (³J_{C,F} = 8.6 Hz), 144.5, 144.5, 147.6, 149.1, 151.7, 157.5, 161.6 (¹J_{C,F} = 242.9 Hz), 163.5 (¹J_{C,F} = 242.9 Hz).

Example 34: 8-(4-(4-Fluorophenyl)-1*H*-1,2,3-triazol-1-yl)-1,3-dimethyl-1*H-*purine-2,6(3H,7H)-dione (2d)

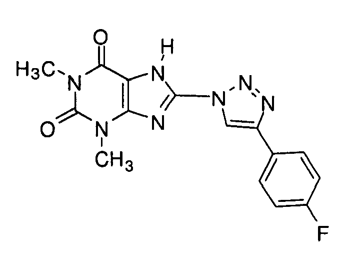

Reaction conditions: According to the general synthesis procedure 0.11 g (0.50 mmol, 1.0 eq.) 8-azido-1,3-dimethyl-1*H*-purine-2,6(3H,7*H*)-dione **(5a)** and 0.10 g (0.84 mmol, 1.7 eq.) 1-ethynyl-4-fluorobenzene **(6d)** were used. The product **2d** was isolated as a rose solid in 71 % yield (0.12 g).
Analytical data: **mp** 286 °C. **TLC:** R_{f} = 0.37 (silica gel, dichloromethane/methanol 9 : 1). **LC-MS (m/z):** 314.4 [M-N₂+H]⁺ + 342.3 [M+H]⁺ + 364.1 [M+Na]⁺ + 297.1 [M-N₂-CH₃-H]⁻ + 312.1 [M-N₂-H]⁻ + 340.3 [M-H]⁻. Purity by **HPLC-UV (254 nm)-ESI-MS:** 99.9 %. **¹H-NMR (500 MHz, DMSO):** δ = 3.24 (s; 3H), 3.45 (s; 3H), 7.29 (s; 2H), 8.02 (s; 2H). **¹³C-NMR (126 MHz, DMSO):** δ = 27.4, 29.9, 114.3, 115.6 (²J_{C,F} = 21.6 Hz), 115.8 (²J_{C,F} = 21.6 Hz), 127.2, 127.3 (³J_{C,F} = 8.1 Hz), 127.3 (³J_{C,F} = 8.1 Hz), 144.7, 147.7, 149.1, 151.7, 157.5, 160.7 (¹J_{C,F} = 244.4 Hz), 162.7 (¹J_{C,F} = 244.4 Hz).

**Example 35:** 8-(4-(2-Methoxyphenyl)-1*H*-1,2,3-triazol-1-yl)-1,3-dimethyl-1*H-*purine-2,6(3*H*,7*H*)-dione (**2e**)

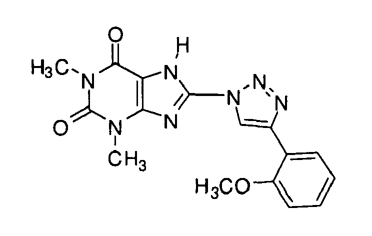

Reaction conditions: According to the general synthesis procedure 0.11 g (0.51 mmol, 1.0 eq.) 8-azido-1,3-dimethyl-1*H*-purine-2,6(3H,7*H*)-dione (**5a**) and 0.21 g (1.60 mmol, 3.1 eq.) 1-ethynyl-2-methoxybenzene (**6e**) were used. The crude product was purified by precipitation by dissolving in acetone and treatment with petroleum ether. The precipitate was filtered off and washed with petroleum ether. The solid was dried at 50-75 °C in a drying oven. The product **2e** was isolated as a light brown solid in 64 % yield (0.11 g).
Analytical data: **mp** 267 °C. **TLC:** R_{f} = 0.33 (silica gel, dichloromethane/methanol 9 : 1). **LC-MS (m/z):** 354.4 [M+H]⁺ + 376.3 [M+Na]⁺ + 294.1 [M-N₂-2CH₃-H]⁻ + 309.3 [M-N₂-CH₃-H]⁻ + 324.4 [M-N₂-H]⁻ + 352.1 [M-H]⁻. Purity by **HPLC-UV (254 nm)-ESI-MS:** 99.5 %. **¹H-NMR (500 MHz, DMSO):** δ = 3.24 (s; 3H), 3.46 (s; 3H), 3.97 (s; 3H), 7.09-7.10 (m; 1H), 7.16-7.18 (m; 1H), 7.36 (m; 1H), 8.21-8.22 (m; 1H). **¹³C-NMR (126 MHz, DMSO):** δ = 27.5, 30.0, 55.5, 111.6, 114.2, 118.8, 120.6, 126.7, 129.0, 141.0, 147.8, 149.2, 151.7, 155.5, 157.5.

**Example 36:** 8-(4-(3-Methoxyphenyl)-1*H*-1,2,3-triazol-1-yl)-1,3-dimethyl-1*H-*purine-2,6(3*H*,7*H*)-dione (**2f**)

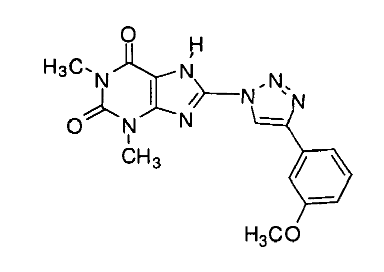

Reaction conditions: According to the general synthesis procedure 0.11 g (0.50 mmol, 1.0 eq.) 8-azido-1,3-dimethyl-1*H*-purine-2,6(3*H*,7*H*)-dione **(5a)** and 0.11 g (0.82 mmol, 1.7 eq.) 1-ethynyl-3-methoxybenzene **(6f)** were used. The crude product was purified by precipitation by dissolving in acetone and treatment with petroleum ether. The precipitate was filtered off and washed with petroleum ether. The solid was dried at 50-75 °C in a drying oven. The product **2f** was isolated as a white solid in 71 % yield (0.13 g).
Analytical data: **mp** 283 °C. **TLC:** R_{f} = 0.40 (silica gel, dichloromethane/methanol 9: 1). **LC-MS (m/z):** 354.3 [M+H]⁺ + 376.3 [M+Na]⁺ + 266.2 [M-N₂-OCH₃-2CH₃+H]⁻ + 281.2 [M-N₂-3CH₃+H]⁻ + 309.3 [M-N₂-CH₃-H]⁻ + 324.2 [M-N₂-H]⁻ + 352.1 [M-H]⁻. Purity by **HPLC-UV (254 nm)-ESI-MS:** 100 %. **¹H-NMR (500 MHz, DMSO):** δ = 3.24 (s; 3H), 3.46 (s; 3H), 3.83 (s; 3H), 6.90-6.92 (m; 1H), 7.35-7.38 (m; 1H), 7.54-7.56 (m; 2H). **¹³C-NMR (126 MHz, DMSO):** δ = 27.5, 30.0, 55.2, 110.4, 113.8, 114.3, 117.6, 130.0, 131.9, 145.5, 147.8, 149.1, 151.7, 157.6, 159.7.

**Example 37:** 8-(4-(4-Methoxyphenyl)-1*H*-1,2,3-triazol-1-yl)-1,3-dimethyl-1*H-*purine-2,6(3*H*,7*H*)-dione **(2g)**

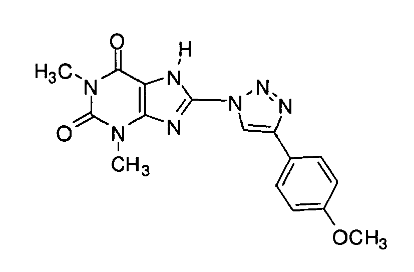

Reaction conditions: According to the general synthesis procedure 0.11 g (0.50 mmol, 1.0 eq.) 8-azido-1,3-dimethyl-1*H*-purine-2,6(3*H*,7*H*)-dione **(5a)** and 0.13 g (0.98 mmol, 2.0 eq.) 1-ethynyl-4-methoxybenzene **(6g)** were used. The crude product was purified by precipitation by dissolving in acetone and treatment with petroleum ether. The precipitate was filtered off and washed with petroleum ether. The solid was dried at 50-75 °C in a drying oven. The product **2g** was isolated as a light brown solid in 57 % yield (0.10 g).
Analytical data: **mp** 268 °C. **TLC:** R_{f} = 0.44 (silica gel, dichloromethane/methanol 9 : 1). **LC-MS (m/z):** 326.3 [M-N₂+H]⁺ + 354.3 [M+H]⁺ + 376.4 [M+Na]⁺ + 294.1 [M-N₂-2CH₃-H]⁻ + 309.3 [M-N₂-CH₃-H]⁻ + 352.0 [M-H]⁻. Purity by **HPLC-UV (254 nm)-ESI-MS:** 99.8 %. **¹H-NMR (500 MHz, DMSO):** δ = 3.24 (s; 3H), 3.45 (s; 3H), 3.80 (s; 3H), 7.02-7.03 (m; 2H), 7.88-7.89 (m; 2H). **¹³C-NMR (126 MHz, DMSO):** δ=27.5, 30.0, 55.2, 114.3, 123.2, 126.7, 145.6, 147.9, 149.2, 151.8, 157.6, 159.1.

**Example 38:** 8-(4-(2-(Hydroxymethyl)phenyl)-1*H*-1,2,3-triazol-1-yl)-1,3-dimethyl-1*H*-purine-2,6(3*H*,7*H*)-dione **(2h)**

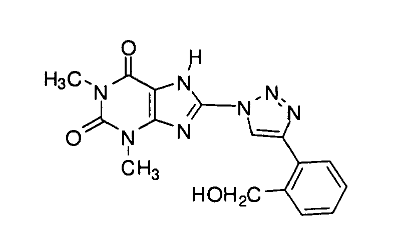

Reaction conditions: According to the general synthesis procedure 0.11 g (0.50 mmol, 1.0 eq.) 8-azido-1,3-dimethyl-1*H*-purine-2,6(3*H*,7*H*)-dione (**5a**) and 0.10 g (0.78 mmol, 1.6 eq.) (2-ethynylphenyl)methanol **(6h)** were used. The product **2h** was isolated as a light brown solid in 58 % yield (0.10 g).
Analytical data: **mp** 269 °C. **TLC:** R_{f} = 0.26 (silica gel, dichloromethane/methanol 9 : 1). **LC-MS (m/z):** 336.0 [M-CH₃-2H]⁺ + 354.4 [M+H]⁺ + 376.3 [M+Na]⁺ + 324.3 [M-N₂-H]⁻ + 352.1 [M-H]⁻. Purity by **HPLC-UV (254 nm)-ESI-MS:** 100 %. **¹H-NMR (500 MHz, DMSO):** δ = 3.24 (s; 3H), 3.46 (s; 3H), 4.67 (s; 2H), 7.38-7.40 (m; 2H), 7.57-7.59 (m; 1H), 7.85-7.87 (s; 1H). **¹³C-NMR (126 MHz, DMSO):** δ = 27.5, 30.0, 61.7, 114.3, 127.2, 127.8, 128.2, 128.5, 128.8, 143.0, 144.3, 147.7, 149.1, 151.7, 157.5.

**Example 39:** 8-(4-(4-(Hydroxymethyl)phenyl)-1*H*-1,2,3-triazol-1-yl)-1,3-dimethyl-1*H*-purine-2,6(3*H*,7*H*)-dione (**2i**)

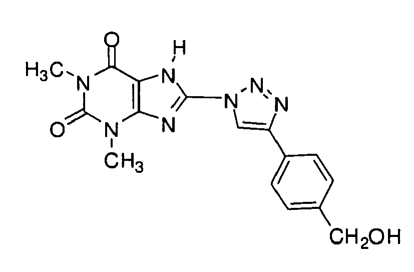

Reaction conditions: According to the general synthesis procedure 0.11 g (0.51 mmol, 1.0 eq.) 8-azido-1,3-dimethyl-1*H*-purine-2,6(3*H*,7*H*)-dione (**5a**) and 0.10 g (0.79 mmol, 1.6 eq.) (4-ethynylphenyl)methanol (**6i**) were used. The product **2i** was isolated as a light brown solid in 67 % yield (0.12 g).
Analytical data: **mp** 277 °C. **TLC:** R_{f} = 0.13 (silica gel, dichloromethane/methanol 9 : 1). **LC-MS (m/z):** 326.3 [M-N₂+H]⁺ + 354.4 [M+H]⁺ + 376.3 [M+Na]⁺ + 309.3 [M-N₂-CH₃-H]⁻ + 324.3 [M-N₂-H]⁻ + 352.1 [M-H]⁻. Purity by **HPLC-UV (254 nm)-ESI-MS:** 100 %. **¹H-NMR (500 MHz, DMSO):** δ = 3.25 (s; 3H), 3.46 (s; 3H), 4.53 (s; 2H), 7.40-7.42 (m; 2H), 7.93-7.94 (m; 2H), 8.99 (s; 1H). **¹³C-NMR (126 MHz, DMSO):** δ=27.4, 29.9, 62.7, 114.3, 125.0, 126.9, 128.9, 142.5, 145.6, 147.8, 149.1, 151.7, 157.5.

**Example 40:** 8-(4-(3-Hydroxyphenyl)-1*H*-1,2,3-triazol-1-yl)-1,3-dimethyl-1*H-*purine-2,6(3*H*,7*H*)-dione (2j)

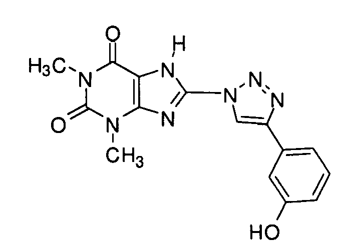

Reaction conditions: According to the general synthesis procedure 0.11 g (0.51 mmol, 1.0 eq.) 8-azido-1,3-dimethyl-1*H*-purine-2,6(3*H*,7*H*)-dione (**5a**) and 0.18 g (1.55 mmol, 3.0 eq.) 3-ethynylphenol **(6j)** were used. The product **2j** was isolated as a light brown solid in 70 % yield (0.12 g).
Analytical data: **mp** 291 °C. **TLC:** R_{f} = 0.29 (silica gel, dichloromethane/methanol 9 : 1). **LC-MS (m/z):** 340.3 [M+H]⁺ + 362.3 [M+Na]⁺ + 295.1 [M-N₂-CH₃-H]⁻ + 310.1 [M-N₂-H]⁻ + 338.3 [M-H]⁻. Purity by **HPLC-UV (254 nm)-ESI-MS:** 99 %. **¹H-NMR (500 MHz, DMSO):** δ = 3.24 (s; 3H), 3.45 (s; 3H), 6.15-6.17 (m; 1H), 6.54-6.55 (m; 1H), 6.72 (m; 1H), 6.80-6.83 (m; 1H). **¹³C-NMR (126 MHz, DMSO):** δ = 27.4, 29.9, 105.5, 114.1, 116.1, 119.2, 128.8, 130.5, 148.1, 149.1, 151.7, 157.6, 171.5.

**Example 41:** 1,3-Dimethyl-8-(4-(pyridin-2-yl)-1*H*-1,2,3-triazol-1-yl)-1*H-*purine-2,6(3*H*,7*H*-dione **(2k)**

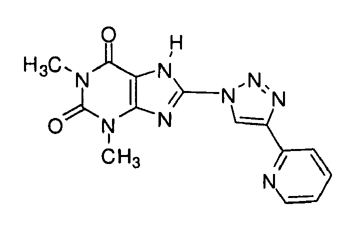

Reaction conditions: According to the general synthesis procedure 0.11 g (0.51 mmol, 1.0 eq.) 8-azido-1,3-dimethyl-1*H*-purine-2,6(3*H*,7*H*)-dione (**5a**) and 0.29 g (2.81 mmol, 5.5 eq.) 2-ethynylpyridine **(6k)** were used. The reaction was stirred for 18.5 h. The product was precipitated by water only. The crude product was purified by a second precipitation step using petroleum ether only. The precipitate was filtered off and washed with water. The solid was dried at 50-75 °C in a drying oven. The product **2k** was isolated as a yellow solid in 60 % yield (98.7 mg).

Analytical data: **mp** > 400 °C. **LC-MS (m/z)**: 325.3 [M+H]⁺ + 280.1 [M-N₂-CH₃-H]⁻ + 295.2 [M-N₂-H]⁻ + 323.3 [M-H]⁻. Purity by **HPLC-UV (254 nm)-ESI-MS:** 100 %. **¹H-NMR (500 MHz, DMSO):** δ = 3.24 (s; 3H), 3.45 (s; 3H), 7.36-7.38 (m; 1H), 7.90-7.94 (m; 1H), 8.10-8.11 (m; 1H), 8.63-8.64 (s; 1H). **¹³C-NMR (126 MHz, DMSO**): δ = 27.5, 30.0, 114.4, 119.8, 123.0, 137.2, 146.5, 147.6, 149.2, 149.7, 149.8, 151.7, 157.6.

**Example 42:** 1,3-Dimethyl-8-(4-(pyridin-3-yl)-1*H*-1,2,3-triazol-1-yl)-1*H-*purine-2,6(3*H*,7*H*)-dione **(21)**

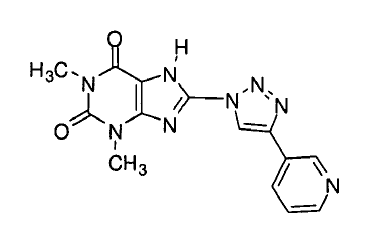

Reaction conditions: According to the general synthesis procedure 0.11 g (0.51 mmol, 1.0 eq.) 8-azido-1,3-dimethyl-1*H*-purine-2,6(3*H*,7*H*)-dione **(5a)** and 0.11 g (1.03 mmol, 2.0 eq.) 3-ethynylpyridine **(61)** were used. The product was precipitated by water only. The product **21** was isolated as a yellow solid in 89 % yield (0.15 g).
Analytical data: **mp** 344 °C. **LC-MS (m/z):** 297.5 [M-N₂+H]⁺ + 325.4 [M+H]⁺ + 347.3 [M+Na]⁺ + 280.3 [M-N₂-CH₃-H]⁻ + 295.1 [M-N₂-H]⁻ + 323.3 [M-H]⁻. Purity by **HPLC-UV (254 nm)-ESI-MS:** 100 %. **¹H-NMR (500 MHz, DMSO):** δ = 3.24 (s; 3H), 3.46 (s; 3H), 7.49-7.51 (m; 1H), 8.32-8.33 (m; 1H), 8.54-8.55 (m; 1H), 9.17 (s; 1H). **¹³C-NMR (126 MHz, DMSO):** δ = 27.5, 30.0, 114.4, 124.0, 126.6, 132.6, 142.9, 146.5, 147.7, 148.8, 149.2, 151.8, 157.6.

### Example 43: 8-(4-Benzyl-1H-1,2,3-triazol-1-yl)-1,3-dimethyl-1H-purine-2,6(3H,7H)-dione (2m)

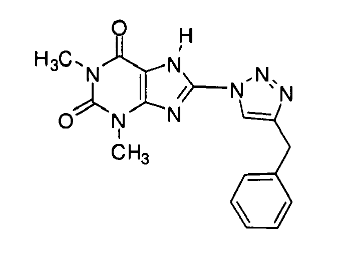

Reaction conditions: According to the general synthesis procedure 0.11 g (0.51 mmol, 1.0 eq.) 8-azido-1,3-dimethyl-1*H*-purine-2,6(3*H*,7*H*)-dione **(5a)** and 0.13 g (1.13 mmol, 2.2 eq.) prop-2-ynylbenzene **(6m)** were used. The crude product was purified by precipitation by dissolving in acetone and treatment with petroleum ether. The precipitate was filtered off and washed petroleum ether. The solid was dried at 50-75 °C in a drying oven. The product **2m** was isolated as a white solid in 57 % yield (97.8 mg).
Analytical data: **mp** 240 °C. **TLC:** R_{f} = 0.56 (silica gel, dichloromethane/methanol 9 : 1). **LC-MS (m/z):** 338.4 [M+H]⁺ + 360.3 [M+Na]⁺ + 293.2 [M-N₂-CH₃-H]⁻ + 308.2 [M-N₂-H]⁻ + 336.1 [M-H]⁻. Purity by **HPLC-UV (254 nm)-ESI-MS:** 99 %. **¹H-NMR (500 MHz, DMSO):** δ = 3.26 (s; 3H), 3.45 (s; 3H), 4.12 (d; 2H), 7.22-7.33 (m; 5H), 8.54 (s; 1H), 14.81 (s; 1H). **¹³C-NMR (126 MHz, DMSO):** δ = 27.7, 29.8, 30.7, 85.1, 121.5, 126.2, 126.3, 128.3, 128.4, 128.5, 138.0, 138.7, 140.5, 150.9, 154.1.
**Example 44:** 1,3-Dimethyl-8-(4-tosyl-1*H*-1,2,3-triazol-1-yl)-1*H*-purine-2,6(3*H*,7*H*)-dione **(2n)**

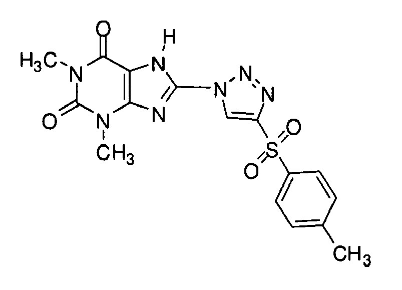

Reaction conditions: According to the general synthesis procedure 0.11 g (0.50 mmol, 1.0 eq.) 8-azido-1,3-dimethyl-1*H*-purine-2,6(3*H*,7*H*)-dione (**5a**) and 0.14 g (0.79 mmol, 1.6 eq.) 1-(ethynylsulfonyl)-4-methylbenzene **(6n)** were used. The crude product was purified by precipitation by dissolving in acetone and treatment with water and petroleum ether. The precipitate was filtered off and washed with water and petroleum ether. The solid was dried at 50-75 °C in a drying oven. The product **2n** was isolated as a white solid in 94 % yield (0.19 g).
Analytical data: **mp** 268 °C. **TLC:** R_{f} = 0.36 (silica gel, dichloromethane/methanol 9 : 1). **LC-MS (m/z):** 402.3 [M+H]⁺ + 419.4 [M+NH₄]⁺ + 217.4 [M-SO₂-C₆H₄-3CH₃+H]⁻ + 357.1 [M-N₂-CH₃-H]⁻ + 372.1 [M-N₂-H]⁻ + 400.0 [M-H]⁻. Purity by **HPLC-UV (254 nm)-ESI-MS:** 99 %. **¹H-NMR (500 MHz, DMSO):** δ = 2.41 (s; 3H), 3.30 (s; 3H), 3.52 (s; 3H), 7.50-7.51 (m; 2H), 7.96-7.98 (m; 2H), 9.61 (s; 1H). **¹³C-NMR (126 MHz, DMSO):** δ = 21.0, 27.8, 29.9, 107.3, 127.1, 127.6, 130.2, 136.6, 139.3, 145.3, 146.3, 148.4, 150.9, 154.2.
**Example 45:** 8-(4-Cyclohexyl-1*H*-1,2,3-triazol-1-yl)-1,3-dimethyl-1*H*-purine-2,6(3*H*,7*H*)-dione (**2o**)

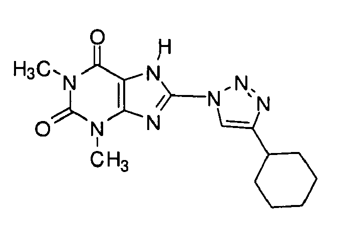

Reaction conditions: According to the general synthesis procedure 0.11 g (0.51 mmol, 1.0 eq.) 8-azido-1,3-dimethyl-1*H*-purine-2,6(3*H*,7*H*)-dione (**5a**) and 0.43 g (3.96 mmol, 7.8 eq.) ethynylcyclohexane **(60)** were used. The reaction was stirred for 25.5 h. The crude product was purified by precipitation by dissolving in acetone and treatment with petroleum ether. The precipitate was filtered off and washed with water and petroleum ether. The solid was dried at 50-75 °C in a drying oven. The product **2o** was isolated as a yellow solid in 32 % yield (54.0 mg). Analytical data: **mp** > 400 °C. **TLC:** R_{f} = 0.60 (silica gel, dichloromethane/methanol 9 : 1). **LC-MS** (m/z): 330.5 [M+H]⁺ + 285.0 [M-N₂-CH₃-H]⁻ + 300.1 [M-N₂-H]⁻ + 328.3 [M-H]⁻. Purity by **HPLC-UV (254 nm)-ESI-MS:** 99 %. **¹H-NMR (500 MHz,** DMSO): δ = 1.22-1.30 (m; 2H), 1.35-1.51 (m; 4H), 1.68-1.70 (m; 1H), 1.76-1.78 (m; 2H), 2.00-2.03 (m; 2H), 2.77-2.81 (m; 1H), 3.27 (s; 3H), 3.47 (s; 3H). **¹³C-NMR (126 MHz, DMSO):** δ = 25.6, 27.4, 29.9, 32.4, 34.5, 55.2, 114.1, 148.1, 149.1, 151.4, 157.4.
**Example 46:** 8-(4-(2-Aminocyclohexyl)-1*H*-1,2,3-triazol-1-yl)-1,3-dimethyl-1*H-*purine-2,6(3*H*,7*H*)-dione **(2p)**

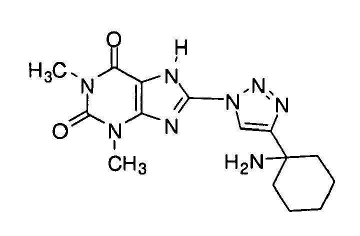

Reaction conditions: According to the general synthesis procedure 0.11 g (0.51 mmol, 1.0 eq.) 8-azido-1,3-dimethyl-1*H*-purine-2,6(3*H*,7*H*)-dione (**5a**) and 0.13 g (1.05 mmol, 2.1 eq.) 1-ethynylcyclohexanamine (**6p**) were used. The product was precipitated by water only. The product **2p** was isolated as a light blue solid in 35 % yield (61.6 mg).
Analytical data: **mp** 330 °C. **TLC:** R_{f} = 0.15 (silica gel, dichloromethane/methanol 9 : 1). **LC-MS (m/z):** 300.4 [M-N₂-NH₂]⁺ + 345.5 [M+H]⁺ + 367.3 [M+Na]⁺ + 300.1 [M-N₂-CH₃-H]⁻ + 315.3 [M-N₂-H]⁻ + 343.3 [M-H]⁻. Purity by **HPLC-UV (254 nm)-ESI-MS:** 100 %. **¹H-NMR (500 MHz, DMSO):** δ = 1.32-1.47 (m; 4H), 1.57-1.59 (m; 2H), 1.65-1.69 (m; 2H), 1.95-1.99 (m; 2H), 3.22 (s; 3H), 3.42 (s; 3H). **¹³C-NMR (126 MHz, DMSO):** δ = 21.8, 25.5, 27.5, 30.0, 38.4, 49.9, 114.1, 148.1, 149.1, 151.7, 156.0, 157.5.

**Example 47:** 1,3-Dimethyl-8-(4-(thiophen-3-yl)-1*H*-1,2,3-triazol-1-yl)-1*H-*purine-2,6(3*H*,7*H*)-dione **(2q)**

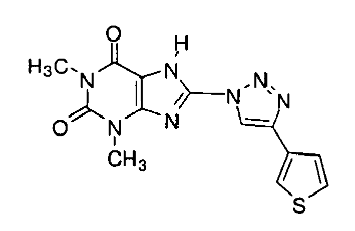

Reaction conditions: According to the general synthesis procedure 0.11 g (0.50 mmol, 1.0 eq.) 8-azido-1,3-dimethyl-1*H*-purine-2,6(3*H*,7*H*)-dione **(5a)** and 0.12 g (1.10 mmol, 2.2 eq.) 3-ethynylthiophene **(6q)** were used. The product **2q** was isolated as a rose solid in 78 % yield (0.13 g).
Analytical data: **mp** 275 °C. **TLC:** R_{f} = 0.52 (silica gel, dichloromethane/methanol 9 : 1). **LC-MS (m/z):** 302.1 [M-N₂+H]⁺ + 330.4 [M+H]⁺ + 285.0 [M-N₂-H-CH₃]⁻ + 300.1 [M-N₂-H]⁻ + 328.3 [M-H]⁻. Purity by **HPLC-UV (254 nm)-ESI-MS:** 100 %. **¹H-NMR (500 MHz, DMSO):** δ = 3.27 (s; 3H), 3.56 (s; 3H), 7.72 (s; 2H), 8.12 (s; 1H), 9.33 (s; 1H), 15.00 (s; 1H). **¹³C-NMR (126 MHz, DMSO):** δ = 27.6, 30.1,114.4, 125.9, 126.0, 127.0, 131.8, 142.3, 147.9, 149.2, 151.8, 157.7.

**Example 48:** 1,3-Dimethyl-8-(4-(1-methyl-1*H*-imidazol-5-yl)-1*H*-1,2,3-triazol-1-yl)-1*H*-purine-2,6(3*H*,7*H*)-dione (**2r**)

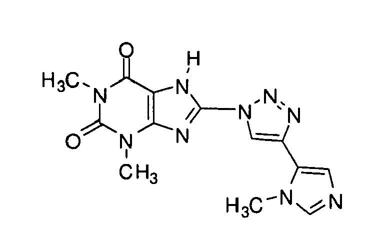

Reaction conditions: According to the general synthesis procedure 0.11 g (0.50 mmol, 1.0 eq.) 8-azido-1,3-dimethyl-1*H*-purine-2,6(3*H*,7*H*)-dione (**5a**) and 0.17 g (1.62 mmol, 3.2 eq.) 5-ethynyl-1-methyl-1*H*-imidazole **(6r)** were used. The product was precipitated by water only. The product **2r** was isolated as a light brown solid in 45 % yield (75.1 mg).
Analytical data: mp 360 **°C. LC-MS (m/z):** 328.3 [M+H]⁺ + 283.3 [M-N₂-CH₃-H]⁻ + 298.4 [M-N₂-H]⁻ + 326.1 [M-H]⁻. Purity by **HPLC-UV (254 nm)-ESI-MS:** 100 %. **¹H-NMR (500 MHz, DMSO):** δ = 3.23 (s; 3H), 3.45 (s; 3H), 3.86 (s; 3H), 7.31 (s; 1H), 7.72 (s; 1H). **¹³C-NMR (126 MHz, DMSO):** δ = 27.5, 30.0, 32.9, 114.4, 123.4, 128.0, 136.8, 139.6, 147.6, 149.2, 151.8, 157.6.

**Example 49:** 8-(4-(3-Methoxyphenyl)-1*H*-1,2,3-triazol-1-yl)-1,3,7-trimethyl-1H-purine-2,6(3*H*,7*H*)-dione **(2s)**

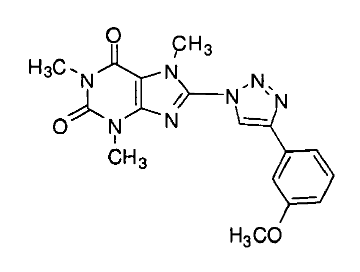

Reaction conditions: According to the general synthesis procedure 0.10 g (0.43 mmol, 1.0 eq.) 8-azido-1,3,7-trimethyl-1*H*-purine-2,6(3*H*,7*H*)-dione **(5a)** and 0.11 g (0.85 mmol, 2.0 eq.) 1-ethynyl-3-methoxybenzene **(6s)** were used. The crude product was purified by dissolving in a mixture of dimethyl formamide and dimethyl sulfoxide under heating. The residue was filtered off and the filtrate was evaporated under reduced pressure. The solid was dried at 50-75 °C in a drying oven. The product **2s** was isolated as a white solid in 46 % yield (71.7 mg).
Analytical data: **mp** 219 °C. **TLC: R_{f} = 0.65** (silica gel, dichloromethane/methanol 9.75 : 0.25). **LC-MS (m/z):** 288.3 [M-78.7]⁺ + 340.4 [M-N₂+H]⁺ + 368.0 [M+H]⁺ + 208.1 [M-158.9]⁻ + 305.1 [M-62.3]⁻ + 323.0 [M-N₂-CH₃-H]⁻ + 338.1 [M-N₂-H]⁻ + 356.0 [M-11.4]⁻ + 426.1 [M+ CH₃COO]⁻ Purity by **HPLC-UV (254 nm)-ESI-MS:** 97 %. **¹H-NMR (500 MHz, CDCl₃): δ** = 3.41 (s; 3H), 3.56 (s; 3H), 3.86 (s; 3H), 4.34 (s; 3H), 6.91-6.93 (m; 1H), 7.35-7.47 (m; 3H), 8.46 (s; 1H). **¹³C-NMR (126 MHz, CDCl₃):** δ = 28.1, 29.8, 34.6, 55.4, 107.5, 111.2, 114.9, 118.3, 120.1, 130.1, 130.1, 140.4, 146.3, 147.5, 151.3, 155.1, 160.1.

**Example 50:** 8-(4-(4-(Hydroxymethyl)phenyl)-1*H*-1,2,3-triazol-1-yl)-1,3,7-trimethyl-1H-purine-2,6(3*H*,7*H*)-dione **(2t)**

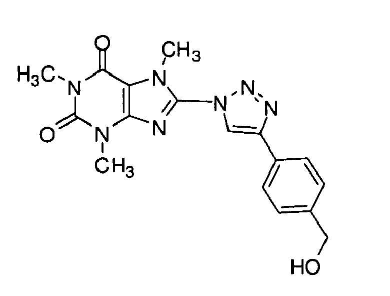

Reaction conditions: According to the general synthesis procedure 0.12 g (0.51 mmol, 1.0 eq.) 8-azido-1,3,7-trimethyl-1*H*-purine-2,6(3*H*,7*H*)-dione (**5a**) and 93.8 mg (0.71 mmol, 1.4 eq.) (4-ethynylphenyl)methanol **(6t)** were used. The crude product was purified by precipitation twice by dissolving in acetone and treatment with diluted hydrochloric acid. The precipitate was filtered off and washed with water. The solid was dried at 50-75 °C in a drying oven. The product **2t** was isolated as a light brown solid in 11 % yield (21.4 mg).
Analytical data: **mp** 220 °C. **TLC:** R_{f} = 0.14 (silica gel, dichloromethane/methanol 2 : 1). **LC-MS (m/z):** 368.1 [M+H]⁺ + 338.2 [M-N₂-H]⁻ + 426.4 [M+CH₃COO]⁻. Purity by **HPLC-UV (254 nm)-ESI-MS:** 99 %. **¹H-NMR (500 MHz, DMSO):** δ = 3.27 (s; 3H), 3.46 (s; 3H), 4.09 (s; 3H), 4.55 (s; 2H), 5.23 (s; 1H), 7.43-7.45 (m; 2H), 7.96-7.98 (m; 2H), 9.22 (s; 1H). **¹³C-NMR (126 MHz, DMSO):** δ = 27.6, 29.5, 33.5, 62.4, 107.0, 122.1, 125.3, 126.9, 127.4, 140.0, 143.1, 145.7, 146.6, 150.7, 154.5.

### Biological Assays

**Example 51: Radioligand binding assays at A₁, A_{2A}, A_{2B} and A₃ adenosine receptors**
Radioligand binding assays at rat A₁ (rat brain cortical membranes), human A₁ (expressed in CHO cells), rat A_{2A} (rat brain striatal membranes), human A_{2A} (expressed in CHO cells), human A_{2B} (expressed in CHO cells) and human A₃ adenosine receptors (expressed in CHO cells) were performed as previously described by Müller et al. (J. Med. Chem. 2009, 52, 3994-4006). The following radioligands were used as A₁, A_{2A}, A_{2B} and A₃ radioligands, respectively: [³H]2-chloro-N⁶-cyclopentyladenosine ([³H]CCPA, 48 Ci/mmol, 1 nM), [³H](*E*)-3-(3-hydroxypropyl)-8-(2-(3-methoxyphenyl)vinyl)-7-methyl-1-prop-2-ynyl-3,7-dihydropurine-2,6-dione ([³H]MSX-2, 84 Ci/mmol, 1 nM), [³H]8-(4-(4-(4-chlorophenyl)piperazine-1-sulfonyl)phenyl)-1-propylxanthine ([³H]PSB-603, 73 Ci/mmol, 0.3 nM), and [³H]2-phenyl-8-ethyl-4-methyl-(8*R*)-4,5,7,8-tetrahydro-1*H*-imidazo[2.1-*i*]purin-5-one ([³H]PSB-11, 53 Ci/mmol, 1 nM). The radioligands were commercially available or custom-labeled by GE Healthcare from suitable precursors as described by Müller, Klotz and others (Bioorg. Med. Chem. 2006, 14, 2837-2849, Naunyn-Schmiedeberg's Arch. Pharmacol. 1989, 340, 679-683, Eur. J. Pharm. Sci. 2000, 10, 259-265, Bioorg. Med. Chem. Lett. 2002, 12, 501-503).

Data analysis: The data were analyzed using GraphPad Prism 4.0 (GraphPad Software, Inc., San Diego, CA, USA).

Concentration-inhibition curves were recorded at various concentrations from 0.01-10 µM of three separate experiments each run in triplicate. From each curve, mean values and the Kᵢ value were calculated, which are given in nM ± SEM (n = 3). For **1a**, an exemplary curve is displayed in Fig. 3 below.

The quantitative experimental results for representative compounds are summarized in Table 1 below.

**Example 52: cAMP assay**
The preparation of cAMP binding protein was carried out as previously described by Nordstedt and Fredholm (Anal. Biochem. 1990, 189, 231-234). 5'-(*N-*Ethylcarboxamido)adenosine (NECA) was obtained from Sigma. For cAMP experiments HEK cells, expressed the human recombinant A_{2B} receptor were removed from a confluent 175 cm² flask, transferred into a falcon tube and spun down at 200 g, 4 °C for 5 min. After removal of the supernatant, the cell pellet was resuspended in Hank's buffered saline solution (HBSS, pH 7.4). 200 µl of the cell solution (∼ 200.000 cells per well) with 2 U/ml ADA was transferred into each well of a 24-well plate. After an incubation time of 1.5 h at 37 °C, 5 % CO₂, 25 µL of the phosphodiesterase (PDE) inhibitor Ro-20-1724 (40 µM f.c.), dissolved in 100 % HBSS-puffer was added to each well and incubated for 10 min at 37 °C. Then, 12.5 µl of antagonist **1a**, dissolved in HBSS-buffer containing 10 % DMSO was added. After 10 min incubation time 12.5 µl of agonist (NECA) dissolved in the same buffer as well, was added and incubated for 15 min at 37 °C. NECA was incubated in concentrations of 10 µM and 1 µM alone as an control experiment. The final DMSO concentration did not exceed 1.4 %. cAMP accumulation was stopped by lysing the cells with 250 µL of hot lysis buffer (100 °C; 8 mM EDTA, 0.02 % trition X-100). Well plates were put on ice and each well was subsequently homogenised by pipetting 6-7 times up and down. Competition binding experiments were performed in a final volume of 120 µl containing 50 µl of cell lysates, 30 µl of [³H]cAMP (f.c. 3 nM) in lysis buffer (4 mM EDTA, 0.01 % trition X-100) and 40 µl of cAMP binding protein in the same buffer (50 µg protein per vial). Total binding was determined in the absence of cold cAMP and nonspecific binding was determined in the absence of cold cAMP and without binding protein. For a cAMP standard curve 50 µl of known cAMP concentration was put instead of 50 µl cell lysate. After an incubation time of 60 min on ice, the assay mixture was filtered through GF/B glass fiber filters using a Brandel harvester (Brandel, Gaithersburg, MD). Filters were washed three times (3-4 ml each) with ice-cold 50 mM Tris-HCl buffer, pH 7.4. Then, filters were transferred to vials, incubated for 9 h with 2.5 mL of scintillation cocktail (Beckmann Coulter), and counted in a liquid scintillation counter (Tricarb 2700TR) with a counting efficiency of ∼ 54%.

By a functional experiment compound **1a** was confirmed as an antagonist for human A_{2B} adenosine receptors. NECA was used as an agonist in concentrations of 10 µM and 1 µM in combination with the new antagonist **1a** in a concentration of 5 µM. The A_{2B} agonist NECA leads to a concentration-dependent increase in cAMP formation, which was inhibited by compound **1a**. Compound **1a** itself showed no effect on cAMP levels. An example is shown in Fig. 4A below.

By a second functional experiment compounds **1d** and **1f** were confirmed as antagonists for human A_{2B} adenosine receptors. Dose-response curves for the agonist NECA were determined in the absence and in the presence of a single concentration of the new antagonists **1d** and **1f,** using a ∼ 5-fold concentration of the Kᵢ-values. The A_{2B} agonist NECA led to a concentration-dependent increase in cAMP formation. The NECA curve which was shifted to the right by the antagonists indicating competitive antagonism. An example is shown in Fig. 4B below.

**Example 53:** ELISA Assay
The ELISA assay was used to investigate the effects of adenosine antagonists. Therfore ESdM cells were activated with lipopolysaccharide (LPS) and adenosine receptors were stimulated with adenosine or the synthetic adenosine receptor agonist NE-CA, which resulted in an expression of the proinflammatory cytokine TNFα. In the presence of the antagonists to be investigated, their influence on TNFα expression was analyzed. The cytokine expression was determined in the ELISA (Fig. 5).

To evaluate, whether ESdM cells can be stimulated by Adenosine 3 x 10⁵ ESdM cells were seeded in 12 wells. The next day the cells have been stimulated with 1 µM Adenosine (Sigma-Aldrich) and different concentrations of LPS (10 ng/ml, 100 ng/ml and 500 ng/ml, Sigma-Aldrich). Supernatants were collected 24 hours later and were analysed for TNFα expression in an ELISA (EBioscience). As shown here (Fig. 6A), ESdM cells are sensitive for stimulation with 1 µM adenosine. Best results are achieved with a LPS concentration of 100 ng/ml, whereas 500 ng/ml of LPS provide better detectable TNFα values.

To examine, if ESdM cells can also be stimulated with the synthetic adenosine receptor agonist NECA, ESdM cells were seeded in 12 wells in the same concentration as mentioned before. These cells were treated with different concentrations of NECA (1 µM and 100 nM) and 100 ng/ml LPS. The next day supernatants were collected and analysed for TNFα expression. As shown in Fig. 6B, ESdM cells are sensitive for stimulation with 1 µM and 100 nM of NECA, wherein 100 nM of NECA provide the better detectable TNFα values.

To analyse the effect of the A_{2B} antagonist **1a,** 3 x 10⁵ ESdM cells were seeded in 12 wells. The next day cells were stimulated with 100 nM NECA, 100 ng/ml LPS and various concentrations (10 pM-10 nM) of A_{2B}-receptor antagonist **1a** Supernatants of the cells were investigated for TNFα expression in an ELISA. The A_{2B} antagonist **1a** showed a dose-dependent decrease in TNF alpha expression. The determined EC₅₀ concentration amounts to 2.6 µM (Fig. 7A, B). A dose dependent decrease in TNF alpha expression was also shown for the A_{2B} antagonists **1b** (Fig. 8A, B), **1d** (Fig 9A, B), **1f** (Fig. 10 A, B) and **1g** (Fig. 11 A, B). The evaluated EC₅₀ concentrations are displayed in Table 2.

By this functional experiment the compounds **1a, 1b, 1d, 1f** and **1g** were confirmed as antagonists of human A_{2B} adenosine receptors.

## Claims

1. Compounds of general formulae **I** and **II** 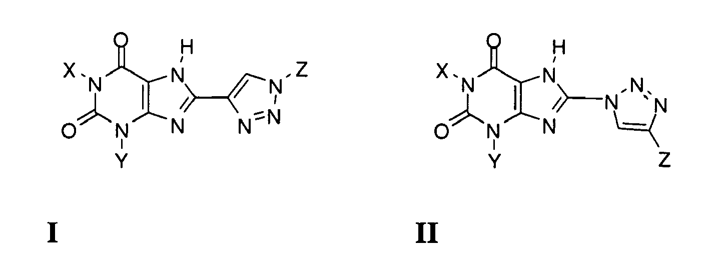 in which the substituents have the following meanings:
X, Y are independently
H
C₁₋C₁₀ alkyl
C₂-C₁₀ alkenyl
C₂-C₁₀ alkynyl
C₃-C₆ cycloalkyl
C₃-C₆ heterocycloalkyl
aryl
C₃-C₆ heteroaryl
benzyl
Z is H
C₁-C₁₀ alkyl
C₂-C₁₀ alkenyl C₂-C₁₀ alkynyl
C₃-C₆ cycloalkyl
C₃-C₆ heterocycloalkyl
aryl
C₃-C₆ heteroaryl
benzyl

2. The compound according to claim 1, wherein the substituents have the following meanings:
X = 2-propynyl, methyl, ethyl, propyl, cyclopropyl or cyclopropylmethyl
Y = H, methyl, ethyl or propyl
Z = aryl, benzyl

3. The compound according to claim 1 or 2 selected from:
1,3-Dimethyl-8-(1-(3-(trifluoromethyl)benzyl)-1*H*-1,2,3-triazol-4-yl)-1*H-*purine-2,6,(3*H*,7*H*)-dione,
1,3-Diethyl-8-(1-(3-trifluoromethyl)benzyl)-1*H*-1,2,3-triazol-4-yl)-1*H*-purine-2,6,(3*H*,7*H*)-dione,
Ethyl-8-(1-(3-trifluoromethyl)benzyl)-1*H*-1,2,3-triazol-4-yl)-1*H*-purine-2,6,(3*H*,7*H*)-dione,
1-Propyl-8-(1-(3-trifluoromethyl)benzyl)-1*H*-1,2,3-triazol-4-yl)-1*H*-purine-2,6,(3*H*,7*H*)-dione,
3-Methyl-8-(1-(3-(trifluoromethyl)benzyl)-1*H*-1,2,3-triazol-4-yl)-1*H*-purine-2,6(3*H*,7*H*)-dione,
3-Methyl-1-(prop-2-ynyl)-8-(1-(3-(trifluoromethyl)benzyl)-1*H*-1,2,3-triazol-4-yl)-1*H*-purine-2,6,(3*H*,7*H*)-dione,
1-(Cyclopropylmethyl)-3-methyl-8-(1-(3-(trifluoromethyl)benzyl)-1*H*-1,2,3-triazol-4-yl)-1*H*-purine-2,6(3*H*,7*H*)-dione, or
1,3,7-Trimethyl-8-(1-(3-(trifluoromethyl)benzyl)-1*H*-1,2,3-triazol-4-yl)-1*H-*purine-2,6(3*H*,7*H*)-dione

4. Pharmaceutical composition containing one or more compounds according to any of claims 1 to 3 as well as conventional excipients and carriers.

5. A compound as defined in any of claims 1 to 3 for the use in a method of treatment of neurodegenerative diseases.

6. The compound for the use as defined in claim 5, wherein the neurodegenerative disease is stroke, Alzheimer's disease or amylotrophic lateral sclerosis.

7. A process for the production of compounds according to any of claims 1 to 3, comprising the following process steps:
- 8-Ethynyl-1*H*-purine-2,6(3*H*,7*H*)-dione derivatives of general formula **III** reacted with azides of general formula **IV**.
- Selectively alkylation of position 7 with 2-(trimethylsilyl)ethoxymethyl chloride (SEMCl) as protective group, followed by further alkylation of position 1 and subsequent removal of the protective group under acidic conditions.
- Alkylation of 8-(1*H*-1,2,3-triazol-4-yl)-1*H*-purine-2,6(3*H*,7*H*)-dione derivatives of general formula **I** by alkylating reagents for example iodomethane.
- Alkylation of 8-bromo-1H-purine-2,6(3*H*,7*H*)-dione derivatives in position 7 with 2-(trimethylsilyl)ethoxymethyl chloride (SEMCl) or iodomethane, followed by further reaction with sodium azide at position 8 and subsequent removal of the protective group under acidic conditions.
- 8-Azido-1*H*-purine-2,6(3*H*,7*H*)-dione derivatives of general formula **V** reacted with alkynes of general formula **VI**.
